# EUROPEAN PATENT SPECIFICATION

(11) **EP 3 321 280 B1**
(45) Date of publication and mention of the grant of the patent: **23.12.2020**
(21) Application number: 16198189.9
(22) Date of filing: 10.11.2016
(51) Int. Cl.: C07K 14/705, C07K 16/28, C12N 15/113, G01N 33/574

(54) **IMMUNE MODULATORS FOR REDUCING IMMUNE-RESISTANCE IN MELANOMA AND OTHER PROLIFERATIVE DISEASES**
IMMUNMODULATOREN ZUR REDUZIERUNG DER IMMUNRESISTENZ IN MELANOMEN UND ANDEREN PROLIFERATIVEN ERKRANKUNGEN
MODULATEURS IMMUNITAIRES DE RÉDUCTION D'IMMUNO-RÉSISTANCE DANS UN MÉLANOME ET D'AUTRES MALADIES PROLIFERATIVES

(43) Date of publication of application: 16.05.2018
(73) Proprietor: Deutsches Krebsforschungszentrum Stiftung des Öffentlichen Rechts, 69120 Heidelberg (DE)
(72) Inventor: Beckhove, Philipp, 93059 Regensburg (DE); Michels, Tillmann, 93049 Regensburg (DE); Khandelwal, Nisit, 80469 München (DE); Boutros, Michael, 69117 Heidelberg (DE); Breinig, Marco, 69198 Schriesheim (DE); Sorrentino, Antonio, 93051 Regensburg (DE); Volpin, Valentina, 93053 Regensburg (DE)
(74) Representative: Kuttenkeuler, David

(56) References cited:
- WO-A1-2015/028515
- WO-A1-2015/121454
- WO-A2-2008/085601
- US-A1- 2013 130 379
- Weizmann Institute of Science: "OR10H1", Weizmann Institute of Science , XP002769440, Retrieved from the Internet: URL:http://www.genecards.org/cgi-bin/cardd isp.pl?gene=OR10H1&keywords=or10h1 [retrieved on 2017-04-21]
- TOPALIAN SUZANNE L ET AL: "Safety, activity, and immune correlates of anti-PD-1 antibody in cancer", NEW ENGLAND JOURNAL OF MEDICINE, THE - NEJM, MASSACHUSETTS MEDICAL SOCIETY, US, vol. 366, no. 26, 28 June 2012 (2012-06-28), pages 2443-2454, XP002767416, ISSN: 1533-4406

## Description

The present invention pertains to novel modulators of resistance against T-cell mediated cytotoxic immune responses in accordance with the appended set of claims. The invention provides antagonists of immune escape mechanisms and therefore offers a novel approach for treating, or aiding a treatment, of cancerous diseases, in particular melanoma, pancreatic cancer and colorectal cancer in accordance with the appended set of claims. The invention specifically discloses the receptor Olfactory Receptor, Family 10, Subfamily H, Member 1 (OR10H1) as a checkpoint molecule in tumor resistance against cytotoxic T-cells. Disclosed is the inhibition of OR10H1 expression and/or function as a strategy for enhancing tumor susceptibility to a patients T-cell mediated immune response. Provided are antigen binding constructs for the detection of the human OR10H1 protein, as well as siRNA molecules targeting human OR10H1 and anti-ORlOHl antibodies, for impairing the immune escape mediated by human OR10H1 in accordance with the appended set of claims. The invention furthermore provides screening methods for the identification of novel cancer therapeutics based on the modulation of human OR10H1 expression/function, diagnostic methods for the detection of immune resistance of a tumor to cytotoxic T-cell responses, as well as pharmaceutical compositions, in each case in accordance with the appended set of claims.

Peripheral immune tolerance is important to prevent autoimmune disorders. However, tumor cells use immune checkpoints to prevent immune recognition (Zitvogel et al, Nat Rev Immunol. 2006;6:715-727; Rabinovich et al, Annu Rev Immunol. 2007;25:267-296). Blocking antibodies against surface-expressed immune-regulatory proteins, such as CTLA4 and PD-L1 (Chambers et al, Annu Rev Immunol. 2001;19:565-594; Blank et al, Cancer Res. 2004;64:1140-1145), boost anti-tumor immunity and are successfully applied in clinical trials (van Elsas et al, J Exp Med. 1999;190:355-366; Weber, Oncologist. 2007;12:864-872; Brahmer et al, New Engl J Med. 2012;366:2455-2465; Topalian et al, New Engl J Med. 2012;366:2443-2454). Still, treatment unresponsiveness is frequent among patients (Topalian et al, New Engl J Med. 2012;366:2443-2454), indicating that other immune-checkpoint pathways may be active. Therefore, successful cancer immunotherapy requires a systematic delineation of the entire immune-regulatory circuit-the 'immune modulatome'-expressed on tumors (Woo et al, Cancer Res. 2012;72:917-927; Berrien-Elliott et al, Cancer Res. 2013;73:605-616).

A comprehensive detection of immune-checkpoint molecules has been technically challenging due to the lack of robust high-throughput assays that enable a qualitative and quantitative analysis of heterologous interactions between tumor cells and T cells. Screening strategies before have relied on interferon-gamma (IFN-γ) release as an indicator of anti-tumor NK cell activity (Hill & Martins, Methods. 2006;38:312-316; Bellucci et al, J Clin Investig. 2012;122:2369-2383). However, IFN-γ secretion alone by immune cells does not always correlate with cellular cytotoxicity (Bachmann et al, Eur J Immunol. 1999;29:291-299; Slifka et al, Nature. 1999;401:76-79).

Olfactory receptors (ORs) are members of the seven transmembrane G protein-coupled Receptor (GPCR) class A (Rhodopsin-like). They sense the chemical environment and can be distinguished by the chemostimuli to which they respond. Olfactory receptors signal mainly via a unique G protein-coupled adenylyl cyclase cascade. Subsequently, cAMP is the key messenger of olfactory G protein signaling. Olfactory signaling leads to the specific cAMP production by adenylyl cyclase type III. AC3 in turn is activated by the olfactory-restricted G protein alpha subunit GαOlf. It was shown that olfactory receptors could couple *in vitro* to Gas and Gα15 G proteins, which might alter the specificity of the receptor. Furthermore, olfactory receptors can signal via other mechanisms. Olfactory receptor activation leads to production of cGMP, opens cyclic nucleotide-gated channels (CNC) by cAMP and cGMP, stimulates the production of Inositol-1,4,5-trisphosphate (IP3) and increases influx of calcium. Olfactory receptors are a highly divergent group of receptors ranging in a length of 300-350 amino acids, depending on the length of the N- and C-terminal stretches. They are coded by single coding-exon genes, but exons in the 5' untranslated region may undergo alternate splicing. Due to a high number of degenerated pseudogenes only about 390 human functional OR genes are known (compared to 855 OR genes in total). Human ORs are organized in 18 families (sequence similarity >40%) and around 300 subfamilies (similarity >60%). As mentioned before ORs contain seven hydrophobic membrane-spanning domains and belong to the GPCR class A. Several characteristic conserved amino acids motifs distinguish ORs from other GPCRs (e.g. extracellular NXS/T consensus for N-linked glycosylation). Interestingly, published data suggest that the transmembrane domains (e.g. amino acids in TM3, TM5 and TM6) are essential for the specificity of odorant binding pockets.

WO 2008/085601 A2 relates to the selection of a marker set of polymorphisms for use in genome wide association studies based on linkage disequilibrium mapping in context of asthma. The document mentions certain genes, including in a table SeqID 1798 encoding OR10H1, and relates to the fields of pharmacogenomics, diagnostics, patient therapy and the use of genetic haplotype information to predict an individual's susceptibility to asthma and/or an individual's response to a particular drug or drugs.

US 20131130379 A1 relates to amino acid sequences that are directed against G-protein coupled receptors (GPCRs), as well as to proteins and polypeptides that comprise such amino acid sequences, such as antigen binding constructs. The document mentions OR10H1 within a list of GPCRs.

WO2015028515 describes a compound selectively binding to a sensory receptor or selectively altering the expression of a sensory receptor for use in a method for treating or preventing a disease associated with a pathologic cellular cytotoxic T cell (CTL) response, preferably a disease selected from the group consisting of neoplasia, in particular cancer, an autoimmune disease, an infection and graft-versus-host disease.

Preferably, the sensory receptor is, in particular, TAS2R3, OR1F1, OR2J2, OR51E2, VN1R4, or OPN3.

There is a need in the art for novel approaches to circumvent tumor immune escape mechanisms and to render tumors more susceptible for the immune system. The present invention seeks to provide novel therapeutic compounds, as well as therapeutic and diagnostic approaches and methods involving such compounds, that are able to modulate a host's immune response, in particular to strengthen a cytotoxic T cell response against tumor cells. Furthermore, the invention seeks to provide novel strategies to diagnose tumor resistance to immune response and screening approaches for the identification of compounds that are useful in cancer treatment.

The above problem is solved in a first aspect by a compound for use in the treatment of a disease of a subject, wherein the compound is a modulator of the expression, function and/or stability of human Olfactory Receptor, Family 10, Subfamily H, Member 1 (OR10H1), or of a variant of human OR10H1;
wherein the variant of human OR10H1 is a protein comprising an amino acid sequence having at least 95% sequence identity to the sequence of SEQ ID NO: 25; and wherein the disease is a human OR10H1 positive cancer, or a cancer positive for the variant of human OR10H1; and
wherein the compound is: (i) a small interfering RNA (siRNA) that binds directly to mRNA of said human OR10H1 or the variant of human OR10H1, and so inhibits expression of said human OR10H1, or the variant of human OR10H1; or (ii) an antigen binding construct that specifically binds said human OR10H1, or the variant of human OR10H1, and that reduces resistance of a tumour- or cancer cell characterized by the expression of human OR10H1, or the variant of human OR10H1, to cytotoxic T lymphocyte (CTL) responses..

The term "olfactory receptor family 10" pertains to a family of olfactory receptor proteins containing 29 subfamilies, wherein the subfamily H consists of five functional OR genes (no pseudogenes). Human olfactory receptor family 10 subfamily H member 1 (OR10H1) is also known as AC004510, OR19-27, HSOR19.4.4, ORL733, ORL525 (HUGO Gene Nomenclature Committee symbol: HGNC:8172). The human OR10H1 gene (chromosome 19: 15,807,003-15,808,126; GRCh38:CM000681.2) is 1124 base pairs long and codes a protein of 318 amino acids (SEQ ID NO: 25, see below), UniProtKB identifier Q9Y4A (Sequence version 1 of 01-Nov-1999; Entry version 126 of 05-Oct-2016).

The terms "OR10H1-protein" or "protein of OR10H1" as used in context of the herein disclosed invention shall pertain to a protein (such as a full-length protein, fusion protein or partial protein) comprising a sequence as shown in SEQ ID NO: 25. The terms shall also refer to a protein comprising the amino acid sequence according to SEQ ID NO: 25 with any protein modifications. Such protein modifications preferably do not alter the amino acid sequence of the polypeptide chain, but constitute a functional group, which is conjugated to the basic amino acid polymer chain. Protein modifications in context of the invention may be selected from a conjugation of additional amino acid sequences to the OR10H1 amino acid chain, such as ubiquitination, sumolation, neddylation, or similar small protein conjugates. Other protein modifications include, but are not limited to, glycosylation, methylation, lipid-conjugation, or other natural or artificial post-translational modifications known to the skilled person. The terms "protein of a variant of OR10H1" and the like, shall have the corresponding meaning with respect to a variant of OR10H1.

The terms "OR10H1-mRNA" or "mRNA of OR10H1" as used in context of the herein disclosed invention shall pertain to a messenger ribonucleic acid (such as a full-length mRNA, fusion mRNA or partial mRNA, and/or splice-variants thereof) comprising a region encoding for an amino acid sequence as shown in SEQ ID NO: 25. The terms shall also refer to an mRNA comprising a region encoding for the amino acid sequence according to SEQ ID NO: 25 with any codon or nucleotide modifications. Such modifications preferably would not alter the amino acid sequence of the encoded polypeptide chain. The terms "mRNA of a variant of OR10H1" and the like, shall have the corresponding meaning with respect to a variant of OR10H1.

A variant of human OR10H1 is a protein comprising an amino acid sequence having at least 95% (such as at least 98%) sequence identity to SEQ ID NO: 25 (the human OR10H1 amino acid sequence).

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site). In a particular embodiment, for example when comparing the protein or nucleic acid sequence of OR10H1 to another protein/gene, the percentage identity can be determined by the Blast searches supported at the Human Olfactory Data Explorer (eg, https://genome.weizmann.ac.il/cgi-bin/horde/blastHorde.pl); in particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

A variant of human OR10H1 is, in certain embodiments, a functional variant of human OR10H1 protein. In other embodiments of the invention, the variant of human OR10H1 is selected from the group consisting of an ortholog of human OR10H1, and a functional fragment of an human OR10H1 protein.

The term "ortholog" refers to homologs in different species that evolved from a common ancestral gene by speciation. Typically, orthologs retain the same, essentially the same or similar function despite differences in their primary structure (mutations). The term "paralog" refers to homologs in the same species that evolved by genetic duplication of a common ancestral gene. In many cases, paralogs exhibit related but not always similar function. The term "splice variant" refers to a related protein expressed from the same genomic locus as a parent protein, but having a different amino acid sequence based on a different exon composition due to differential splicing of the transcribed RNA.

In context of the present disclosure the term "subject" or "patient" preferably refers to a mammal, such as a mouse, rat, guinea pig, rabbit, cat, dog, monkey, or preferably a human, for example a human patient. The subject of the invention may be at danger of suffering from a proliferative disease such as a cancer or a tumor disease, or suffer from a cancer or tumor disease, preferably, wherein the tumor disease is a tumor having a resistance or increased resistance to the host's (the patient's) immune response. A preferred immune response in context of the invention is a cell-mediated immune response such as a cytotoxic T-cell response. A more detailed description of medical indications relevant in context of the invention is provided herein elsewhere.

The term "modulator" in context of the present invention shall include inhibitors/antagonists. A preferred embodiment of the invention pertains to inhibitors/antagonists as modulators of the expression, function and/or stability of human Olfactory Receptor, Family 10, Subfamily H, Member 1 (OR10H1), or of a variant of OR10H1, in accordance with the appended set of claims.

A "modulator of expression, function and/or stability of OR10H1, or of a variant of OR10H1", or grammatically similar expressions, in context of the disclosure may be any compound that affects, for example when an inhibitor/antagonist impairs or interferes with, the expression, function and/or stability of OR10H1, or of a variant of OR10H1, in particular the expression, function and/or stability of protein of OR10H1 or the variant, and/or the expression, function and/or stability of mRNA of OR10H1 or the variant.

Preferred modulators are, in context of the invention, inhibitors or antagonists that inhibit (eg, impair or interfere with) human OR10H1 or its variant's expression, function and/or stability, in particularly specifically and/or selectively, in cells showing a pathological form of cell proliferation, growth or survival. A preferred example is a cell that has become a tumor cell, or originates from a tumor cell.

In one embodiment, the modulator of expression, function and/or stability of human OR10H1, or of a variant of human OR10H1, may modulate human OR10H1, or the variant of human OR10H1, via a direct interaction (such as non-covalent and covalent binding) between the modulator and the human OR10H1 protein, or a protein of a human OR10H1 variant, or their RNA transcripts. For example, a modulator of the invention may inhibit the expression, function and/or stability of human OR10H1, or of a variant of human OR10H1, binding directly to a protein of human OR10H10 or the variant, and so for example inhibit the function of OR10H1 or the variant (such as a modulator that is an inhibitory antibody against protein of human OR10H1 or of the variant), or may bind directly to mRNA of human OR10H10 or the variant, and so for example inhibit the expression of human OR10H1 or the variant.

As used herein, the terms "inhibitor of OR10H1 expression" and the like (including similarly, "antagonist of OR10H1 expression" and the like) shall relate to any of the herein disclosed modulators (for example, the antigen binding constructs or anti-sense molecules described herein), which has an antagonistic activity toward the expression of an OR10H1 protein, such that it impairs, suppresses, reduces and/or lowers the expression of an OR10H1 protein such as may be determined by measuring an amount (or change in an amount) of OR10H1 protein or OR10H1 mRNA. The term "expression" means in this context the cellular process of transcribing a gene into an mRNA and the following translation of the mRNA into a protein. "Gene expression" therefore may refer only to the generation of mRNA, irrespectively from the fate of the so produced mRNA, or alternatively/additionally to the translation of the expressed mRNA into a protein. The term "protein expression" on the other hand shall refer to the complete cellular process of synthesis of proteins.. The terms "inhibitor of expression of a variant of OR10H1" and the like, shall have the corresponding meaning with respect to a variant of OR10H1.

The terms "inhibitor of OR10H1 stability" and the like (including similarly, "antagonist of OR10H1 stability" and the like) shall refer to any of the herein disclosed modulators (for example, the antigen binding constructs or anti-sense molecules described herein), which has a negative activity towards the stability of an OR10H1 protein. The term, in context of the present disclosure, shall be understood in its broadest sense. Such modulators are included by the term, which, for example, interfere with and reduce the OR10H1 protein half-live or interfere with and disturb OR10H1 protein folding or protein presentation on the surface of the cell. In one preferred example, an inhibiting modulator of the invention, such as an antigen binding construct, may induce internalisation, and optionally degradation, of OR10H1 protein from the surface of the cell. Other inhibiting modulators are included by the term, which, for example, interfere with and reduce the OR10H1 mRNA half-live or interfere with and disturb OR10H1 mRNA presence in the cytoplasm of the cell or presentation to a ribozyme. The terms "inhibitor of stability of a variant of OR10H1" and the like, shall have the corresponding meaning with respect to a variant of OR10H1.

The terms an "inhibitor of OR10H1 function" and the like (including similarly, "antagonist of OR10H1 function" and the like) shall refer to any of the herein disclosed modulators (for example, the antigen binding constructs or anti-sense molecules described herein) that impairs, such as induces a decrease or reduction in the amount or rate of one or more activities of OR10H1 protein or mRNA (for example, by impairing the expression and/or stability of OR10H1 protein or mRNA), such as one or more of those activities described herein, for example, the activity of OR10H1 as a modulator of T-cell activation and/or viability. For example, such an inhibiting modulator may impair binding of one or more of the chemostimuli of OR10H1 protein, may impair signaling by the G protein-coupled adenylyl cyclase cascade of OR10H1 protein and/or may impair the coupling or activity of one or more of the Gas and Gα15 G proteins of OR10H1 protein. The terms "inhibitor of function of a variant of OR10H1" and the like, shall have the corresponding meaning with respect to a variant of OR10H1.

Such an inhibiting modulator can act directly, for example, by binding to OR10H1 and decreasing the amount or rate of one or more of the properties of OR10H1 such as its expression, function and/or stability. An OR10H1 antagonist or inhibitor can also decrease the amount or rate of OR10H1 function or activity by impairing its expression, stability, for example, by binding to OR10H1 protein or mRNA and modifying it, such as by removal or addition of a moiety, or altering its three-dimensional conformation; and by binding to OR10H1 protein or mRNA and reducing its stability or conformational integrity.. Thus, an OR10H1 inhibitor or antagonist can act by any mechanisms that impair, such as result in a decrease in, the amount or rate of OR10H1 expression, function and/or stability.

Therefore, a "functional variant" of OR10H1 (such as a functional fragment of an OR10H1 protein) is a variant of, such as a fragment of, the protein of OR10H1 that provides, possesses and/or maintains one or more of the herein described functions/activities of the non-variant protein of OR10H1. For example, such functional variant may bind one or more of the same chemostimuli as OR10H1 protein, may signal the same G protein-coupled adenylyl cyclase cascade as the OR10H1 protein and/or may be coupled to one or more of the same Gas and Gα15 G proteins as OR10H1 protein, such as having the same, essentially the same or similar specificity and/or function as a receptor as OR10H1 protein. In other embodiments, such a functional variant may possess other activities than those possessed by the non-variant OR10H1 protein, as long as, preferably, it provides, possesses and/or maintains at least one function/activity that is the same, essentially the same or similar as OR10H1 protein. In more preferred embodiments, a functional variant of OR10H1 protein may act as an immune checkpoint inhibitor, such as by inhibiting cell-based immune response to a cancer cell that expresses such functional variant.

### Antigen Binding Construct

Insofar the invention pertains to an isolated antigen binding construct as such, independent of its use in the treatment of a disease of a subject, the isolated antigen binding construct comprises all features as defined in appended claims 9 or 10.

Preferred embodiments of other aspects of the invention involve an antigen binding construct capable of specifically binding to human OR10H1, or to a variant of human OR10H1, wherein the antigen binding construct inhibits the expression, function and/or stability of human OR10H1, or the variant of human OR10H1, in accordance with the appended set of claims. In particular embodiments, the antigen binding construct of the invention binds to protein of human OR10H1, or of the variant of human OR10H1, under conditions that are similar, essentially the same or the same as physiological conditions, such as those which can be created or mimicked by one or more of the in-vitro assays described by the examples herein.

The term "antigen binding construct" includes all varieties of antibodies and T cell receptor (TCR) derived polypeptides, which comprise an epitope binding domain, including binding fragments thereof. Further included are constructs that include 1, 2, 3, 4, 5, and/or 6 Complementary Determining Region (CDR)s, the main regions mediating antibody or TCR binding ability and specificity to a given antigenic epitope. In some embodiments, these CDRs can be distributed between their appropriate framework regions in a typical antibody or TCR variable domain. In some embodiments, the CDRs can be within a single peptide chain in others they are located in two or more peptide chains (heavy/light or alpha/beta respectively). In some embodiments, the two or more peptides are covalently linked together, for example via disulfide bonds. In some embodiments, they can be linked via a linking molecule or moiety. In some embodiments, the antigen binding proteins are non- covalent, such as a diabody and a monovalent scFv. Unless otherwise denoted herein, the antigen binding constructs described herein bind to an OR10H1 protein, or a variant protein of OR10H1 as described in detail herein above. Preferred embodiments of the invention pertain to antibodies, or antibody derived polypeptides, as antigen binding constructs of the invention.

In a more preferred embodiment, the antigen binding construct is isolated.

The term "isolated" as used herein in the context of a polypeptide, such as an antigen binding construct (an example of which could be an antibody), refers to a polypeptide that is purified from proteins or polypeptides or other contaminants that would interfere with its therapeutic, diagnostic, prophylactic, research or other use. An antigen binding construct according to the disclosure may be a recombinant, synthetic or modified (non-natural) antigen binding construct. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. In this context, a "recombinant" protein/polypeptide or nucleic acid is one made using recombinant techniques. Methods and techniques for the production of recombinant nucleic acids and proteins are well known in the art.

In another embodiment, the isolated antigen binding construct is an antibody obtainable from hybridoma Di-8A11-H12-E6 (DSMZ Deposition Number: DSM ACC3310, deposited 26-Oct-2016), or it is an antigen binding fragment obtainable from such antibody. Hybridoma clone Di-8A11-H12-E6 is a hybridoma comprising Rat B lymphocytes fused with murine Ag8 myeloma cells, and can be cultured at 37°C in a 5% CO₂ atmosphere in RPMI-1640 medium supplemented with 10% Fetal calf serum, 10 U/ml human recombinant IL-6 and 1% penicillin-streptomycin (10,000 U/mL). The optimal split ratio is 1:1. The viability of these hybridoma cells may be relatively low when first thawed, however this recovers over a few days in culture.

In particular embodiments, such hybridoma-obtainable antigen binding construct is capable of specifically binding to human OR10H1, or to a variant of human OR10H1, and in more particularly embodiments wherein such antigen binding construct inhibits the expression, function and/or stability of human OR10H1, or the variant of human OR10H1.

Preferred embodiments of other aspects of the invention involve an (isolated) antigen binding construct in accordance with the appended set of claims and comprising at least one CDR3 having an amino acid sequence with at least 80% sequence identity to an amino acid sequence selected from SEQ ID Nos: 3, 7, 11, 15, 19, and 23. The antigen binding construct may comprise a CDR3 region that has an amino acid sequence having no more than three or two, preferably one amino acid substitution(s), deletion(s) and/or insertion(s) relative to a sequence selected from the group consisting of SEQ ID NOs. 3, 7, 11, 15, 19, and 23. Some CDR3 sequences of the disclosure are 8 amino acids long (see table D1 below). For such longer CDR3 sequences, the degree of sequence identity may be preferably increased in some embodiments to 85%. In particular embodiments, such CDR3-comprising antigen binding construct is capable of specifically binding to human OR10H1, or to a variant of human OR10H1, and in more particularly embodiments wherein such antigen binding construct inhibits the expression, function and/or stability of human OR10H1, or the variant of human OR10H1.

In some additional embodiments, the antigen binding construct further comprises at least one CDR1, and at least one CDR2. Further preferred antigen binding constructs of the invention include at least two of each kind of CDR sequences.

As mentioned earlier, preferred antigen binding constructs are antibodies and antibody-like constructs. The term "antibody" includes, but is not limited to, genetically engineered or otherwise modified forms of immunoglobulins, such as intrabodies, chimeric antibodies, fully human antibodies, humanized antibodies (e.g. generated by "CDR-grafting"), antibody fragments, and heteroconjugate antibodies (e.g., bispecific antibodies, diabodies, triabodies, tetra-bodies, etc.). The term "antibody" includes cys-diabodies and minibodies. Thus, each and every embodiment provided herein in regard to "antibodies", or "antibody like constructs" is also envisioned as, bi-specific antibodies, diabodies, scFv fragments, chimeric antibody receptor (CAR) constructs, diabody and/or minibody embodiments, unless explicitly denoted otherwise. The term "antibody" includes a polypeptide of the immunoglobulin family or a polypeptide comprising fragments of an immunoglobulin that is capable of non-covalently, reversibly, and in a specific manner binding a corresponding antigen, preferably human OR10H1, or a variant of human OR10H1, as disclosed herein. An exemplary antibody structural unit comprises a tetramer. In some embodiments, a full length antibody can be composed of two identical pairs of polypeptide chains, each pair having one "light" and one "heavy" chain (connected through a disulfide bond). Antibody structure and isotypes are well known to the skilled artisan (for example from Janeway's Immunobiology, 9th edition, 2016).

The recognized immunoglobulin genes of mammals include the kappa, lambda, alpha, gamma, delta, epsilon, and mu constant region genes, as well as the myriad immunoglobulin variable region genes (for more information on immunoglobulin genes see the international Im-MunoGeneTics information system®, Lefranc M-P et al, Nucleic Acids Res. 2015 Jan;43(Database issue):D413-22; and http://www.imgt.org/). For full-length chains, the light chains are classified as either kappa or lambda. For full-length chains, the heavy chains are classified as gamma, mu, alpha, delta, or epsilon, which in turn define the immunoglobulin classes, IgG, IgM, IgA, IgD, and IgE, respectively. The N-terminus of each chain defines a variable region of about 100 to 110 or more amino acids primarily responsible for antigen recognition. The terms variable light chain (VL) and variable heavy chain (VH) refer to these regions of light and heavy chains respectively. As used in this disclosure, an "antibody" encompasses all variations of antibody and fragments thereof. Thus, within the scope of this concept are full length antibodies, chimeric antibodies, humanized antibodies, single chain antibodies (scFv), Fab, Fab', and multimeric versions of these fragments (e.g., F(ab')2) with the same, essentially the same or similar binding specificity. In some embodiments, the antibody binds specifically to protein of human OR10H1, or of a variant of human OR10H1. Preferred antigen binding constructs according to the invention include an antibody heavy chain, preferably the variable domain thereof, or an antigen binding fragment thereof, and/or an antibody light chain, preferably the variable domain thereof, or an antigen binding fragment thereof. In more preferred embodiments of the invention, the antigen binding fragment binds (such as specifically) to protein of human OR10H1, or of a variant of human OR10H1, and in most preferred embodiments wherein such antigen binding fragment inhibits the expression, function and/or stability of human OR10H1, or the variant of human OR10H1.

In some embodiments of the invention, the (isolated) antigen binding construct comprises the sequences of an antibody heavy chain variable region CDR1, CDR2, and CDR3; and/or the sequences of an antibody light chain variable region CDR1, CDR2, and CDR3. In preferred embodiments the antigen binding constructs of the invention comprise an antibody heavy chain sequence and/or an antibody light chain sequence, or an antigen binding fragment thereof; wherein the antibody heavy chain sequence, or the fragment thereof, comprises a CDR3 having at least 80% (for 8 amino acid long CDR3 sequences preferably 85%) sequence identity to an amino acid sequence selected from SEQ ID Nos. 3, 11, and 19, and/or wherein antibody light chain sequence, or the fragment thereof, comprises a CDR3 having at least 80% (for 8 amino acid long CDR3 sequences preferably 85%) sequence identity to an amino acid sequence selected from SEQ ID Nos. 7, 15, and 23.

In some embodiments the antibody heavy chain sequence of an antigen binding construct of the invention, or the antigen binding fragment thereof, further comprises a CDR1 having at least 80% sequence identity to an amino acid sequence selected from SEQ ID Nos. 1, 9, and 17; and/or a CDR2 having at 80% sequence identity to an amino acid sequence selected from SEQ ID Nos. 2, 10, and 18. In some embodiments the antibody light chain sequence, or the antigen binding fragment thereof, further comprises a CDR1 having at least 80% sequence identity to an amino acid sequence selected from SEQ ID Nos. 5, 13, and 21; and/or a CDR2 having at least 80% sequence identity to an amino acid sequence selected from SEQ ID Nos. 6, 14, and 22. Also in case of CDR1 or CDR2 sequences, the preferred degree of identity may be increased depending on the length of the CDR sequence. Preferred higher degrees of sequence identity include 85%, 90%, and 95%.

In some embodiments the (isolated) antigen binding construct according to the invention comprises an antibody variable chain sequence having at least 80%, preferably 85%, 90%, 95%, or 98% sequence identity to an amino acid sequence selected from SEQ ID Nos. 4, 8, 12, 16, 20, and 24.

In some embodiments the (isolated) antigen binding construct of the invention comprises an antigen binding fragment of an antibody, wherein said antigen binding fragment comprises CDR1, CDR2 and CDR3, preferably which are selected from the CDR1, CDR2 and CDR3 sequences having the respective amino acid sequences of SEQ ID Nos. 1, 2, 3; or 5, 6, 7; or 9, 10, 11; or 13, 14, 15; or 17, 18, 19; or 21, 22, 23; in each case independently, optionally with not more than three or two, preferably one, amino acid substitution(s), insertion(s) and/or deletion(s) compared to these sequences.

In some embodiments of the invention, said CDR1 has an amino acid sequence of SEQ ID No 1, 5, 9, 13, 17 or 21, and CDR2 has an amino acid sequence of SEQ ID No 2, 6, 10, 14, 18, or 22, and CDR3 has an amino acid sequence of SEQ ID No 3, 7, 11, 15, 19, and 23; in each case independently, optionally with not more than three or two, preferably one, amino acid substitution(s), insertion(s) and/or deletion(s) compared to these sequences.

In alternative or additional embodiments of the invention, the (isolated) antigen binding construct is an antibody, or an antigen binding fragment thereof, composed of at least one, preferably two, antibody heavy chain sequences, and at least one, preferably two, antibody light chain sequences, wherein at least one, preferably both, of said antibody heavy chain sequences comprise CDR1 to CDR3 sequences having the amino acid sequences of SEQ ID NO: 1 to 3, and at least one, preferably both, of said antibody light chain sequences comprise CDR1 to CDR3 sequences having the amino acid sequences of SEQ ID NO: 5 to 7; or wherein at least one, preferably both, of said antibody heavy chain sequences comprise CDR1 to CDR3 sequences having the amino acid sequences of SEQ ID NO: 9 to 11, and at least one, preferably both, of said antibody light chain sequences comprise CDR1 to CDR3 sequences having the amino acid sequences of SEQ ID NO: 13 to 15; or wherein at least one, preferably both, of said antibody heavy chain sequences comprise CDR1 to CDR3 sequences having the amino acid sequences of SEQ ID NO: 17 to 19, and at least one, preferably both, of said antibody light chain sequences comprise CDR1 to CDR3 sequences having the amino acid sequences of SEQ ID NO: 21 to 23; in each case of a CDR independently, optionally with not more than three or two, preferably one, amino acid substitution(s), insertion(s) or deletion(s) compared to these sequence.

In alternative or additional embodiments of the invention, an (isolated) antigen binding construct is preferred, wherein the antigen binding construct is an antibody, or an antigen binding fragment thereof, composed of at least one, preferably two, antibody heavy chain sequence, and at least one, preferably two, antibody light chain sequence, wherein said antibody heavy chain sequence comprises a variable region having the amino acid sequence of SEQ ID NO: 4, and wherein said antibody light chain sequence comprises a variable region sequence having the amino acid sequence of SEQ ID NO: 8; or wherein said antibody heavy chain sequence comprises a variable region sequence having the amino acid sequence of SEQ ID NO: 12, and wherein said antibody light chain sequence comprises a variable region sequence having the amino acid sequence of SEQ ID NO: 16; or wherein said antibody heavy chain sequence comprises a variable region sequence having the amino acid sequence of SEQ ID NO: 20, and wherein said antibody light chain sequence comprises a variable region sequence having the amino acid sequence of SEQ ID NO: 24; in each case of a variable region sequence independently, optionally with not more than ten, nine, eight, seven, six, five, four, three, two or one, preferably not more than three, amino acid substitutions, insertions or deletions compared to these sequence.

Preferred embodiments of other aspects of the invention involve an (isolated) antigen binding construct that competes for antigen binding to an antigen binding construct comprising at least one Complementary Determining Region (CDR) 3 having an amino acid sequence with at least 80% sequence identity to an amino acid sequence selected from SEQ ID NOs. 3, 7, 11, 15, 19, and 23. In particular embodiments, such CDR3-comprising antigen binding construct is capable of specifically binding to human OR10H1, or to a variant of human OR10H1, and in more particular embodiments wherein such antigen binding construct inhibits the expression, function and/or stability of human OR10H1, or the variant of human OR10H1. In a preferred embodiment, such an antigen binding construct competes for binding (such as to human OR10H1 protein, or to protein of a variant of human OR10H1) with one or more of the antibodies of the invention described in the example, such competing for binding with 1C3-A1-A1, 1C3-A1-A2 and/or 8A11-B9-A1. In a more preferred embodiment, such as an antigen binding construct competes for binding (such as to human OR10H1 protein, or to protein of a variant of human OR10H1) with the antibody 8A11-H12-E6; and/or with an antibody obtainable from hybridoma Di-8A11-H12-E6 (DSMZ Deposition Number: DSM ACC3310, deposited 26-Oct-2016).

The term "compete" when used in the context of antigen binding constructs (e.g., modulating antigen binding constructs) that compete for the same epitope, means competition between antigen binding constructs as determined by an assay in which the antigen binding construct (e.g., antibody or antigen binding fragment thereof) being tested prevents or inhibits (e.g., reduces) binding of a reference antigen binding fragment (e.g., a reference antibody such as one described in the examples like 8A11-H12-E6/one obtainable from hybridoma Di-8A11-H12-E6, DSMZ Deposition Number: DSM ACC3310, deposited 26-Oct-2016) to a common antigen (e.g., OR10H1 protein or a fragment thereof). Numerous types of competitive binding assays can be used to determine if one antigen binding construct competes with another, for example: solid phase direct or indirect radioimmunoassay (RIA), solid phase direct or indirect enzyme immunoassay (EIA), sandwich competition assay; solid phase direct biotin-avidin EIA etc. Typically, such an assay involves the use of purified antigen bound to a solid surface or cells bearing either of these, an unlabelled test antigen binding construct and a labelled reference antigen binding construct. Competitive inhibition is measured by determining the amount of label bound to the solid surface or cells in the presence of the test antigen binding construct. Usually the test antigen binding construct is present in excess, antigen binding constructs identified by competition assay (competing antigen binding constructs) include antigen binding constructs binding to the same epitope as the reference antigen binding constructs and antigen binding constructs binding to an adjacent epitope sufficiently proximal to the epitope bound by the reference antigen binding construct for steric hindrance to occur. Usually, when a competing antigen binding construct is present in excess, it will inhibit (e.g., reduce) specific binding of a reference antigen binding construct to a common antigen (such as human OR10H1 protein or a fragment thereof) by at least 20%, 30%, 40%, 50%, 55%, 60%, 65%, 70%, 75% or more. In some instances, binding is inhibited by at least 80, 85%, 90%, 95%, or 97% or more, in particular between about 20% and 80%, preferably by at least about 30%, and more preferably by at least about 50%.

In some embodiments the (isolated) antigen binding construct of the invention may comprise in at least one, preferably all, polypeptide chains, antibody constant domain sequences. The origin of the constant domain sequence may be selected from a mouse, rat, donkey, rabbit or human antibody constant domain sequence. The selection of the constant domain is dependent on the intended use of the antigen binding construct of the invention. In some embodiments of the invention the antigen binding construct is chimerized, optionally is humanized or murinized. Preferred embodiments of the invention pertain to antigen binding constructs that comprise a rat heavy chain constant domain selected from constant domain sequences of rat IgG 1, 2a, 2b, 2c, and/or rat light chain constant domain kappa A allele or kappa B allele sequences (see Table E3 below). In preferred embodiments of those antibodies of the invention described in the examples, the IgG subclass of the heavy chain is a rat IgG2a or a rat IgG2b (see Table E3 below), and in more preferred of such embodiments, the IgG subclass of the heavy chain of antibody 1C3-A1-A1, 1C3-A1-A2 or 8A11-B9-A1 is rat IgG2b, and the IgG subclass of the heavy chain of antibody 8A11-H12-E6 is rat IgG2a (in each case, see Table E3).

A preferred embodiment of the invention pertains to a monoclonal antibody as an (isolated) antigen binding construct. An antibody of the invention may be an IgG type antibody, for example having any of the IgG isotypes.

A modulator of human OR10H1 activity, or of the activity of a variant of human OR10H1, that is an antibody can be, for example, an antibody that binds to human OR10H1, or to the variant of human OR10H1, and modulates, such as inhibits, human OR10H1 function, or the function of the variant of human OR10H1, or alters the activity of a molecule that regulates human OR10H1, or the variant of human OR10H1, expression or activity, such that the amount or rate of function of human OR10H1, or of the variant of human OR10H1, or its expression or stability is altered, such as decreased. An antibody useful in a method of the invention can be a naturally occurring antibody format, including monoclonal or polyclonal antibodies or fragments thereof, or a non-naturally occurring antibody format, including but not limited to a single chain antibody, chimeric antibody, bifunctional antibody, complementarity determining region-grafted (CDR-grafted) antibody, CAR, and humanized antibody or an antigen-binding fragment thereof.

In particular embodiments of the invention, the antigen binding construct, such as an antibody, is non-natural and/or is not a product of nature. In one of such embodiments, the antigen binding construct may be a non-natural antigen binding construct, such as a synthetic, modified or recombinant antigen binding construct. In particular, an antigen binding construct of the invention may contain at least one amino acid substitution (or deletion) modification (such as 2, 3, 4, 5, 6, 7, 8, 9, 10 or more than 10 such modifications, in particular between 1 and about 5 such modifications, preferably 2 or 3 such modifications) relative to a product of nature, such as a human antibody or a rabbit antibody (such as a polyclonal rabbit antibody) or a murine or rat antibody. In another of such embodiments, the antigen binding construct may be first generated following non-natural immunization of a (species of) mammal; such as by immunization with an antigen to which such (species of) mammal is not exposed in nature, and hence will not have naturally raised antibodies against.

The anti-ORlOHl antibodies (or the anti-variant of OR10H1 antibodies) of the invention may be monoclonal or polyclonal antibodies. Monoclonal antibodies may be prepared using hybridoma-based methods, such as those described by Kohler and Milstein (1975) Nature 256:495. In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

An immunizing agent typically includes the human OR10H1, or the variant of human OR10H1, protein, or fragments thereof, or a fusion protein thereof. However, antibodies may be prepared by genetic immunization methods in which native proteins are expressed in vivo with normal post-transcriptional modifications, avoiding antigen isolation or synthesis. For example, hydrodynamic tail or limb vein delivery of naked plasmid DNA expression vectors (eg, those encoding protein of human OR10H1 or of a variant of human OR10H1) can be used to produce the antigen of interest in vivo in mice, rats, and rabbits and thereby induce antigen-specific antibodies (Tang et al, Nature 356(6365): 152-4 (1992); Tighe et al, Immunol. Today 19(2) 89-97 (1998); Bates et al, Biotechniques, 40(2) 199-208 (2006); Aldevron-Genovac, Freiburg DE). This allows the efficient generation of high-titre, antigen-specific antibodies which may be particularly useful for diagnostic and/or research purposes. A variety of gene delivery methods can be used, including direct injection of naked plasmid DNA into skeletal muscle, lymph nodes, or the dermis, electroporation, ballistic (gene gun) delivery, and viral vector delivery.

Generally, either peripheral blood lymphocytes ("PBLs") from the immunized host animal are isolated and used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding (1986) Monoclonal Antibodies: Principles and Practice, Academic Press, pp. 59-103). Immortalized cell lines may be transformed mammalian cells, particularly myeloma cells of rodent, bovine, and human origin. Rat- or mouse- myeloma cell lines may be employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high-level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, Calif, and the American Type Culture Collection, Manassas, Va. Human myeloma and mouse-human heteromyeloma cell lines also have been described for the production of human monoclonal antibodies (Kozbor (1984) J. Immunol. 133:3001; Brodeuretal (1987) Monoclonal Antibody Production Techniques and Applications, Marcel Dekker, Inc., New York, pp. 51-631).

The culture medium in which the hybridoma cells are cultured can then be assayed for the presence of monoclonal antibodies directed against human OR10H1 protein (or against protein of the variant of human OR10H1). The binding specificity of monoclonal antibodies produced by the hybridoma cells can be determined by inmunoprecipitation or by an *in vitro* binding assay, such as radioimmunoassay (RIA) or enzyme-linked immunosorbent assay (ELISA). Such techniques and assays are known in the art. The binding affinity of the monoclonal antibody can be determined, for example, by the Scatchard analysis of Munson and Pollard (1980) Anal. Biochem. 107:220. Furthermore, in order to identify antibodies that modulate (for example, inhibit) the expression, function and/or stability of human OR10H1 or of a variant of human OR10H1, the candidate antibodies can be used in the herein described melanoma, CRC or pancreatic tumor TIL screening setup (see example section), or the herein described screening method of the invention. In particular, such antibodies are selected which increase the tumor cell susceptibility to TILs.

After the desired hybridoma cells are identified, the clones may be subcloned by limiting dilution procedures and grown by standard methods. The monoclonal antibodies secreted by the subclones may be isolated or purified from the culture medium or ascites fluid by conventional immunoglobulin purification procedures, such as, protein A-Sepharose, hydroxylapatite chromatography, gel electrophoresis, dialysis, or affinity chromatography.

Monoclonal antibodies of the present invention may also be made by recombinant DNA methods, such as those described in U.S. Pat. No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. The DNA also may be modified, for example, by substituting the coding sequence for human heavy and light chain constant domains in place of the homologous murine sequences (U.S. Pat. No. 4,816,567; Morrison et al., Proc Natl Acad Sci U S A. 1984 Nov; 81(21): 6851-6855.) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

An antibody of the present invention may be a mouse, rat, rabbit, horse, goat, antibody, or a humanized or chimeric antibody. Most preferably, the antibody of the invention has a modulating effect, such as an inhibitory effect, on the immune modulatory function of human OR10H1 (or of a variant of human OR10H1) as described in context of the herein disclosed invention.

Another embodiment of the description pertains to a hybridoma cell capable of producing an antigen binding construct, preferably a monoclonal or polyclonal antibody, as described above.

Yet a further aspect of the disclosure describes a method for producing an antibody against OR10H1, comprising a step of culturing a hybridoma cell of the disclosure and optionally recovering/isolating the antibody from the culture.

Yet, a further aspect of the disclosure provides a nucleic acid encoding for an antigen binding construct as described above. In a preferred embodiment of the disclosure, a nucleic acid of the invention is isolated.

In some embodiments, the polypeptides of the antigen binding constructs can be encoded by nucleic acids and expressed *in vivo* or *in vitro.* Thus, in some embodiments according to the appended set of claims, a nucleic acid, or nucleic acids, encoding an antigen binding construct is provided. In some embodiments, the nucleic acid encodes one part or monomer of an antigen binding construct of the invention (for example one of two chains of an antibody), and another nucleic acid encodes another part or monomer of an antigen binding construct of the invention (for example the other of two chains of an antibody). In some embodiments, the nucleic acid encodes two or more antigen binding construct polypeptide chains, for example, at least 2 antibody chains. Nucleic acids encoding multiple antigen binding construct chains can include nucleic acid cleavage sites between at least two chain sequences, can encode transcription or translation start site between two or more chains sequences, and/or can encode proteolytic target sites between two or more antigen binding construct chains.

Yet, one further aspect of the disclosure provides a vector (such as an expression vector) that comprises a nucleic acid encoding an antigen binding construct as disclosed herein, or a part or monomer of an antigen binding construct. For example, in some embodiments, where the antigen binding construct is a multimeric protein, the nucleic acid encodes only a single polypeptide chain of the antigen construct. Therefore, to express such an antigen binding construct, an expression vector of the disclosure may contain two or more nucleic acids that each encode a separate part or monomer of an antigen binding construct, which in combination would express an entire antigen binding construct. Analogously, an expression vector of the disclosure that comprises a nucleic acid that encodes only part or monomer of an antigen binding construct, may be used in combination with other separate expression vectors of the invention that each encode a separate part or monomer of an antigen binding construct. The nucleic acid of the disclosure may encode multiple polypeptide chains of the antigen binding construct of the invention. The expression vector of the disclosure may include pcDNA3.1™/myc-His (-) Version A vector for mammalian expression (Invitrogen, Inc.) or a variant thereof. The pcDNA3.1 expression vector features a CMV promoter for mammalian expression and both mammalian (Neomycin) and bacterial (Ampicillin) selection markers. The expression vector of the disclosure may include a plasmid. The vector of the disclosure may include a viral vector, for example a retroviral or adenoviral vector. The vector of the disclosure may include a cosmid, YAC, or BAC.

Yet, another further aspect of the disclosure provides a cell line (such as a recombinant cell) which comprises a modulator of the present invention (as described herein, such as an antigen binding construct or antisense molecule of the invention) or at least one nucleic acid or at least one vector according to the invention. Preferably the cell line expresses at least one of the antigen binding constructs described herein. A mammalian cell line of the disclosure (for example, a CHO cell line) may be an expression system to produce the antigen binding constructs as described herein. The antigen binding constructs of the disclosure or fragments thereof described herein may be non-glycosylated, and a mammalian expression system is not required, as such, post- translational modifications are not needed. Thus, one or more of a wide variety of mammalian or non-mammalian expression systems may be used to produce the antigen binding constructs disclosed herein including, but not limited to mammalian expression systems (for example, CHO- Kl cells), bacterial expression systems (for example, *E*. *Coli, B. subtilis*) yeast expression systems (for example, *Pichia, S. cerevisiae*) or any other known expression system. Other systems can include insect cells and/or plant cells.

Furthermore disclosed is a recombinant host cell, comprising a modulator of the present invention (as described herein, such as an antigen binding construct or antisense molecule of the invention), or at least one nucleic acid, or at least one vector according to the invention. The recombinant host cell is preferably a human cell, preferably an autologous human cell. More preferably, the recombinant host cell is cable of expressing an antigen binding construct of the invention.

Another aspect of the disclosure then pertains to a method for producing (such as manufacturing) a recombinant cell line capable of expressing an antigen binding construct of the invention, such as one specific for OR10H1, or for a OR10H1 variant, comprising
(a) providing a suitable host cell,
(b) providing a at least one genetic construct comprising one or more coding sequence that (eg, in combination) encode the antigen binding construct as described herein,
(c) introducing into said suitable host cell said genetic construct(s),
(d) optionally, expressing said genetic construct(s) by said suitable host cell under conditions that allow for the expression of the antigen binding construct, and optionally conditions that allow for assembly of the antigen binding construct.

One further aspect of the disclosure then pertains to a method for producing (such as manufacturing) an antigen binding construct of the invention, such as one specific for OR10H1, or for a OR10H1 variant, said method comprising
(a) providing a hybridoma or host cell capable of expressing an antigen binding construct of the invention, for example a recombinant cell line comprising at least one genetic construct comprising one or more coding sequence(s) that (eg, in combination) encode an antigen binding construct of the invention,
(b) culturing said hybridoma or host cell under conditions that allow for the expression of the antigen binding construct, and optionally conditions that allow for assembly of the antigen binding construct.

Either of these methods of the disclosure may further include the isolation and/or purification of said expressed and optionally assembled antigen binding construct from the host cell. The genetic construct(s) may be an expression construct comprising a promoter sequence operably linked to said coding sequence. Said antigen binding construct may be of mammalian origin, preferably of human origin. Said suitable host cell may be a mammalian cell, optionally a CHO cell. Said antigen binding construct of the disclosure may be a modified antibody, wherein said modification comprises addition of a functional moiety selected from a detectable label or a cytotoxic moiety.

### Antisense Molecules

Insofar the invention pertains to a small interfering RNA (siRNA) for use according to the invention, such siRNA for use comprises all features as defined for the siRNA in appended claim 1.
An anti-sense nucleotide molecule can, by virtue of it comprising an anti-sense nucleotide sequence, bind to a target nucleic acid molecule (eg based on sequence complementarity) within a cell and modulate the level of expression (transcription and/or translation) of OR10H1 (or of a variant of OR10H1).
RNA interference (RNAi) is a process of sequence-specific gene silencing by post-transcriptional RNA degradation or silencing (prevention of translation). RNAi is initiated by use of double-stranded RNA (dsRNA) that is homologous in sequence to the target gene to be silenced. A suitable double-stranded RNA (dsRNA) for RNAi contains sense and antisense strands of about 21 contiguous nucleotides corresponding to the gene to be targeted that form 19 RNA base pairs, leaving overhangs of two nucleotides at each 3' end (Elbashir et al., Nature 411:494-498 (2001); Bass, Nature 411:428-429 (2001); Zamore, Nat. Struct. Biol. 8:746-750 (2001)). dsRNAs of about 25-30 nucleotides have also been used successfully for RNAi (Karabinos et al., Proc. Natl. Acad. Sci. USA 98:7863-7868 (2001). dsRNA can be synthesized in vitro and introduced into a cell by methods known in the art.

A particularly preferred example of an antisense molecule is a small interfering RNA (siRNA) in accordance with the appended claims.

As described above, a modulator that is an siRNA may bind to and directly inhibit or antagonise the expression of mRNA of human OR10H1 (or of a variant of human OR10H1).

The sequence identity of the antisense molecule according to the disclosure in order to target a human OR10H1 mRNA, or an mRNA of a human OR10H1 variant protein (or to target mRNA of a gene controlling expression, function and/or stability of human OR10H1 or the variant), is with increasing preference at least 75%, at least 80%, at least 85%, at least 90%, at least 95%, at least 98%, at least 99% and 100% identity to a region of a sequence encoding the human OR10H1 protein, or variant thereof, as disclosed herein (or of such other controlling gene). Preferably, the region of sequence identity between the target gene and the modulating antisense molecule is the region of the target gene corresponding to the location and length of the modulating antisense molecule. For example, such a sequence identity over a region of about 19 to 21bp of length corresponding to the modulating siRNA molecule). Means and methods for determining sequence identity are known in the art. Preferably, the BLAST (Basic Local Alignment Search Tool) program is used for determining the sequence identity with regard to one or more human OR10H1 RNAs as known in the art. On the other hand, siRNAs of the present invention are preferably chemically synthesized using appropriately protected ribonucleoside phosphoramidites and a conventional RNA synthesizer. Suppliers of RNA synthesis reagents include Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK).

The ability of siRNA to potently, but reversibly, silence genes *in vivo* makes these molecules particularly well suited for use in the pharmaceutical composition of the invention which will be also described herein below. Ways of administering siRNA to humans are described in De Fougerolles et al., Current Opinion in Pharmacology, 2008, 8:280-285. Accordingly, such pharmaceutical compositions may be administered directly formulated as a saline, via liposome based and polymer-based nanoparticle approaches, as conjugated or complexation pharmaceutical compositions, or via viral delivery systems. Direct administration comprises injection into tissue, intranasal and intratracheal administration. Liposome based and polymer- based nanoparticle approaches comprise the cationic lipid Genzyme Lipid (GL) 67, cationic liposomes, chitosan nanoparticles and cationic cell penetrating peptides (CPPs). Conjugated or complexation pharmaceutical compositions comprise PEI-complexed siRNA. Further, viral delivery systems comprise influenza virus envelopes and virosomes.

The siRNAs may comprise modified nucleotides such as locked nucleic acids (LNAs). The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired. Such oligomers are synthesized chemically and are commercially available. The locked ribose conformation enhances base stacking and backbone pre-organization. This significantly increases the hybridization properties (melting temperature) of oligonucleotides. Particularly preferred example of siRNAs is GapmeR (LNA™ GapmeRs (Exiqon)). GapmeRs are potent antisense oligonucleotides used for highly efficient inhibition of human OR10H1 mRNA, or of mRNA of a human OR10H1 variant protein (or of mRNA of a gene controlling expression, function and/or stability of human OR10H1 or the variant). GapmeRs contain a central stretch of DNA monomers flanked by blocks of LNAs. The GapmeRs are preferably 14-16 nucleotides in length and are optionally fully phosphorothioated. The DNA gap activates the RNAse H-mediated degradation of targeted RNAs and is also suitable to target transcripts directly in the nucleus.

Preferred antisense molecules for targeting human OR10H1, or human OR10H1-variant, are antisense molecules or constructs having a sequence complementary to a region (such as one described above) of a nucleic acid sequence of an human OR10H1 mRNA, or of an mRNA of a variant of human OR10H1, preferably a sequence complementary to a region of a sequence encoding the amino acid sequence shown in SEQ ID NO: 25, more preferably, a sequence complementary to a region of between about 15 to 25 bp (such as between about 19 and 21 bp) of a sequence encoding the amino acid sequence shown in SEQ ID NO: 25. Most preferred is an antisense molecule comprising, or consisting essentially of, a sequence according to SEQ ID NO: 26-34; for example an siRNA having a sequence at least 90% identical to a sequence according to any of SEQ ID NO: 26 to 29. In one other preferred embodiment, the modulating siRNA molecule comprises, or consists essentially of, a sequence identical to a sequence according to any of SEQ ID NO: 26 to 29, optionally with no more than three, two or one, most preferably no more than one, nucleotide substitution or deletion compared to such sequence.

In one embodiment the antisense molecules of the invention may be isolated. In another embodiment, the antisense molecules of the invention may be recombinant, synthetic and/or modified, or in any other way non-natural or not a product of nature. For example, a nucleic acid of the invention may contain at least one nucleic acid substitution (or deletion) modification such as between 1 and about 5 such modifications, preferably no more than 1, 2 or 3 such modifications) relative to a product of nature, such as a human nucleic acid. As described above, the antisense molecules of the invention may be modified by use of non-natural nucleotides, or may be conjugated to another chemical moiety. For example, such chemical moieties may be a heterologous nucleic acid conferring increased stability or cell/nucleus penetration or targeting, or may be a non-nucleic acid chemical moiety conferring such properties, of may be a label.

### Treatment Methods and Diseases

A method of treatment with respect to compounds or compositions for use of the invention preferably, comprises a step of contacting a cell with a modulator of human OR10H1, or variant of human OR10H1, expression, stability and/or function, more preferably contacting a tumor or cancer cell with an inhibitor of human OR10H1, or variant of human OR10H1, expression, stability and/or function. In context of the invention, it was surprisingly found that human OR10H1 mediates melanoma, pancreatic and colorectal tumor cell resistance against cytotoxic T lymphocytes (CTL) by inhibiting tumor infiltrating lymphocyte (TIL) viability and/or activity. Therefore, the present invention for the first time indicates a method for reducing resistance of a tumor or cancer cell to CTL responses by impairing human OR10H1 expression, function and/or stability in the tumor. Thus, it is preferred that said tumor or cancer cell is characterized by a detectable cell surface expression of human OR10H1 (or of a variant of human OR10H1) before contacting the cell with an inhibitor of expression, function and/or stability of human OR10H1, or of a variant of human OR10H1. More preferred is that the tumor or cancer is characterized by a resistance of the tumor or cancer against autologous or heterologous T-cell mediated immune responses, and/or is a refractory/recurrent tumor or cancer, a metastatic tumor or a multidrug resistant tumor or cancer.

A disease treatable by the compounds and methods of the disclosure includes one or more proliferative diseases. "Proliferative disease" refers to a disease characterized by abnormal proliferation of cells. A proliferative disease does not imply any limitation with respect to the rate of cell growth, but merely indicates loss of normal controls that affect growth and cell division. Thus, in some embodiments of the disclosure, cells of a proliferative disease can have the same cell division rates as normal cells but do not respond to signals that limit such growth. Within the ambit of "proliferative disease" is neoplasm or tumor, which is an abnormal growth of tissue. Cancer refers to any of various malignant neoplasms characterized by the proliferation of cells that have the capability to invade surrounding tissue and/or metastasize to new colonization sites.

A disease treatable by the compounds and methods of the invention is preferably one characterised by a resistance of one or more cells affected by the disease (such as the proliferative disease) against a defence response of the host organism, such as resistance that is associated with a pathological phenotype. A preferred example is a resistance of a tumor or cancer against one or more immune responses, in particular cell-mediated immune response, mounted by the subject/patient suffering from the tumor or cancer. A disease treatable by the compounds and methods of the invention is more preferably one characterised by expression of human OR10H1 (or expression of a variant of human OR10H1); in particular one characterised by such expression that is aberrant, for example over- (or under-) expression on a given cell or tissue (such as those cells or tissues associated with the proliferative disease of the subject) compared to that in a healthy subject or a normal cell.

The term "resistance" refers to an acquired or natural resistance of a cell of a proliferative disease, such as tumor or cancer cell, to a patient's own immune response (such as a cell-mediated immune response), or to immune responses aided by immune therapy such as adoptive T-cell transfer. Therefore, a resistant tumor or cancer cell is more likely to escape and survive humoral and/or cellular immune defense mechanisms in a subject having the tumor or cancer. A treatment of a resistant proliferative disease, such as tumor/cancer resistance, in context of the invention shall be effective if compared to a non-treated control, the disease cell (such as a cell of the tumor of cancer) becomes more sensitive or susceptible to an immune response (such as a cell-mediated immune response) - in other words will be more likely to be "identified" and neutralized by a patient's immune response.

The term "tumor resistance" is a resistance to a cell-mediated immune response, such as cytotoxic T lymphocyte (CTL) response (i.e., the tumor or tumor cell being nonresponsive to, or having reduced or limited response to a CTL targeting a tumor cell). A tumor cell may show a reduced sensitivity when contacted with a CTL specific for an antigen expressed on that tumor cell. A reduced sensitivity is a reduction to a 90% cytotoxic T cell response, preferably a reduction to 80%, 70%, 60%, 50% or more preferably a reduction to 40%, 30%, 20% or even less. In this case, 100% would denote the state wherein the CTLs can kill all of the cells in a cancer sample. Whether or not a tumor cell is resistant to a patient's (cell-mediated) immune response may be tested in-vitro by contacting autologous tumor cell with autologous T-cells and thereafter quantifying the survival/proliferation rate of the tumor cells. As an alternative, the reduction in (cell-mediated) immune response is determined by comparing cancer samples of the same cancer before and after the resistance is acquired (for example induced by therapy), or by comparing with a cancer sample derived from a different cancer which is known to have no resistance to the CTL. On the other hand, the treatments of the present invention include the sensitization of tumor cells against CTL and therefor to decrease tumor cell resistance. A decrease of tumor cell resistance against CTL is preferably a significant increase of CTL toxicity, preferably a 10% increase, more preferably 20%, 30%, 40%, 50%, 60%, 70%, 80% or more, even more preferably 2 fold increase, 3 fold, 4 fold, 5 fold or more.

A proliferative disease in context of the invention may also be a tumor or cancer disease, selected from a liquid or solid tumor, and preferably is lung cancer, bladder cancer, ovarian cancer, uterine cancer, endometrial cancer, breast cancer, liver cancer, pancreatic cancer, stomach cancer, cervical cancer, lymphoma, leukemia, acute myeloid leukemia, acute lymphocytic leukemia, salivary gland cancer, bone cancer, brain cancer, colon cancer, rectal cancer, colorectal cancer, kidney cancer, skin cancer, melanoma, squamous cell carcinoma, pleomorphic adenoma, hepatocellular carcinoma, and/or adenocarcinoma. More preferably, the disease is one selected from the list consisting of: melanoma, pancreatic cancer and colorectal cancer. Most preferably, the disease is characterized by the expression of human OR10H1, or of a variant of human OR10H1, in a cell associated with the pathology of the disease. A preferred disease is in some embodiments an human OR10H1- or human OR10H1 variant protein- expressing tumor or cancer.

The compounds of the invention are preferably for use in aiding a cell-mediated immune response in a subject, such as aiding a patient's T-cell mediated immune response, against a proliferative diseases such as a tumor or cancer disease. For example, the compounds of the invention can be for use in a treatment that includes a transfer of cells such as a transfer of immune cells to the patient, in particular the compounds of the invention can be for use in a treatment that include adoptive T-cell transfer based therapy of cancer. The cells transferred in such treatment may include those being autologous cells of the subject, for example autologous immune cells, such as T-cells or Natural Killer (NK)-cells, of the subject.

As mentioned above, in certain embodiments the compound of the invention is an inhibitor or antagonist of expression, function and/or stability of human OR10H1, or the variant protein thereof. In one particular of such embodiments, the inhibition of the expression, function and/or stability of said human OR10H1, or the variant of human OR10H1, enhances an immune response, preferably enhances a cell-mediated immune response in a subject (such as a patient), and more preferably enhances a T-cell mediated immune response in the subject. Such an enhancement of immune response can be used for treating a proliferative disease, for example treating a cancer disease. In another particular of such embodiments of the disclosure, the inhibition of the expression, function and/or stability of human OR10H1, or the variant protein thereof, in the disease of a subject (such as the patient), such as a cancer, enhances an immune response (such a cell-mediated immune response) in the subject; in particular where such an enhancement may be associated with an increases T-cell activity and/or survival in said patient. In this context the T-cells may be endogenous T-cells, or transferred autologous or foreign T-cells.

It will now be apparent to the person of ordinary skill, given the present invention is related to the surprising finding of human OR10H1-mediated resistance to one or more immune responses, that an "enhancement" or an "increase" in immune response (such as a cell-based immune response), in particular an increase in T-cell activity and/or survival, may be an increase or enhancement relative to that mediated (eg reduced) by this newly described human OR10H1 activity (eg the resistance). Accordingly, the "enhancement" or "increase" (and like terms) may equally be considered a "release" of the reduction of an immune response mediated by human OR10H1 (and reflected by the resistance to the immune response), such that the immune response resulting from inhibition of human OR10H1 (or of the variant of human OR10H1) expression, function and/or stability, may be so enhanced or increased relative to that in the subject prior to such inhibition, but the resulting immune response may also be considered to be at a level (after such human OR10H1 inhibition) equivalent to that typically present in a healthy subject.

The increase in a subject's T-cell activity and/or T cell survival can, in certain embodiments of the invention, be associated with an inhibition of cAMP signaling mediated by OR10H1 or by the variant of OR10H1. In further embodiments, the increase in T-cell activity and/or survival may be associated with reduced phosphorylation of cAMP response element-binding protein (CREB),; and/or with activation of lymphocyte-specific protein tyrosine kinase (Lck), for example by reduced phosphorylation of the LcK Tyr505 domain in such immune cell(s). In some of such embodiments, the increase in said T-cell activity and/or survival is associated with reduced phosphorylation of protein kinase A (PKA) in said immune T-cells. In further of such embodiments the increase in said T-cell activity and/or survival is associated with a reduction in the level of cAMP in said T-cells. The term "associated with", in the context of such and other embodiments can mean that two variables, effects or phenotypes are correlated to each other, that they are related to each other, or that there is some kind of causative link between a first variable, effect or phenotype and the second such as the second is in response to the first, the second is a consequence of the first, or the second is caused by the first.

### Detection, Diagnosis and Screening

The object of the invention is furthermore solved by an in vitro method for diagnosing a resistance phenotype of a cancer disease against an immune response in a human subject, the method comprising the steps of
(a) Providing a sample of tumor or cancer cells of the subject,
(b) Determining the presence or absence of protein or mRNA of human OR10H1, or of a variant of human OR10H1, in the tumor or cancer cells, wherein the variant of human OR10H1 is a protein comprising an amino acid sequence having at least 95% sequence identity to the sequence of SEQ ID NO: 25, and
(c) Diagnosing a resistance phenotype of the cancer disease against an immune response in the subject when protein or mRNA of said human OR10H1, or of the variant of human OR10H1, is present in the tumor or cancer cells.

In certain embodiments, the phenotype to be diagnosed by the method of the invention is one of an increased resistance phenotype, and/or one that is a resistance to a cell-mediated immune response such as a T-cell immune response.

In further certain embodiments of the diagnostic methods the presence of an amount or level of said protein or mRNA of OR10H1, or of the variant of OR10H1, is determined in step (b) as a measured amount.

In preferred embodiments, the presence of an amount or level of said protein or mRNA of human OR10H1, or of the variant of human OR10H1, in the tumor cells in step (b) is a measured amount of said protein or mRNA of human OR10H1, or of the variant of human OR10H1, which if greater than: (i) a measured quantity of said protein or mRNA of human OR10H1, or of the variant of human OR10H1, in control cells; and/or (ii) a standard or cutoff value for said quantity of said protein or mRNA of human OR10H1, or of the variant of human OR10H1, then the diagnosis in step (c) is made.

In some embodiments step (b) of the diagnostic method of the invention involves contacting the sample with means for the detection of protein or mRNA of human OR10H1, or of a variant of human OR10H1, wherein said means is preferably selected from: (i) an antigen binding construct specifically binding to said protein of human OR10H1, or of the variant of human OR10H1, and detecting the binding between said antigen binding construct and said protein of human OR10H1, or of the variant of human OR10H1; and/or (ii) a nucleic acid binding to said mRNA sequence of human OR10H1, or of the variant of human OR10H1, and detecting the binding between said nucleic acid and said mRNA of human OR10H1, or of the variant of human OR10H1. Preferably, the antigen binding construct is an antigen binding construct as described herein before.

In some embodiments, such detection and diagnostic methods may be a computer-implemented method, or one that is assisted or supported by a computer. In some embodiments, information reflecting the presence/absence or an amount of human OR10H1, or of the variant thereof, in a sample is obtained by at least one processor, and/or information reflecting the presence/absence or an amount of the human OR10H1 or variant thereof in a sample is provided in user readable format by another processor. The one or more processors may be coupled to random access memory operating under control of or in conjunction with a computer operating system. The processors may be included in one or more servers, clusters, or other computers or hardware resources, or may be implemented using cloud-based resources. The operating system may be, for example, a distribution of the Linux™ operating system, the Unix™ operating system, or other open-source or proprietary operating system or platform. Processors may communicate with data storage devices, such as a database stored on a hard drive or drive array, to access or store program instructions other data. Processors may further communicate via a network interface, which in turn may communicate via the one or more networks, such as the Internet or other public or private networks, such that a query or other request may be received from a client, or other device or service.

Therefore, the present disclosure also provides a kit (such as a detection and/or diagnostic kit) comprising means for the determination of the presence or absence of OR10H1 (or of a variant of OR10H1), such as in a cell associated with a proliferative disease such as a tumor or cancer cell. The diagnostic kit is suitable for diagnosing an absent or decreased immune susceptibility of a proliferative disease to an immune response, such as of a tumor or cancer towards a cell-mediate immune response (eg that mediated T-cells). The kit may preferably comprise specific and selective anti-ORlOHl antibodies as described herein before. Alternatively, the diagnostic kit may comprise nucleic acid primers and/or probes for detecting the expression of OR10H1 in a tumor cell. The kit as described may include other known means for detecting OR10H1 protein expression. The kit may further comprise instructions for use and/or with one or more additional components useful for said detection. Such instructions may consist of a printed manual or computer readable memory comprising such instructions, or may comprise instructions as to identify, obtain and/or use one or more other components to be used together with the kit. Such additional component may comprise one or more other item, component, reagent or other means useful for the use of the kit or practice of a detection method of the invention, including any such item, component, reagent or means disclosed herein useful for such practice. For example, the kit may further comprise reaction and/or binding buffers, labels, enzymatic substrates, secondary antibodies and control samples, materials or moieties etc.

In preferred embodiments of the kit of the description or the diagnostic methods, the means for the detection of protein or mRNA of OR10H1, or of a variant of OR10H1, is labeled, for example is coupled to a detectable label. The term "label" or "labelling group" refers to any detectable label. In general, labels fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

In another aspect, the disclosure relates to a method of diagnosing and treating a proliferative disease, such as one characterised by (aberrant) expression of OR10H1, or of a variant of OR10H1 (such as a tumor or cancer disease) in a subject, such as a patient, comprising:
(a) Providing a (biological) sample from said subject, said sample comprising cells or tissue of said subject, or an extract of said cells or tissue; and
(b) Detecting whether OR10H1 or the variant is present in the biological sample;
(c) Diagnosing the subject with the disease depending on if the presence of OR10H1 or the variant in the biological sample is detected; and
(d) Administering an effective amount of an inhibitor of the expression, function and/or stability of OR10H1 (or of a variant of OR10H1) function to the diagnosed subject, preferably an inhibiting or antagonistic compound of the invention as described herein.

In another aspect of the invention, an in vitro method is provided for identifying a compound suitable for the treatment of a disease characterized by expression of human OR10H1, or a variant thereof, wherein the variant of human OR10H1 is a protein comprising an amino acid sequence having at least 95% sequence identity to the sequence of SEQ ID NO: 25, the method comprising the steps of
(a) Providing a first cell expressing a protein or mRNA of said human OR10H1, or of the variant of human OR10H1, wherein said first cell is a tumour cell or a cell derived from a tumour, and
(b) Providing a candidate compound, and
(c) Optionally, providing a second cell which is a cytotoxic immune cell, for example a cytotoxic T-lymphocyte (CTL), capable of immunologically recognizing said first cell, and
(d) Bringing into contact the first cell and the candidate compound, and optionally the second cell, and
(e) Determining subsequent to step (d), either or both of:
   (i) expression, function and/or stability of said protein or mRNA of said human OR10H1, or of the variant of human OR10H1, in said first cell, wherein a reduced expression, function and/or stability of said protein or mRNA of said human OR10H1, or of the variant of human OR10H1, in said first cell contacted with the candidate compound compared to said first cell not contacted with said candidate compound indicates that the candidate compound is a compound suitable for the treatment of a disease characterized by expression of said protein or mRNA of said human OR10H1, or of the variant of human OR10H1; and/or
   (ii) cytotoxicity of said second cell against said first cell, wherein an enhanced cytotoxicity of said second cell against said first cell contacted with the candidate compound compared to the cytotoxicity of said second cell against said first cell not contacted with the candidate compound indicates that the candidate compound is a compound suitable for the treatment of a disease characterized by expression of said human OR10H1, or the variant of human OR10H1.

The reduction (or enhancement) of expression, function and/or stability or human OR10H1 (or of the variant thereof), or the enhancement (or reduction) in cytotoxicity is, preferably, identified by reference to a control method, for example one practiced in the absence of any candidate compound, or with compound having a known effect on such expression, function and/or stability (such as a positive or negative control).

Said candidate compound is selected from a polypeptide, peptide, glycoprotein, a peptidomimetic, an antigen binding construct (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tra-crRNA.

### Pharmaceutical Compositions

Another aspect of the present invention pertains to a pharmaceutical composition for use in the prevention or treatment of a tumor disease according to the appended set of claims. The pharmaceutical composition of the invention comprises a compound of the invention, and a pharmaceutical acceptable carrier and/or excipient.. Also provided by the invention are pharmaceutical compositions, comprising the (isolated) antigen binding construct, or the (isolated) nucleic acid(s), , according to the appended set of claims; and a pharmaceutically acceptable carrier, stabilizer and/or excipient.

As used herein the language "pharmaceutically acceptable" carrier, stabilizer or excipient is intended to include any and all solvents, solubilizers, fillers, stabilizers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration.

Solutions or suspensions used for parenteral, intradermal, or subcutaneous application, as well as comprising a compound of the invention, can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Kolliphor® EL (formerly Cremophor EL™; BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the compound of the invention in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions, as well as comprising a compound of the invention, generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and ex-pectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring. Furthermore, the compounds of the invention can be administrated rectally. A rectal composition can be any rectally acceptable dosage form including, but not limited to, cream, gel, emulsion, enema, suspension, suppository, and tablet. One preferred dosage form is a suppository having a shape and size designed for introduction into the rectal orifice of the human body. A suppository usually softens, melts, or dissolves at body temperature. Suppository excipients include, but are not limited to, theobroma oil (cocoa butter), glycerinated gelatin, hydrogenated vegetable ails, mixtures of polyethylene glycols of various molecular weights, and fatty acid esters of polyethylene glycol.

For administration by inhalation, the compounds of the invention are typically delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions can be formulated into ointments, salves, gels, or creams as generally known in the art.

In certain embodiments, the pharmaceutical composition is formulated for sustained or controlled release of a compound of the invention. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially (including liposomes targeted to infected cells with monoclonal antibodies to viral antigens) can also be used as pharmaceutically acceptable carriers. These can be prepared according to methods known to those skilled in the art.

It is especially advantageous to formulate oral, rectal or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage of the compound of the invention for use in humans. The dosage of such compounds lies preferably within a range of circulating concentrations that include the ED₅₀ with little or no toxicity. The dosage may vary within this range depending upon the dosage form employed and the route of ad-ministration utilized. For any compound used in the therapeutic approaches of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

### In the Figures:

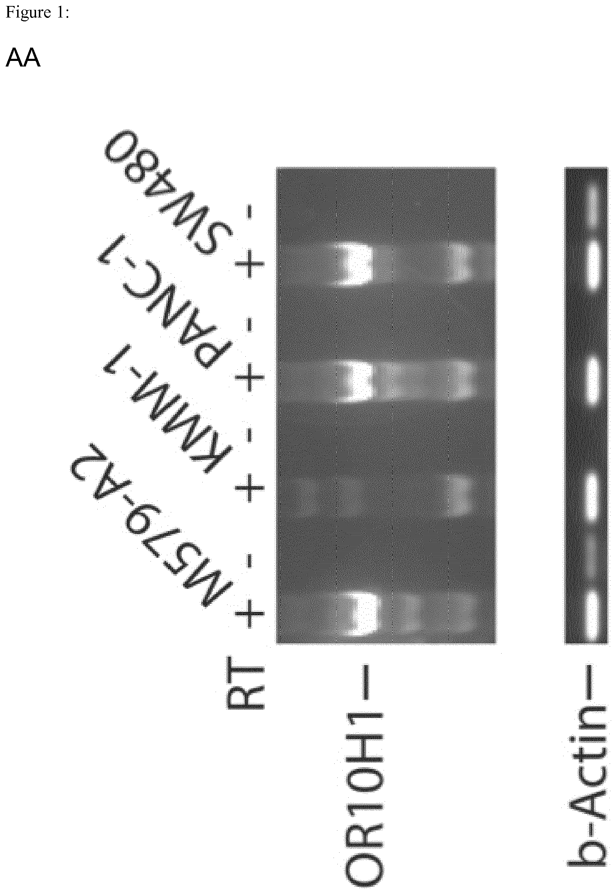

**Figure 1AA****: OR10H1 is expressed by various cancer cell-lines.** RT-PCR shows expression of OR10H1 by the cell lines: M579-A2 (melanoma), PANC-1 (PDAC) and SW480 (colorectal), but not by the myeloma cell line KMM-1.

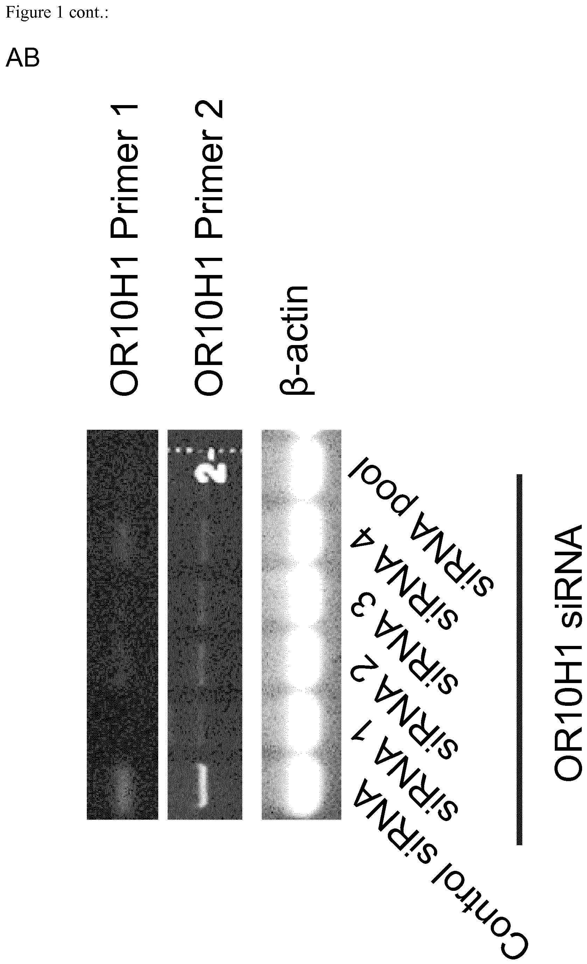

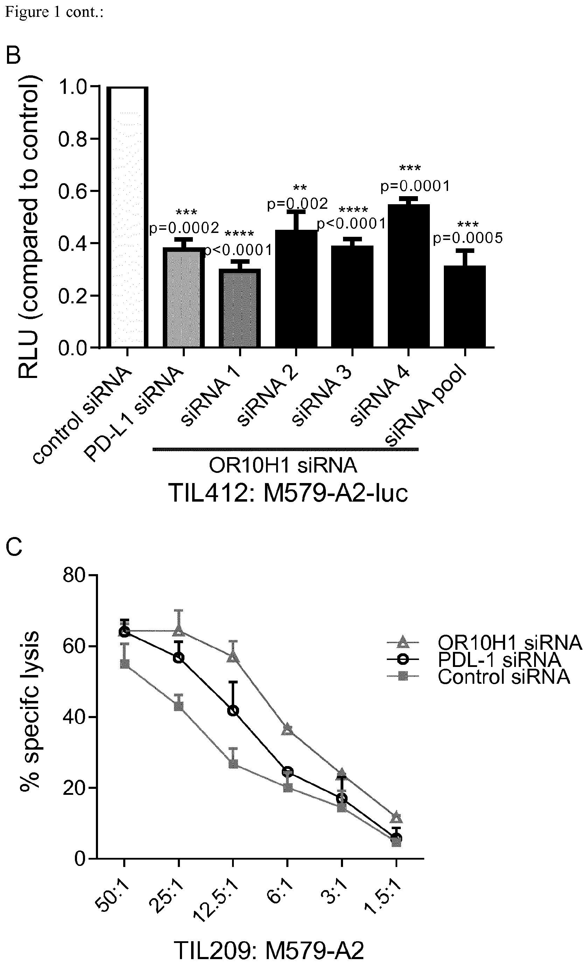

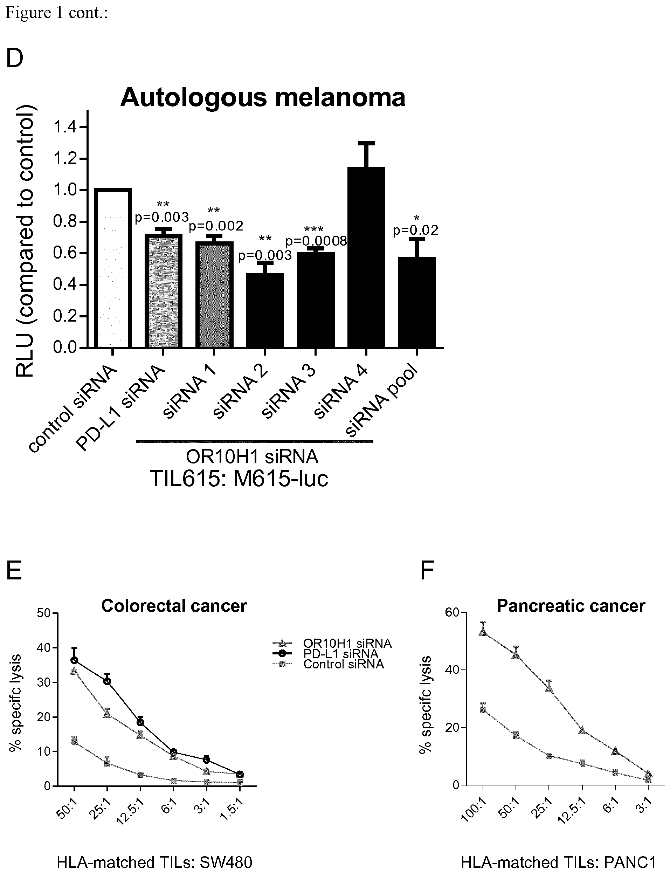

**Figure 1** **cont.: OR10H1 prevents lysis of solid tumors by TILs. AB** M579-A2 cells were transfected with the siRNA sequences for OR10H1 and mRNA was measured by RT-PCR after 72 h. Beta-actin served as a control. **B, C** Killing assays of M579-A2 with two different TIL-cultures. **B** M579-A2-luc cells, which were transfected with individual (s1-s4), pooled OR10H1 siRNA or control siRNA (PD-L1 as positive and non-specific as negative control) and TIL-mediated lysis was measured by Luc-CTL cytotoxicity assay (cumulative data,). C Chromium-release assay showing specific lysis of M579-A2 by TIL209 at different E:T ratios after transfection with OR10H1 siRNA 1 (Δ), positive control PD-L1 siRNA (o) or control siRNA (■). **D** Luc-CTL assay with an autologous pair of melanoma (M615-luc) and TIL (TIL615) conducted in parallel to C (cumulative data,). **E, F** Chromium-release assay showing, respectively lysis of SW480 colorectal cancer and PANC-1 pancreatic adenocarcinoma cells by patient-derived HLA-matched TILs at different E:T ratios upon OR10H1 (Δ), PD-L1 positive control (○) or control (■) knockdown. All experiments were performed in triplicates and are representative (if not indicated otherwise) of at least three independent experiments. Error bars denote ± SEM, and statistical significance was calculated using unpaired, two-tailed Student's t-test.

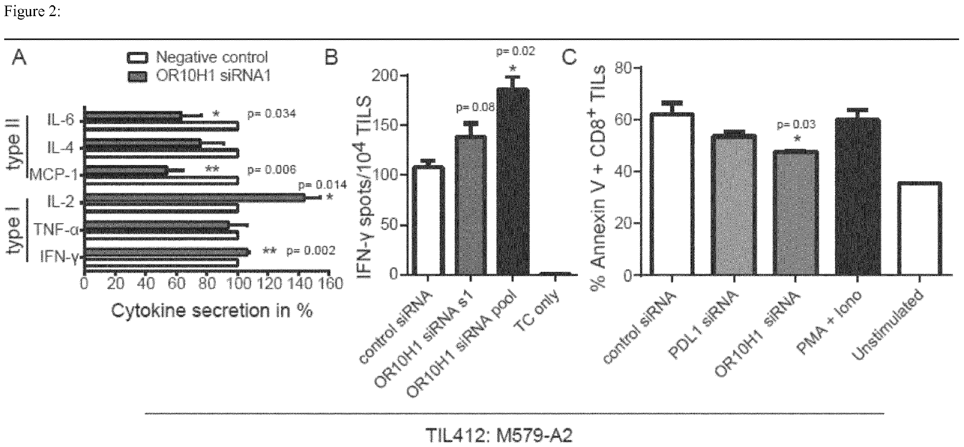

**Figure 2****: OR10H1 inhibits TIL type I cytokine secretion and induces apoptosis. A** Cumulative data of type I vs. type II cytokine secretion of TIL412 after 20 h co-culture with OR10H1 knock-down (siRNA1 or "s1") or OR10H1 expressing (control siRNA) M579-A2 measured by Luminex assay. **B** ELISpot assay showing the number of IFN-γ secreting TILs (spot numbers) after co-culture with OR10H1 knockdown M579-A2 (or control siRNA). TILs without stimulation were used as a negative control. C cumulative data of apoptosis induction (measured by FACS staining for Annexin V⁺) in CD8⁺ TILs after co-culture (6h) with OR10H1 knock-down (siRNA1 or siRNA pool) or OR10H1 expressing (control siRNA) M579-A2. TILs stimulated with PMA/ionomycin and unstimulated TILs (no melanoma encounter) were used as positive and negative controls, respectively. A and C show cumulative data from three independent experiments. B shows representative data of two independent experiments (performed in triplicates). Error bars denote ± SEM, and statistical significance was calculated using unpaired, two-tailed Student's t-test.

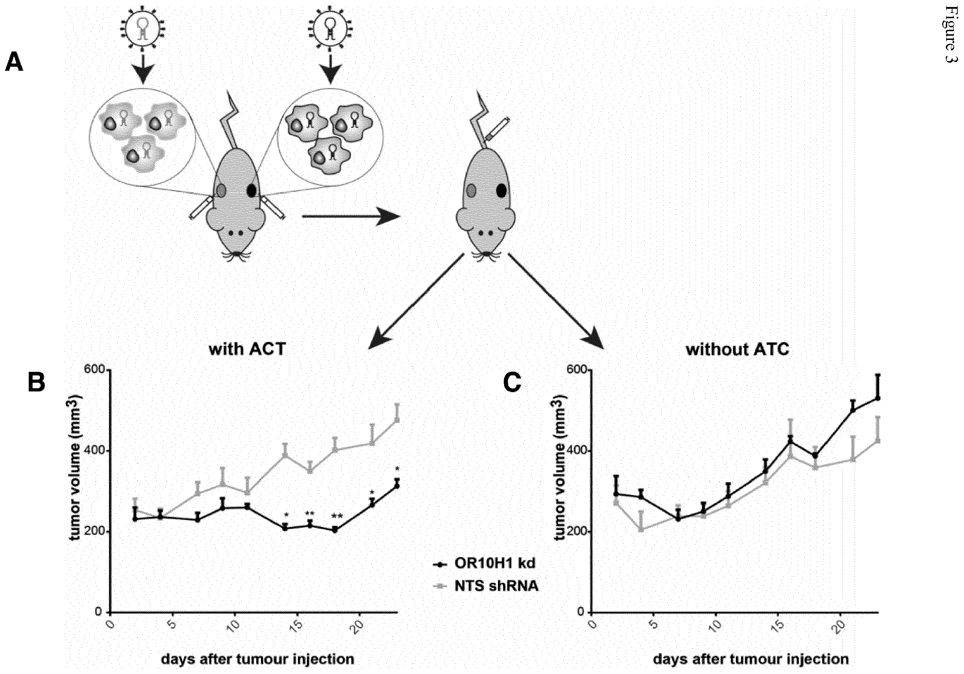

**Figure 3****: OR10H1 functions as an immune checkpoint *in vivo.* A** Stable OR10H1 knockdown M579-A2 (transduced with OR10H1-targeting shRNA) or control transduced M579-A2 (non-targeting sequence shRNA; NTS) were injected subcutaneously (mixed with matrigel) into the left and right flank of NSG mice. On day 2 and 9, mice (n = 6) received adoptive cell transfer of TIL412 (i.v. injection) and the tumor growth was measured. Mice (n = 4) with tumors but without ACT serve as a control group for tumor growth. **B, C** Tumor growth curves showing mean ± SEM tumor volume of OR10H1-negative (ie OR10H1 knockdown; kd) or OR10H1 -positive (ie NTS shRNA control) M579-A2 tumors in TIL412-treated mice, ie, with ACT (**B**), or in the growth control group without ACT (C). Statistical difference was calculated using the Mann-Whitney U test.

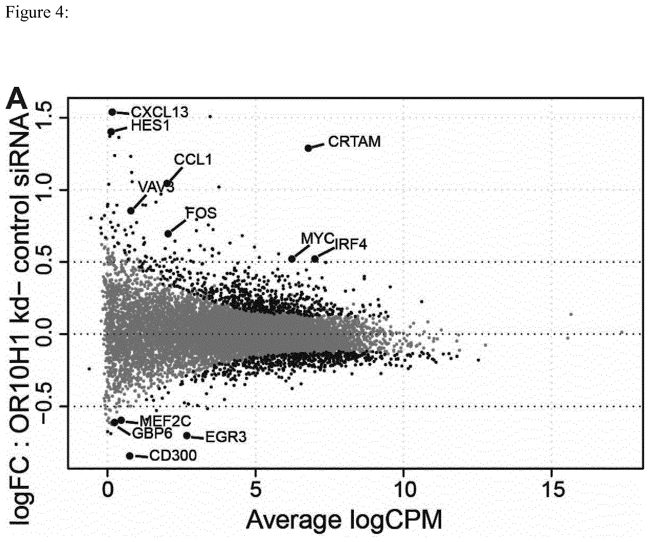

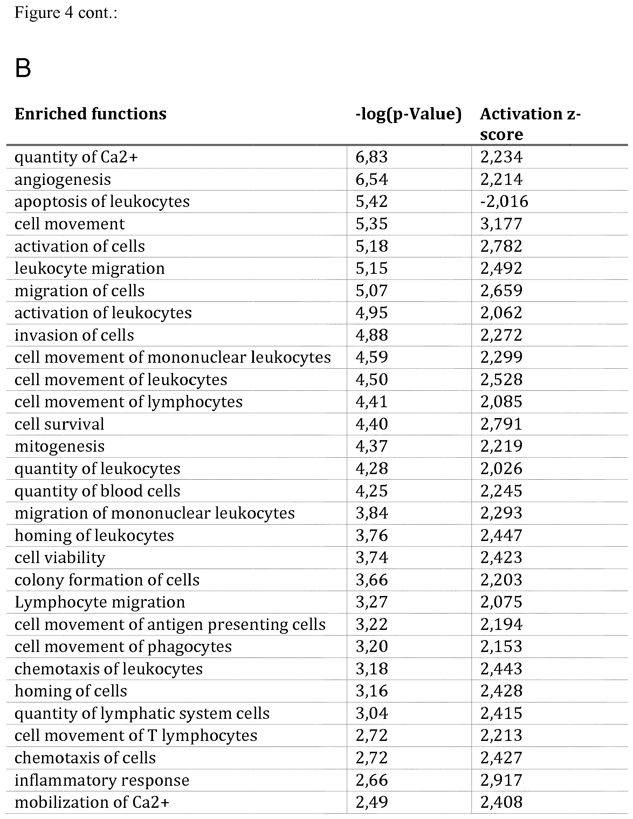

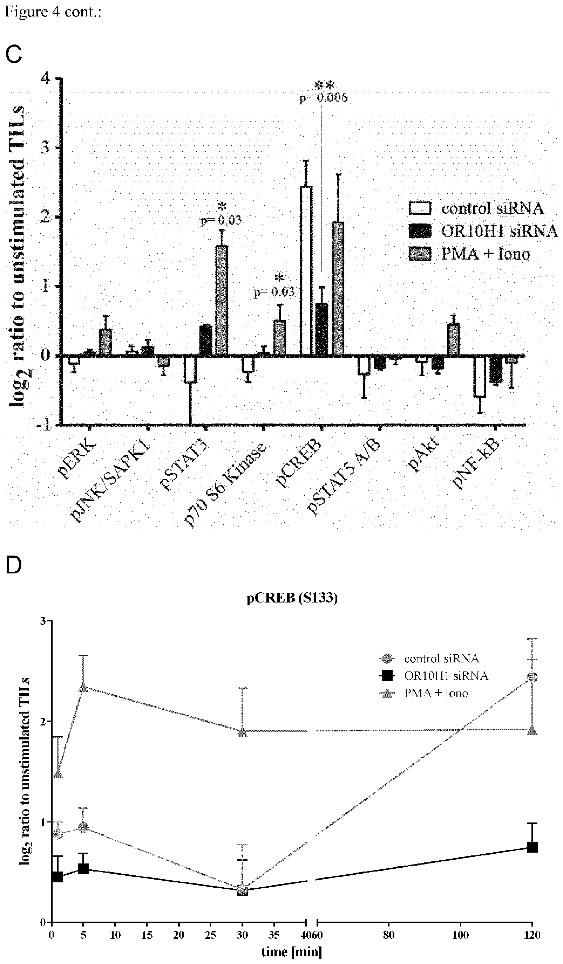

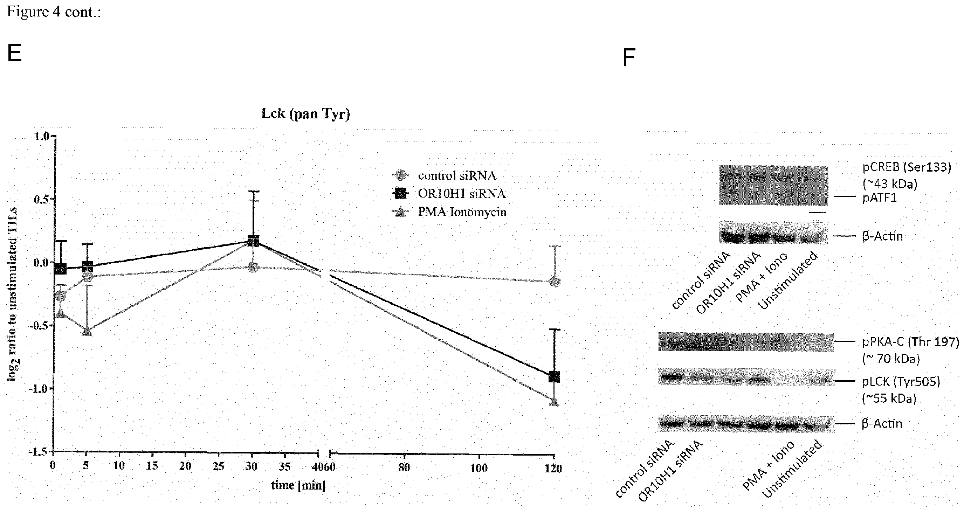

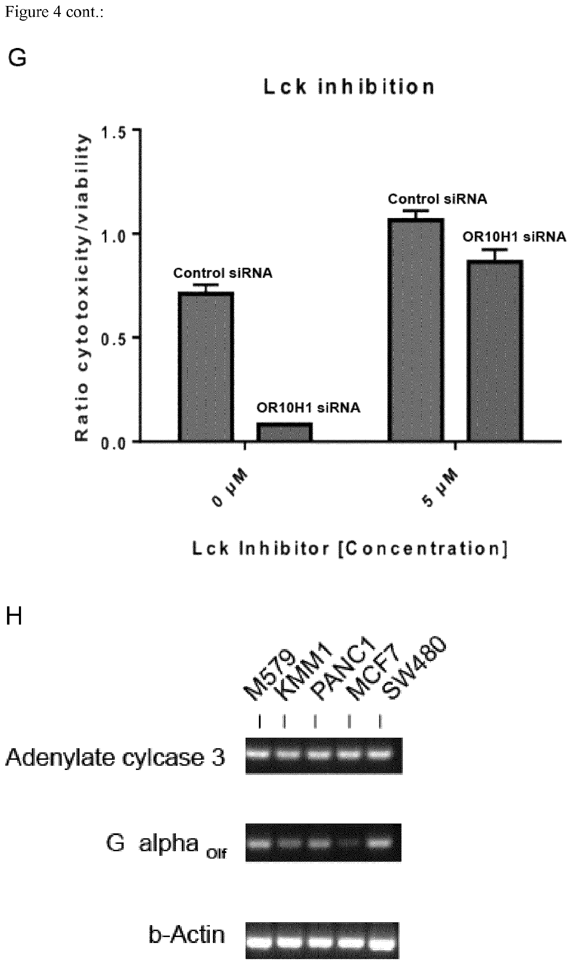

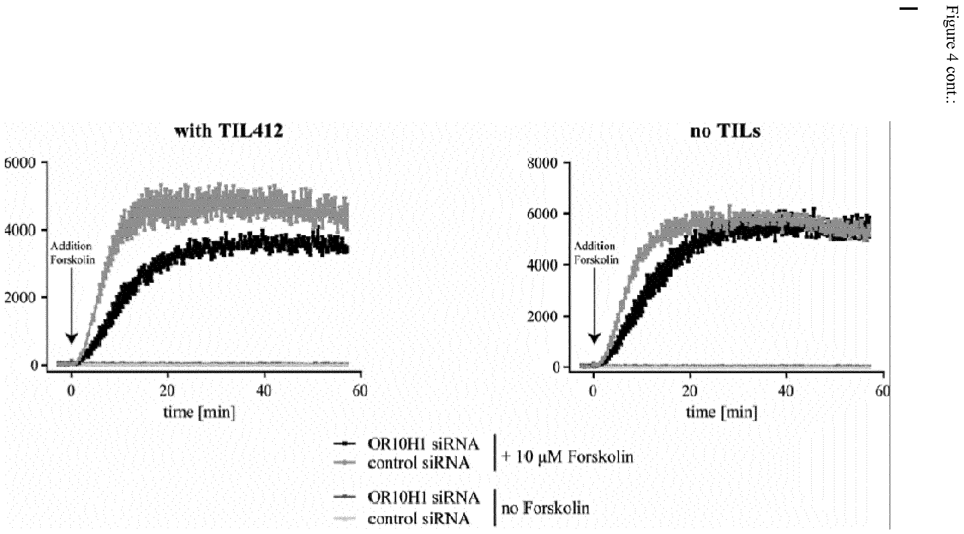

**Figure 4****: OR10H1 reduces Lck and increases CREB activity by cAMP signaling via PKA in TILs. A, B** TIL412 were co-cultured with OR10H1-positive (control siRNA) or OR10H1-negative (OR10H1 siRNA) M579-A2 cells for 10 h and mRNAs were extracted from the respective TILs for transcriptome analysis by RNA sequencing. A Smear plot showing differential gene expression (log2 of fold change) between OR10H-negative (kd)- and OR10H1-postive, control siRNA)- tumor cell-treated TILs versus the average expression (log of count per million). LogFC cutoff at ± 0.5 is represented by horizontal lines. Genes with a false discovery rate (FDR) above 0.05 (depicted in gray) were excluded from subsequent analysis. **B** Functional enrichment analysis (Ingenuity pathway analysis; IPA) based on top upregulated (LogFC > 0.5) and downregulated (LogFC < -0.5) genes with FDR ≤ 0.05. Differential gene expression-associated functions were selected based on enrichment p-value (-log of p-value; threshold = -1.3) and activation Z-score (threshold ±2). RNA-Sequencing was performed in duplicates. **C, D, E, F** Phospho pathway analysis for key signaling proteins in TILs after co-culture (up to 2 h) with OR10H1-positive (ie control siRNA) and OR10H1-negative (ie OR10H1 siRNA) M579-A2. Phosphoplex analysis showing phosphorylation state changes in TILs upon encountering OR10H1-positive- or OR10H1-negative M579-A2 tumor cells (log2 ratio) of classical **(C)** or T cell receptor (TCR) -associated **(D, E)** signaling proteins (CREB and Lck) compared to unstimulated TILs. In each case, TILs stimulated with PMA and ionomycin serve as positive control. **F** Immunoblot analysis showing phospho-CREB (Ser133), phospho-ATFl, phospho-PKA (Thr197) and phospho-Lck (Tyr505) levels in OR10H1-positive tumor cell-treated, OR10H1-negative tumor cell-treated, PMA and ionomycin-treated or unstimulated TILs using the according phospho-specific antibodies. Beta-actin was used as a loading control. C, D and E are cumulative data of three independent experiments. F is representative of three independent experiments. Mean ± SEM are shown, unless stated otherwise, and statistical significance was calculated using unpaired, two-tailed Student's t-test. **G** Lck inhibition by a small molecule abrogates OR10H1 knockdown effect on T-cell mediated cytotoxicity (as reflected the ratio of cytotoxicity/viability) of melanoma cells. **H** Western blot showing that olfactory receptor signalling activates a unique G protein (G-alpha-Olf) and subsequently adenylate cyclase type III. **I** In the presence of TILs the cAMP response in M579-A2 cells is reduced if OR10H1 is knocked-down on the melanoma cells.

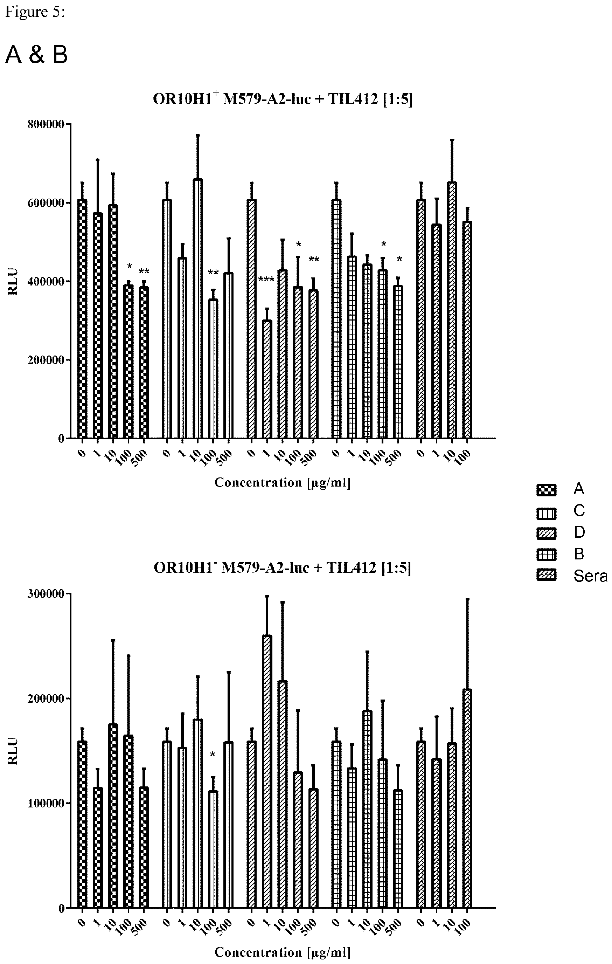

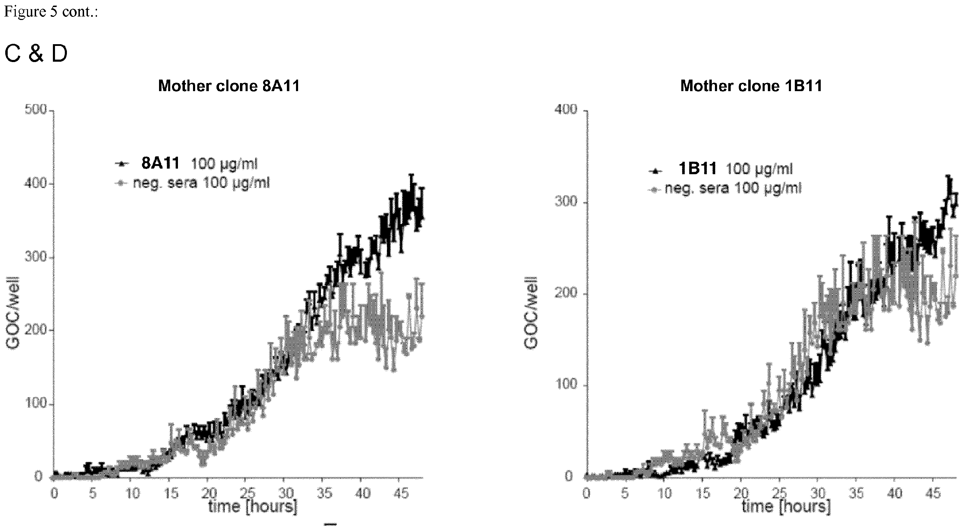

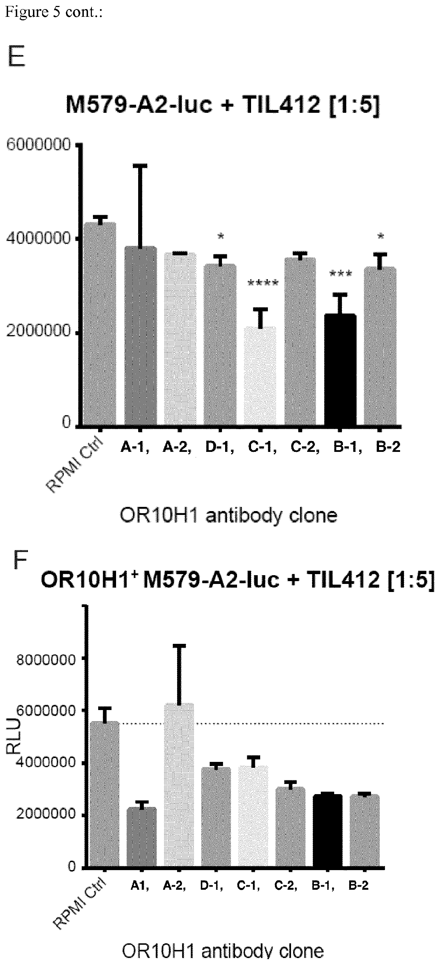

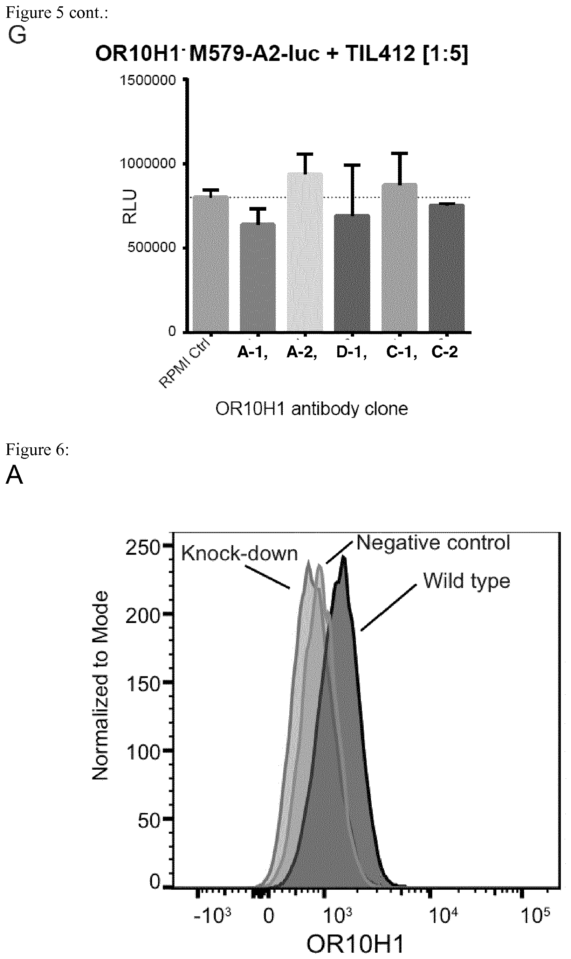

**Figure 5****: Generation of monoclonal blocking antibodies against OR10H1.** Mother clones of OR10H1-blocking antibodies were generated by genetic immunization of three rats with an OR10H1-IgG construct and screening for binding and blocking activity. The four most promising mother clones were enriched and tested for their impact on TIL-mediated killing by Luc-CTL cytotoxicity assay **(A** and **B)** and for two of such clones in an IncuCyte cytotoxicity assay **(C** and **D),** based on caspase activation. **A, B** Blocking efficacy was tested using different antibody concentration (0- 500 µg/ml) for the co-culture of TIL412 with OR10H1⁺- or OR10H1⁻ M579-A2-luc, compared to pre-immunization rat serum. IncuCyte cytotoxicity assays depicting the impact of mother clones 8A11 **(C)** and 1B11 **(D)** on TIL-mediated induction of apoptosis in M579-A2 over 48 hours (measurement every 5 min). The Y axis represents the number of green object counts (event of caspase-3 activation) per well. **E, F, G** Summary of screening for monoclonal antibodies derived from aforementioned mother clones. Luc-CTL cytotoxicity assays showing the impact of monoclonal supernatants (unknown concentration) on the TIL-mediated killing of wild-type **(E),** control knockdown **(F)** or OR10H1 knockdown **(G)** M579-A2-luc. A and B are representative of two independent experiments. C and D are exemplary experiments. E, F and G are results from one screening. A-E were performed in triplicates, F and G were performed in duplicates. Error bars denote ± SEM, and statistical significance was calculated using unpaired, two-tailed Student's t-test with * p ≤ 0.05; ** p ≤ 0.01; *** p ≤ 0.001; **** p ≤ 0.0001. Clone IDs: A = 1C3, B = 1B11, C = 8A11, D = 4B4, A-1 = 1C3-B2, A-2 = 1C3-A9, D-1 = 4B4-A10, C-1 = 8A11-G12, C-2 = 8A11-E7, B-1 = 1B11-B12 and B-2 = 1B11-B9.

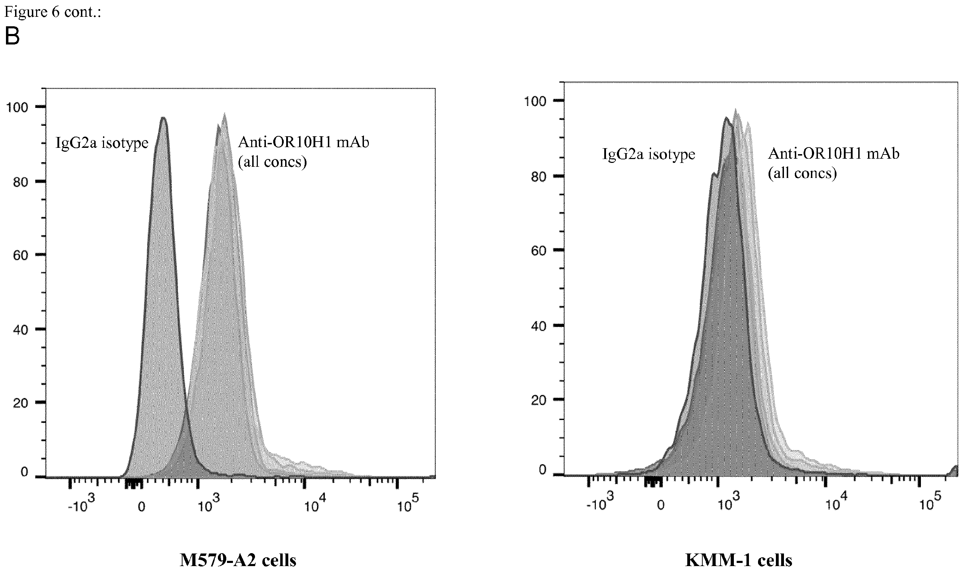

**Figure 6****: Detection of OR10H1 using an antibody of the invention. A** Surface expression of OR10H1 on melanoma M579-A2 transfected with non-specific control siRNA or OR10H1 siRNA 1 detected with post-immunisation polysera against OR10H1. **B** Surface expression of OR10H1 detected by the antibody purified from clone Di-8A11-H12-E6 on M579-A2 melanoma cells that express OR10H1 (determined by RT-PCR), but not detected on KMM-1 myeloma cells that do not express OR10H1 (determined by RT-PCR).

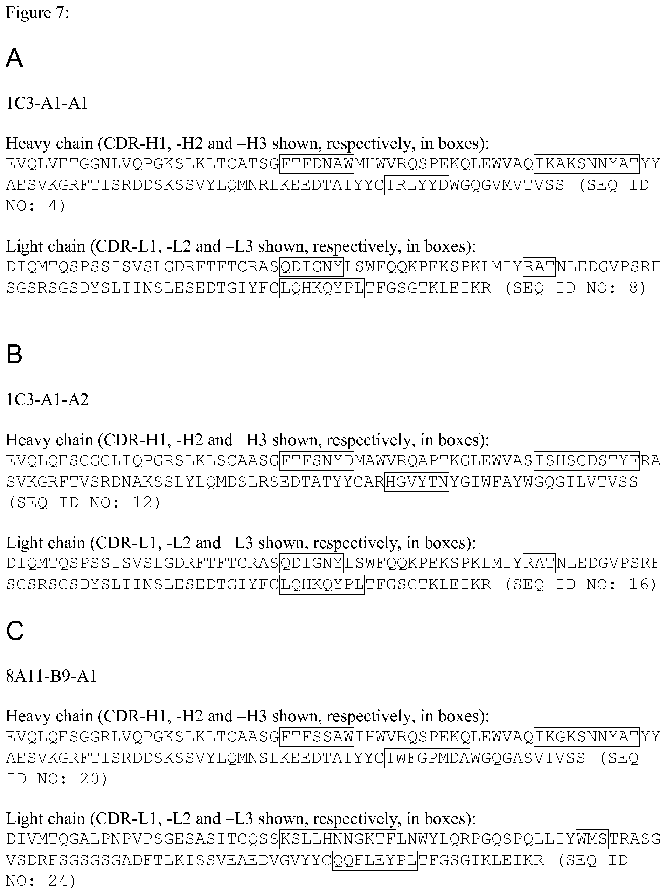

**Figure 7****: Amino acid sequences of certain antibodies of or for use in the present invention. A.** Sequence of the amino acid sequence of the heavy and light chains of antibody 1C3-A1-A1; **B.** Sequence of the amino acid sequence of the heavy and light chains of antibody 1C3-A1-A2; **C.** Sequence of the amino acid sequence of the heavy and light chains of antibody 8A11-B9-A1.

### And in the Sequences:

**Table 1D: Antibody Sequences**

| **SEQ ID NO:** | **Antibody** | **Chain** | **Region** | **Amino Acid Sequence** |
|---|---|---|---|---|
| 1 | 1C3-A1-A1 | heavy | CDR1 | FTFDNAW |
| 2 | 1C3-A1-A1 | heavy | CDR2 | IKAKSNNYAT |
| 3 | 1C3-A1-A1 | heavy | CDR3 | TRLYYD |
| 4 | 1C3-A1-A1 | heavy | variable domain | |
| 5 | 1C3-A1-A1 | light | CDR1 | QDIGNY |
| 6 | 1C3-A1-A1 | light | CDR2 | RAT |
| 7 | 1C3-A1-A1 | light | CDR3 | LQHKQYPL |
| 8 | 1C3-A1-A1 | light | variable domain | |
| 9 | 1C3-A1-A2 | heavy | CDR1 | FTFSNYD |
| 10 | 1C3-A1-A2 | heavy | CDR2 | ISHSGDSTYF |
| 11 | 1C3-A1-A2 | heavy | CDR3 | HGVYTN |
| 12 | 1C3-A1-A2 | heavy | variable domain | |
| 13 | 1C3-A1-A2 | light | CDR1 | QDIGNY |
| 14 | 1C3-A1-A2 | light | CDR2 | RAT |
| 15 | 1C3-A1-A2 | light | CDR3 | LQHKQYPL |
| 16 | 1C3-A1-A2 | light | variable domain | |
| 17 | 8A11-B9-A1 | heavy | CDR1 | FTFSSAW |
| 18 | 8A11-B9-A1 | heavy | CDR2 | IKGKSNNYAT |
| 19 | 8A11-B9-A1 | heavy | CDR3 | TWFGPMDA |
| 20 | 8A11-B9-A1 | heavy | variable domain | |
| 21 | 8A11-B9-A1 | light | CDR1 | KSLLHNNGKTF |
| 22 | 8A11-B9-A1 | light | CDR2 | WMS |
| 23 | 8A11-B9-A1 | light | CDR3 | QQFLEYPL |
| 24 | 8A11-B9-A1 | light | variable domain | |

**SEQ ID NO: 26 to 34 show OR10H1 siRNA/shRNA sequences (see table E1/E2 below).**

### EXAMPLES

### Example 1: OR10H1 knock-down increases TIL-mediated killing of solid tumors (Figure 1).

RT-PCR data and expression database searches suggest that the cell surface-expressed gene OR10H1 is expressed by melanoma, PDAC and colorectal cancer. Therefore, two OR10H1-specific PCR primers are tested by sequencing the respective RT-PCR amplicons according to standard procedures. The results show that OR10H1 is expressed by the following cells: M579-A2 (melanoma) (Machlenkin et al, 2008; Cancer Res 68:2006-13), PANC-1 (PDAC) and SW480 (colorectal) (both, ATCC), but not expressed by KMM-1 (myeloma) (Namba et al, 1989; In Vitro Cell Dev Biol 8:723-9 (Figure 1AA).

SMART pool siRNA against OR10H1 (GE Dharmacon) were tested (by RT-PCR) for their activity in downregulating OR10H1 mRNA in M579 (melanoma) cells. Following transfection into M579-A2 cells, all siRNAs show a knock-down of OR10H1 (Figure 1AB). siRNA 1 shows a complete absence of OR10H1 mRNA, siRNA 3 and pooled OR10H1 siRNAs show a strong reduction, siRNA 2 and 4 show a weaker but clear reduction in OR10H1 transcription. Details of the respective siRNAs are set out in Table E1.

**Table E1: Exemplary siRNAs against ORH10H1**

| **Name** | **Sequence** | **GE Dharmacon order#** | **SEQ ID No.** |
|---|---|---|---|
| OR10H1 siRNA 1 | GGAGACACCUUGAUGGGCA | D-020479-01 | 26 |
| OR10H1 siRNA 2 | AGUAAACUCUACCCAGAAA | D-020479-02 | 27 |
| OR10H1 siRNA 3 | GCAGAGAGCCAAUCACUCC | D-020479-03 | 28 |
| OR10H1 siRNA 4 | GGUCGUGCACUAUGGCUUU | D-020479-04 | 29 |
| OR10H1 pool | | M-020479-01 | |

The inventors were surprised to find that all siRNAs show an impact on TIL412-mediated killing of M579-A2-luc at 5:1 Effector to Target cell (E:T) ratio (Figure 1 B). Transfection of M579-A2-luc cells with OR10H1 siRNA 1 increased HLA-matched patient-derived tumor-infiltrating lymphocyte (TIL, clone 412)-mediated killing to 70% (30% remaining melanoma cells compared to a scrambled negative control siRNA; p = <0.0001). This increase in TIL-mediated lysis is stronger than for PD-L1 positive control knock-down (39% remaining cells). Overall, the effect on the phenotype is comparable to the knock-down efficacy on OR10H1 mRNA. Only siRNA 3 shows an effect on M579-A2-luc viability (without the presence of TILs; data not shown).

TIL-mediated lysis of M579-A2 was validated in chromium release assay (4 h co-culture). Another patient-derived and HLA-matched TIL culture (TIL209) was used to show that the knock-down effect on TIL-mediated killing is not depending specifically on TIL412. Knockdown of OR10H1 (siRNA 1) strongly increased the lysis of M579-A2-luc in all effector to target (E:T) ratios compared to the negative control siRNA (57% to 27% specific lysis at 12.5 E:T ratio; Figure 1C). Indeed, this effect of OR10H1 knock-down was stronger than the PD-L1 positive control knock-down (41% specific lysis at 12.5 E:T ratio; Figure 1C).

OR10H1 knock-down increased TIL-mediated killing in an autologous melanoma setting. For this, M615 tumor cells and TIL615 were derived from the same patient, and M615 was stably transfected with luciferase to produce M615-luc. The basic killing of M615-luc by TIL615 was very low. However, knock-down of OR10H1 increased TIL-mediated killing compared to the negative control siRNA (Figure ID).

Furthermore, it was tested whether OR10H1 knock-down also has an effect in other cancers. Surprisingly, OR10H1 knock-down increased the TIL-mediated tumor cell killing in colorectal cancer (CRC) and pancreatic cancer cells. SW480 (ATCC) and PANC-1 cells were analyzed for their expression of OR10H1 (see above) and the knock-down efficacy of the siRNAs were validated. The CRC cell line SW480 and the PANC-1 cell line were each co-cultured with corresponding HLA-matched patient-derived TILs for chromium release (4 h co-culture). OR10H1 siRNA 1 almost tripled the lysis of SW480 by TILs in all effector to target ratios compared to the negative control siRNA (33% to 12.8% specific lysis at 50:1 E:T ratio; Figure IE). Indeed, knock-down of OR10H1 was almost as effective as knock-down of PD-L1 positive control on TIL-mediated lysis (Figure IE). Such effect was also surprising to see in pancreatic cancer cells: knock-down of OR10H1 (siRNA 1) doubled the lysis of PANC-1 by TILs in all effector to target ratios compared to the negative control siRNA (53% to 26% specific lysis at 50:1 E:T ratio; Figure 1F)

The effects of siRNA knock-down on TIL-mediated killing and lysis in a chromium release assay were investigated as described in Khandelwal et al, 2015 (EMBO Mol Med 7:450-63). Tumor-infiltrating lymphocytes 412 and 209 microcultures were expanded from an inguinal lymph node of a melanoma patient as described in Dudley et al, 2010 (Clin Cancer Res 16:6122-31). M615, TIL615 and other patient-derived TIL cells were obtained analogously as described in Dudley et al, 2010. M579-A2-luc cells and M615-luc cells were produced from M579-A2 and M615, respectively (Machlenkin et al, 2008; Cancer Res 68:2006-13).

### Example 2: OR10H1 knock-down increases TIL activity and survival (Figure 2)

A co-culture of M579-A2 and TIL412 leads to a switch in cytokines secreted by TILs (from immune-suppressive type II to immune-activating type I), showing an increase in anti-tumor response. Cells were co-cultured for 20 hours and the cytokine concentrations in the supernatant were measured by Luminex as described in Khandelwal et al, 2015. As controls unstimulated TILs (no tumor cells) and over-stimulated TILs (PMA and ionomycin) were used (not shown).

As expected unstimulated TILs did not secrete considerable amounts of cytokines, whereas over-stimulated TILs secreted dramatically increased amounts. However, the inventors were surprised to observe that knock-down of OR10H1 on melanoma increased type I cytokine secretion by TIL412 (Figure 2A). IFN-γ secretion increased significantly from 2825 to 3037 pg/ml (increase of 8%; p =0.0236) compared to the negative control. IL-2 secretion significantly increased from 1079 to 1701 pg/ml (increase of 58%; p=0.045). Furthermore, knockdown of OR10H1 was found to surprisingly decrease type II cytokine secretion by TIL412 (Figure 2A). MCP-1 secretion significantly decreased from 6658 to 4035 pg/ml (decrease of 39%; p= 0.016). IL-6 secretion significantly decreased from 49.4 to 31 pg/ml (decrease of 37%; p= 0.028). IL-4 secretion decreased from 14.75 to 11.8 pg/ml (20%; trend).

Knock-down of OR10H1 increased the number of IFN-γ producing TILs. Melanoma cells and TILs were co-cultured for 20 h and the number of IFN-γ producing cells was measured using ELISpot as described in Khandelwal et al, 2015. Although TILs did not produce IFN-g without interaction with melanoma cells, knock-down of OR10H1 (pooled siRNA) significantly increased the number of IFN-g spots/104 TILs from 107.75 to 187.75 (increase of 73%; p=0.02; Figure 2B).

Without being bound by theory, the effect of OR10H1 knock-down (in melanoma cells) on TIL activity appeared to depend on the HLA-TCR interaction (not shown). The inventors then compared the amounts of IFN-γ after the co-culture (6 h) of M579-A2 melanoma cells with TILs. If melanoma cells did not express HLA-A2 (M579), IFN-γ secretion was abrogated regardless of the knock-down of OR10H1. However, knock-down of OR10H1 on melanoma cells surprisingly reduced the induction of apoptosis in TILs after co-culture suggesting that OR10H1 knock-down prolonged survival and/or increased proliferation of TILs. Melanoma cells and TILs were co-cultured for 6 h and the percentage of Annexin V-positive CD8+ TILs (FACS staining) were calculated, in each case according to standard procedures. TILs which were not co-cultured or activated showed 35% of Annexin V-positive CD8+ T cells. Over-activation with PMA and ionomycin resulted in 60% apoptotic CD8+ TCs. Interestingly, co-culture with OR10H1-positive M579-A2 (scrambled siRNA control) increased the percentage of apoptotic CD8+ TCs to 62%. Surprisingly, knock-down of OR10H1 prior to co-culture reduced the number of apoptotic CD8+ TILs to 47% (Figure 2C). The induction of apoptosis (compared to unstimulated TILs) is half as large if OR10H1 is not expressed in the melanoma cells (p= 0.032). Knock-down of PD-L1 decreased the percentage of apoptotic CD8+ TCs to 53% (non-significant compared to scrambled siRNA control)

### Comparative Example 3: OR10H1 functions as an immune checkpoint in vivo (Figure 3)

In order to assess OR10H1 function *in vivo,* a stable OR10H1 knock-down M579-A2 line was generated using lentiviral shRNA particles (MISSION shRNA for OR10H1; Sigma Aldrich), analogously as described in Khandelwal et al, 2015 for the stable CCR9 knockdown in M579-A2. The stable knock-down of OR10H1 increased the specific lysis of M579-A2 by TIL412 and TIL209 *in vitro* (chromium release) compared to negative target sequence (NTS) control-transduced M579-A2 (not shown). ShRNA 4 showed the strongest impact on OR10H1 expression and functionality, whereas stable knock-down of OR10H1 did not affect viability or proliferation of M579-A2 *in vitro* (not shown). Details of the respective shRNAs are set out in Table E2.

**Table E2: Exemplary shRNAs against ORH10H1**

| | | | |
|---|---|---|---|
| **Name** | **Sequence** | **Sigma Aldrich order#** | **SEQ ID No.** |
| OR10H1 shRNA 1 | GTTCCTGCTGATGTACCTGTT | TRCN0000011786 | 30 |
| OR10H1 shRNA 2 | TGCGCTACAACGTGCTCATGA | TRCN0000357706 | 31 |
| OR10H1 shRNA 3 | TGGCTTTGCCTCCGTCATTTA | TRCN0000357707 | 32 |
| OR10H1 shRNA 4 | TCTGCTGAAGGTCGGAACAAG | TRCN0000357708 | 33 |
| OR10H1 shRNA 5 | ACACAAGGAGATCCACCATTT | TRCN0000357775 | 34 |

OR10H1 knock-down and NTS control -transduced M579-A2 cells (in matrigel) were injected subcutaneously into the flanks of immunodeficient (NOD *scid* gamma; NSG) mice (Figure 3A) and on day 2 and 9 after tumor inoculation, TIL412 cells were injected intravenously (adoptive cell transfer; ACT). Subsequently the tumor size was measured for 24 days. A control group of mice was injected with tumor cells but did not receive ACT, so as to validate the effect of OR10H1 knock-down on tumor growth without the presence of TILs.

Tumor growth is strongly reduced in OR10H1 knock-down (kd) tumors (ie, OR10H1-negative tumors) after ACT with TIL412 but not without the ACT (Figure 3B and C). On day 7 (273 mm³) the tumors start to grow regardless of the presence or absence of OR10H1. There is no significant difference in tumor growth of OR10H1 knock-down M579-A2 compared to that of the NTS shRNA control group (Figure 3C). In contrast, and to the surprise of the inventors, if the mice were i.v. injected with TIL412 as ACT, M579-A2 tumor growth of OR10H1-negative (kd), but not growth of shRNA control M579-A2 tumors (ie, OR10H1-negative), was significantly reduced after day 11 (Figure 3B). OR10H1 knock down tumors significantly reduced in volume from 260 to 207 mm³ between day 11 and 14 after inoculation. In contrast, and over the same period, the NTS control tumors grew substantially from 296 to 389 mm³ (p = 0.013). On day 18 the average OR10H1-negative tumor (kd) had a volume of only 203 mm³ compared to 401 mm³ for the average size of NTS control (ie OR10H1-positive) tumors (p= 0.004). Although after day 18, the OR10H1-negative tumors do start to grow again, their tumor mass remains significantly reduced compared to NTS control (ie OR10H1-positive) tumors.

### Example 4: OR10H1 inhibits TIL functionality utilizing cAMP (Figure 4)

TIL412 were co-cultured with (OR10H1 knock-down or negative siRNA control) M579-A2 for 10 h cells to activate signaling pathways in the TILs, and then the melanoma cells were then removed from the co-culture using magnetic melanoma beads specific for MCSP (Miltenyi Biotech). The purity of the remaining TILs was around 99.5% (not shown). Differential gene expression between the TILs isolated from the OR10H1 knock-down and the negative siRNA control was measured by RNA-Sequencing according to standard procedures. Several genes were significantly up or down regulated (fold change above 0.5 or below -0.5 respectively) in the setting with OR10H1 knock-down M579-A2 (siRNA 1) compared to the negative siRNA control (Figure 4A). Down-regulated in the TILs were the following genes: Early growth response gene 3 (Egr3) is a key negative transcriptional regulator of T cell activation and induces anergy [1-3]. Interferon-gamma receptor chain 2 (IFNGR2) expressed in high levels inhibits T cell proliferation and may induce apoptosis [4, 5]. Nuclear Receptor Subfamily 4, Group A, Member 2 (NR4A2) is associated with T cell exhaustion in chronic viral infection [6] and might be involved in apoptosis [7]. Up-regulated in the TILs were the following genes: Chemokine (C-X-C motif) ligand 13 (CXCL13) promotes T cell recruitment [8] and facilitates the inflammatory response of antigen-experienced T helper cells [9]. Cytotoxic And Regulatory T Cell Molecule (CRTAM) is expressed (upon TCR activation) on activated T cells and its interaction with Necl-2 (on tumors) promotes IFN-g secretion by CD8+ T cells [10]. Vav guanine nucleotide exchange factor 3 (VAV3) is involved in TCR signaling via NFAT and SRF activation [11]. FBJ Murine Osteosarcoma Viral Oncogene Homolog (FOS) together with c-Jun builds up activating protein 1 (AP-1), one of three major transcription factors downstream of TCR signaling [12]. V-Myc Avian Myelocytomatosis Viral Oncogene Homolog (c-Myc) is a transcription factor which is involved in proliferation and metabolic reprogramming upon T lymphocyte activation [13, 14]. It modulates the generation of CD8+ immunological memory in tumors and viral infection [15, 16]. Interferon regulatory factor 4 (IRF4) is a transcription factor necessary for the expansion and effector differentiation of CD8+ T cells and represses genes involved in cell cycle arrest and apoptosis. [17, 18]

Upon Ingenuity pathway analysis (Qiagen; Figure 4B) to investigate functions in which the differential expressed genes are enriched (positive or negative activation) in TILs and which signaling networks play a role, the inventors surprisingly found that of the eight pathways analyzed, only CREB is differentially phosphorylated in the setting with OR10H1-negative-melanoma. The log2 ratio (compared to unstimulated TILs) for CREB is significantly reduced after the knock-down of OR10H1 (p= 0.006; Figure 4C).

Western blot analysis for phospho-CREB and ATF1 (same phosphorylation site) in TIL412 confirmed the reduced activation depending on OR10H1 knock-down on melanoma cells (Figure 4F). cAMP response element-binding protein (CREB) downstream of TCR activation is widely known to be important for proliferation and survival of T cells [19] but has been associated with T cell anergy as well [20]. Therefore, key factors of TCR signaling (Lck, LAT, CREB, ZAP-70, Syk, CD3e and ERK1/2) were analyzed.

Knock-down of OR10H1 on melanoma cells leads to decreased phosphorylation of CREB and Lck (inhibitory phospho-Tyrosin 505) in TILs (Figures 4D and 4E). Importantly however, OR10H1 does not play a role in modulating CD3e activation (phosphylation): CD3e does not show increased phosphorylation levels after co-culture with melanoma cells/TILs (regardless of OR10H1 knock-down) compared to unstimulated TILs, and the same is observed for and ERK1/2. Yet, over-stimulation with PMA and ionomycin does dramatically increased phosphorylation of ERK1/2 but does not affect CD3e.

Phosphorylation of Linker of activated T cells (LAT) slightly increases in TILs in the first 30 minutes of co-culture and stays stable up to two hours (for OR10H1-positive and OR10H1-negative melanoma cell/TIL co-cultures and over-activation PMA and ionomycin). Spleen tyrosine kinase (Syk) gets activated in 5 minutes and later on decreases below the level of unstimulated TILs in all three settings. Finally, phosphorylation of Zeta-chain-associated protein kinase 70 (ZAP70) increases in the first 5 minutes and stays stable for 2 h if co-cultured with melanoma cells/TILs (regardless of OR10H1 knock-down). Over-activation with PMA and ionomycin leads to an increased phosphorylation of ZAP70.

Co-culture of TIL412 with OR10H1-positive M579-A2 melanoma cells leads to a dramatically increased phosphorylation of CREB after 2h in TILs (Figure 4D). Interestingly, the first 30 min after activation the phosphorylation levels are similar to the OR10H1 knock-down setting. Over activation with PMA and ionomycin leads to a faster onset of CREB phosphorylation but the levels are similar to the control siRNA (OR10H1-positive) setup. In contrast, Lymphocyte-specific protein tyrosine kinase (Lck) becomes strongly dephosphorylated after 30 min in TILs if co-cultured with OR10H-negative M579-A2 melanoma cells or over-activated with PMA and ionomycin but not in the control siRNA (OR10H1-positive) setup (Figure 4E).

As described above, TCRs of TILs which were co-cultured with melanoma cells were activated in the same way, but showed different signaling at two important hubs, namely CREB and Lck, depending on whether OR10H1 was present or absent on the target melanoma cells. Therefore, OR10H1-mediated inhibition of T cell activity might converge here. Lck has two phosphorylation sites but phosphoplex analysis of Lck measures total Lck phosphorylation. Phosphorylation of Lck-Tyr384 stabilizes the active conformation whereas phosphorylation of Lck-Tyr505 promotes the auto-inhibited conformation of Lck [21-23]. Western blots for phospho-Lck confirmed that phosphorylation of the inhibitory Tyr505 residue was strongly reduced after the knock-down of OR10H1 suggesting an increased activation of Lck in TILs. Indeed, inhibition of Lck activity by a small molecule inhibitor is shown to abrogate OR10H1 knock-down effect on T-cell mediated cytotoxicity (as reflected by the ratio of cytotoxicity/viability) of melanoma cells (Figure 4G). Protein kinase A (PKA) is activated by cAMP and in turn activates c-Src Tyrosine Kinase (Csk). Csk abrogates Lck activity by phosphorylation of Lck-Tyr505 [24, 25]. Indeed, the inventors were able to confirm by western blot analysis, an enhancement of PKA phosphorylation in TILs after the OR10H1-positive (ie control siRNA) M579-A2 melanoma cell/TIL co-culture. (Figure 4F).

Olfactory receptor signaling activates a unique G protein (G-alpha-Olf) and subsequently adenylate cyclase type III (Figure 4H). RNA-sequencing expression data suggest that both genes are expressed in M579-A2. M579-A2 melanoma cells (OR10H1-positive and OR10H1-negative) were transiently transfected with a cAMP reporter luciferase construct and co-cultured with TIL412. The production of cAMP in melanoma is altered by OR10H1 knockdown only if there is an interaction with TILs, while co-culture of M579-A2 cells with TILs alone was not sufficient to trigger a cAMP response (not shown). Indeed, after co-culture of M579-A2 (OR10H1-positive or OR10H1-negative) with TILs for 2 h, 10 uM forskolin was added to raise cAMP levels: without a preceding co-culture with TILs there is no difference in cAMP response in M579-A2 cells, whereas in the presence of TILs the cAMP response in M579-A2 cells is reduced if OR10H1 is knocked-down on the melanoma cells (Figure 4I).

The inventors were surprised to observe data suggesting that that TIL-driven cytotoxicity of the melanoma cells may be inhibited by a mechanism involving connexin 32 (Cx32) and the transport of cAMP from the tumor cell to the T cell. We validated by RNA-sequencing that M579-A2 cells express connexin 32, a protein involved in the formation of gap-junctions. Such gap-junctions are selective for the transport of cAMP and cGMP [28], and hence the potential for cAMP transport from M579-A2 to the TIL412. Indeed, preliminary results suggest that blockade of Cx32 leads to an increased killing of melanoma cells by TILs only if OR10H1 is present (not shown). This is consistent with recent studies have shown that regulatory T cells (Tregs) and tumor cells can transport cAMP via gap-junctions into T cells and lead to inhibition through phosphorylation of Lck and CREB [26, 27].

### Example 5: Development of Antibodies against OR10H1 and their functional activity (Figure 5)

For generating antibodies against OR10H1, genomic immunization of rats was conducted at Aldevron (Freiburg), described briefly as follows.

Firstly, an OR10H1 construct was cloned into an Aldrevon immunization vector (pB8-OR10H1) and an Aldrevon screening vector (pB1-OR10H1). Transient transfection of these vectors into mammalian cells confirmed (weak) cell surface expression. Cell surface expression of such proteins - containing a vector-derived N-terminal *tag*-sequence - was analyzed by flow cytometry on non-fixed living cells using an anti-*tag* murine antibody and a goat antimouse IgG R-phycoerythrin conjugate (Southern Biotech) as a secondary antibody. Secondly, three rats were immunized with the immunization vector (pB8-OR10H1) for 46 days and 5 genetic applications (IS46d-5) followed by an additional 2 genetic immunizations over a subsequent 35 days; in total immunization over for 81 days with 7 genetic applications (IS81d-7)). Thirdly, sera of immunized rats (diluted in PBS 3% FBS) were then tested (by flow cytometry) for reactivity against mammalian cells transiently transfected with the screening vector (pB1-OR10H1) using a goat anti-rat IgG R-phycoerythrin conjugate (Southern Biotech) as a secondary antibody. Compared to pre-immunization sera, a significant (but weak reactivity) was observed (data not shown). Fourthly, splenocytes were isolated from the rats' spleen, the resulting B cells fused with immortalized myeloma cells by standard technologies, and the resulting hybridoma clones screened for the reactivity of their supernatant against the recombinant mammalian cells transiently transfected with the screening vector (as described above). Screening and enrichment of such hybridoma clones resulted in 4 mother clones being selected (1C3, 1B11, 8A11 and 4B4).

Each of these antibodies produced by such clones showed a significant impact on TIL-mediated melanoma killing at a concentration of 100µg/ml in the OR10H1-positive M579-A1-Luc/TIL412 assay (Figures 5A and B) as well as for those tested (8A11 and 1B11) in the IncuCyte cytotoxicity assay based on caspase activation (Figures 5C and D), in each case as described above.

Next, the mother clones were single-cell sorted to generate individual hybridoma clones that produce monoclonal antibodies, which were tested for their functional activity in the OR10H1-positive M579-A1-Luc/TIL412 assay as described above (Figures 5E, F and G), and individual hybridoma clones were selected for sequencing of the antibody they produce.

Such results demonstrate the ability of antibodies of the present invention to have therapeutic and research utility as and/or in the development of compounds for the treatment of proliferative disease such as a cancer disease, in particular of OR10H1-positive cancers.

### Example 6: Detection of OR10H1 using an Antibody of the present invention (Figure 6)

The post-immunisation poly sera was used to demonstrate that it can specifically detect OR10H1 expressed on the surface of tumor cells. FACS detection of melanoma M579-A2 cells transiently transfected with negative control siRNA or OR10H1 knock-down siRNA1 was conducted (Figure 6A), using a chicken anti-rat IgG- AlexaFluor 647 conjugate (ThermoFisher A-21472) as a secondary antibody. Cells knocked-down for OR10H1 show reduced fluorescence compared to wild-type (ie, OR10H1-positive) cells.

In particular, the antibody purified from clone Di-8A11-H12-E6 shows specificity (compared to control IgG2a isotype antibody) by binding to those melanoma cells (M579-A2) that express OR10H1 but not binding to myeloma cells (KMM-1) that do not express OR10H1 (Figure 6B). OR10H1 expression is determined by RT-PCR.

Such results demonstrate the ability of antibodies of the present invention to detected the expression of OR10H1 protein and their utility to determine increased resistance of a cell against an immune response, such as of a cancer cell.

### Example 7: Sequences of antibodies of the invention (Figure 7):

The sequences of variable domains of the antibodies produced by hybridoma clones of Example 5 were determined by standard procedures at Antibody Designs Laboratories (San Diego). Briefly, the following general procedure was followed: (1) total RNA extraction and cDNA Synthesis; (2) 5'RACE extension; (3) amplification of VH and VL domains including leader sequence and partial constant regions CH1 and CL; (4) cloning of PCR positive reactions; (5) colony PCR and sequencing of clones with proper insert size; and (6) sequencing analysis up to 5x coverage or 10 clones per chain. From the nucleic acid sequence the amino acid sequence of the variable domains of the chains of each antibody was determined. The amino acid sequence of the variable domains of heavy and light chain of exemplary antibodies of the invention is shown in Figures 7A, B and C. Hybridoma clone Di-8A11-H12-E6 is deposited at the DSMZ (Deutsche Sammlung von Mikroorganismen und Zellkulturen, Braunschweig, Germany) in accordance with the Budapest Treaty. (DSMZ Deposition Number: DSM ACC3310, deposited 26-Oct-2016). Hybridoma clone Di-8A11-H12-E6 is a hybridoma comprising Rat B lymphocytes fused with murine Ag8 myeloma cells, and can be cultured at 37°C in a 5% CO₂ atmosphere in RPMI-1640 medium supplemented with 10% Fetal calf serum, 10 U/ml human recombinant IL-6 and 1% penicillin-streptomycin (10,000 U/mL). The optimal split ratio is 1:1. The viability of these hybridoma cells may be relatively low when first thawed, however this recovers over a few days in culture. Each antibody chain sequence comprises c-terminally a constant region (Table E3).

**Table E3: Rat Constant Domain Regions:**

| **Rat IgG subclass** | **Uniprot ID for constant-region (accessed 20-Oct-2016)** | **Constant region length (aa)** |
|---|---|---|
| IgG1 | P20759 (Sequence version 1 of 1-Feb-1991; Entry version 113; 5-Oct-2016) | 326 |
| IgG2a | P20760 (Sequence version 1 of 1-Feb-1991; Entry version 116; 5-Oct-2016) | 322 |
| IgG2b | P20761 (Sequence version 1 of 1-Feb-1991; Entry version 105; 5-Oct-2016) | 333 |
| IgG2c | P20762 (Sequence version 1 of 1-Feb-1991; Entry version 96; 5-Oct-2016) | 329 |
| IgG kappa (A allele) | P01836 (Sequence version 1 of 21-Jul-1986; Entry version 80; 5-Oct-2016) | 106 |
| IgG kappa (B allele) | P01835 (Sequence version 1 of 21-Jul-1986; | 106 |
| Entry version 89; 5-Oct-2016) | | |

### Example 8: Peptide fingerprint mapping of antibodies of the invention:

Using standard procedures - briefly, trypsin digestion of antibody protein followed by peptide mass fingerprinting using MALDI-TOF mass spectrometry - (Toplab GmbH, Martinsried, Germany), the IgG subclass of the constant region of the antibody chain(s) is determined, by comparison of the peptide mass-peaks measured by MALDI-TOF mass spectrometry to the mass of peptide fragments predicted to be obtained from trypsin digestion of the respective rat IgG subclasses (eg Table E3).

### References for Examples

1. Safford, M., et al., Egr-2 and Egr-3 are negative regulators of T cell activation. Nat Immunol, 2005. 6(5): p. 472-480.
2. Collins, S., et al., Opposing regulation of T cell function by Egr-1/NAB2 and Egr-2/Egr-3. European Journal of Immunology, 2008. 38(2): p. 528-536.
3. Zheng, Y., Y. Zha, and T.F. Gajewski, Molecular regulation of T-cell anergy. EMBO Rep., 2008. 9: p. 50-55.
4. Bernabei, P., et al., Interferon-y receptor 2 expression as the deciding factor in human T, B, and myeloid cell proliferation or death. Journal of Leukocyte Biology, 2001. 70(6): p. 950-960.
5. Schroder, K., et al., Interferon-y: an overview of signals, mechanisms and functions. Journal of Leukocyte Biology, 2004. 75(2): p. 163-189.
6. Wherry, E.J., et al., Molecular Signature of CD8+ T Cell Exhaustion during Chronic Viral Infection. Immunity, 2007. 27(4): p. 670-684.
7. Zhou, T., et al., Inhibition of Nur77/Nurr1 leads to inefficient clonal deletion of self-reactive T cells. J Exp Med, 1996. 183(4): p. 1879-92.
8. Hui, W., C. Zhao, and S.G. Bourgoin, LPA Promotes T Cell Recruitment through Synthesis of CXCL13. 2015. 2015: p. 1-10.
9. Manzo, A., et al., Mature antigen-experienced T helper cells synthesize and secrete the B cell chemoattractant CXCL13 in the inflammatory environment of the rheumatoid joint. Arthritis & Rheumatism, 2008. 58(11): p. 3377-3387.
10. Boles, K.S., et al., The tumor suppressor TSLC1/NECL-2 triggers NK-cell and CD8+ T-cell responses through the cell-surface receptor CRTAM. Blood, 2005. 106(3): p. 779-786.
11. Charvet, C., et al., Membrane localization and function of Vav3 in T cells depend on its association with the adapter SLP-76. Journal of Biological Chemistry, 2005. 280(15): p. 15289-15299.
12. Huang, Y. and R.L. Wange, T Cell Receptor Signaling: Beyond Complex Complexes. Journal of Biological Chemistry, 2004. 279(28): p. 28827-28830.
13. Dose, M., et al., c-Myc mediates pre-TCR-induced proliferation but not developmental progression. Blood, 2006. 108(8): p. 2669-2677.
14. Wang, R., et al., The Transcription Factor Myc Controls Metabolic Reprogramming upon T Lymphocyte Activation. Immunity, 2011. 35(6): p. 871-882.
15. Haque, M., et al., C-Myc regulation by costimulatory signals modulates the generation of CD8+ memory T cells during viral infection. Open Biology, 2016. 6(1).
16. Chandran, S.S., et al., Tumor-Specific Effector CD8+ T Cells That Can Establish Immunological Memory in Humans after Adoptive Transfer Are Marked by Expression of IL7 Receptor and c-myc. Cancer Research, 2015. 75(16): p. 3216-3226.
17. Yao, S., et al., Interferon Regulatory Factor 4 Sustains CD8+ T Cell Expansion and Effector Differentiation. Immunity, 2013. 39(5): p. 833-845.
18. Huber, M. and M. Lohoff, IRF4 at the crossroads of effector T-cell fate decision. European Journal of Immunology, 2014. 44(7): p. 1886-1895.
19. Wen, A.Y., K.M. Sakamoto, and L.S. Miller, The role of the transcription factor CREB in immune function. Journal of immunology (Baltimore, Md. : 1950), 2010. 185(11): p. 6413-6419.
20. Powell, J.D., et al., The -180 Site of the IL-2 Promoter Is the Target of CREB/CREM Binding in T Cell Anergy. The Journal of Immunology, 1999. 163(12): p. 6631-6639.
21. Abraham, N., et al., Enhancement of T-cell responsiveness by the lymphocyte-specific tyrosine protein kinase p56lck. Nature, 1991. 350(6313): p. 62-66.
22. Gervais, F.G., et al., The SH2 domain is required for stable phosphorylation of p561ck at tyrosine 505, the negative regulatory site. Molecular and Cellular Biology, 1993. 13(11): p. 7112-7121.
23. Yamaguchi, H. and W.A. Hendrickson, Structural basis for activation of human lymphocyte kinase Lck upon tyrosine phosphorylation. Nature, 1996. 384(6608): p. 484-489.
24. Vang, T., et al., Activation of the COOH-terminal Src kinase (Csk) by cAMP-dependent protein kinase inhibits signaling through the T cell receptor. The Journal of experimental medicine, 2001. 193(4): p. 497-507.
25. Taskén, K. and a.J. Stokka, The molecular machinery for cAMP-dependent immunomodulation in T-cells. Biochemical Society transactions, 2006. 34(Pt 4): p. 476-479.
26. Bopp, T., et al., Cyclic adenosine monophosphate is a key component of regulatory T cell-mediated suppression. The Journal of Experimental Medicine, 2007. 204(6): p. 1303-1310.
27. Ye, J., et al., TLR8 signaling enhances tumor immunity by preventing tumor-induced T-cell senescence. EMBO Molecular Medicine, 2014. 6(10): p. 1294-1311.
28. Bevans, C.G., Isoform Composition of Connexin Channels Determines Selectivity among Second Messengers and Uncharged Molecules. Journal of Biological Chemistry, 1998. 273(5): p. 2808-2816.

## Claims

1. A compound for use in the treatment of a disease of a subject, wherein the compound is a modulator of the expression, function and/or stability of human Olfactory Receptor, Family 10, Subfamily H, Member 1 (OR10H1), or of a variant of human OR10H1;
wherein the variant of human OR10H1 is a protein comprising an amino acid sequence having at least 95% sequence identity to the sequence of SEQ ID NO: 25; and
wherein the disease is a human OR10H1 positive cancer, or a cancer positive for the variant of human OR10H1; and
wherein the compound is: (i) a small interfering RNA (siRNA) that binds directly to mRNA of said human OR10H1 or the variant of human OR10H1, and so inhibits expression of said human OR10H1, or the variant of human OR10H1; or (ii) an antigen binding construct that specifically binds said human OR10H1, or the variant of human OR10H1, and that reduces resistance of a tumour- or cancer cell **characterized by** the expression of human OR10H1, or the variant of human OR10H1, to cytotoxic T lymphocyte (CTL) responses.

2. The compound for use according to claim 1, wherein the variant of human OR10H1 is selected from the group consisting of an ortholog of human OR10H1, and a functional fragment of a human OR10H1 protein, wherein said functional fragment inhibits a cell-based immune response to a cancer cell that expresses such functional fragment.

3. The compound for use according to claim 1 or 2, wherein the human OR10H1 positive cancer, or the cancer positive for the variant of human OR10H1 is **characterized by** a pathological immune response and/or expression of said human OR10H1, or the variant of human OR10H1.

4. The compound for use according to any of claims 1 to 3, wherein the human OR10H1 positive cancer, or the cancer positive for the variant of human OR10H1 is selected from melanoma, pancreatic cancer or colorectal cancer.

5. The compound for use according to any of claims 1 to 4, wherein the compound enhances an immune response, preferably enhances a cell-mediated immune response in the subject, such as a T-cell mediated immune response in the subject.

6. The compound for use according to any of claims 1 to 5, wherein the tumour or cancer cell is **characterized by** a detectable cell surface expression of human OR10H1, or the variant of human OR10H1.

7. The compound for use according to any of claims 1 to 6, wherein the tumour or cancer cell is **characterized by** a detectable cell surface expression of human OR10H1, or the variant of human OR10H1, before contacting the cell with the compound.

8. The compound for use according to any of claims 1 to 7, wherein the tumour or cancer cell is **characterized by** aberrant expression of human OR10H1, or the variant of human OR10H1 on such cell compared to that in a healthy subject or a normal cell.

9. An isolated antigen binding construct which is an antibody obtainable from hybridoma Di-8A11-H12-E6 (DSMZ Deposition Number: DSM ACC3310, deposited 26-Oct-2016), or an antigen binding fragment obtainable from such antibody; wherein said antigen binding fragment specifically binds to human OR10H1, or to a variant of human OR10H1, wherein the variant of human OR10H1 is a protein comprising an amino acid sequence having at least 95% sequence identity to the sequence of SEQ ID NO: 25.

10. An isolated antigen binding construct that specifically binds to human OR10H1, or to a variant of human OR10H1, wherein the variant of human OR10H1 is a protein comprising an amino acid sequence having at least 95% sequence identity to the sequence of SEQ ID NO: 25; and
wherein the antigen binding construct is an antibody, or an antigen binding fragment thereof, composed of at least one antibody heavy chain sequence, paired with at least one antibody light chain sequence, wherein the antigen binding construct is selected from the following (I), (II), or (III):
(I) An antibody, or antigen binding fragment thereof: wherein (Ia) said antibody heavy chain sequence comprises CDR1 to CDR3 sequences having the amino acid sequences of SEQ ID NO: 1 to 3, and wherein (Ib) said antibody light chain sequence comprises CDR1 to CDR3 sequences having the amino acid sequences of SEQ ID NO: 5 to 7, in each case of a CDR independently optionally with one amino acid substitution, insertion or deletion compared to these sequences;
(II) An antibody, or antigen binding fragment thereof, wherein (IIa) said antibody heavy chain sequence comprises CDR1 to CDR3 sequences having the amino acid sequences of SEQ ID NO: 9 to 11, and wherein (IIb) said antibody light chain sequence comprises CDR1 to CDR3 sequences having the amino acid sequences of SEQ ID NO: 13 to 15, in each case of a CDR independently optionally with one amino acid substitution, insertion or deletion compared to these sequences;
(III) An antibody, or antigen binding fragment thereof, wherein (IIIa) said antibody heavy chain sequence comprises CDR1 to CDR3 sequences having the amino acid sequences of SEQ ID NO: 17 to 19, and wherein (IIIb) said antibody light chain sequence comprisess CDR1 to CDR3 sequences having the amino acid sequences of SEQ ID NO: 21 to 23; in each case of a CDR independently optionally with one amino acid substitution, insertion or deletion compared to these sequences.

11. The isolated antigen binding construct according to claim 10:
(I) wherein said antibody heavy chain sequence of the antibody, or antigen binding fragment thereof, of (Ia) according to claim 10 comprises a variable region having the amino acid sequence of SEQ ID NO: 4, and wherein said antibody light chain sequence of the antibody, or antigen binding fragment thereof, of (Ib) according to claim 10 comprises a variable region sequence having the amino acid sequence of SEQ ID NO: 8, in each case of a variable region sequence independently with not more than ten, nine, eight, seven, six, five, four, three, two or one, preferably not more than three, amino acid substitutions, insertions or deletions compared to these sequences; or
(II) wherein said antibody heavy chain sequence of the antibody, or antigen binding fragment thereof, of (IIa) according to claim 10 comprises a variable region sequence having the amino acid sequence of SEQ ID NO: 12, and wherein said antibody light chain sequence of the antibody, or antigen binding fragment thereof, of (IIb) according to claim 10 comprises a variable region sequence having the amino acid sequence of SEQ ID NO: 16, in each case of a variable region sequence independently with not more than ten, nine, eight, seven, six, five, four, three, two or one, preferably not more than three, amino acid substitutions, insertions or deletions compared to these sequences; or
(III) wherein said antibody heavy chain sequence of the antibody, or antigen binding fragment thereof, of (IIIa) according to claim 10 comprises a variable region sequence having the amino acid sequence of SEQ ID NO: 20, and wherein said antibody light chain sequence of the antibody, or antigen binding fragment thereof, of (IIIb) according to claim 10 comprises a variable region sequence having the amino acid sequence of SEQ ID NO: 24, in each case of a variable region sequence independently with not more than ten, nine, eight, seven, six, five, four, three, two or one, preferably not more than three, amino acid substitutions, insertions or deletions compared to these sequences.

12. A pharmaceutical composition comprising the isolated antigen binding construct according to any of claims 9 to 11, or a nucleic acid, or nucleic acids, encoding for said antigen binding construct; and a pharmaceutically acceptable carrier, stabilizer and/or excipient.

13. A pharmaceutical composition for use in the treatment of a disease of a subject, wherein the disease is a human OR10H1 positive cancer, or a cancer positive for the variant of human OR10H1, comprising
the compound recited in any of claims 1 to 8, the isolated antigen binding construct according to any of claims 9 to 11, or a nucleic acid, or nucleic acids, encoding for said antigen binding construct; and
a pharmaceutically acceptable carrier, stabilizer and/or excipient.

14. An in vitro method for diagnosing a resistance phenotype of a cancer disease against an immune response such as a cell-mediated immune response in a human subject, the method comprising the steps of
a. Providing a sample of tumor or cancer cells of the subject,
b. Determining the presence or absence of protein or mRNA of human OR10H1, or of a variant of human OR10H1, in the tumor or cancer cells, wherein the variant of human OR10H1 is a protein comprising an amino acid sequence having at least 95% sequence identity to the sequence of SEQ ID NO: 25, and
c. Diagnosing a resistance phenotype of the cancer disease against an immune response in the subject when protein or mRNA of said human OR10H1, or of the variant of human OR10H1, is present in the tumor or cancer cells.

15. The method according to claim 14, wherein the presence of an amount or level of said protein or mRNA of human OR10H1, or of the variant of human OR10H1, is determined in step (b) as a measured amount.

16. The in vitro method according to claim 14 or 15, wherein step (b) involves contacting the sample with means for the detection of protein or mRNA of said human OR10H1, or of the variant of human OR10H1, wherein said means is preferably selected from: (i) an antigen binding construct specifically binding to said protein of human OR10H1, or of the variant of human OR10H1, and detecting the binding between said antigen binding construct and said protein of human OR10H1, or of the variant of human OR10H1; and/or (ii) a nucleic acid binding to said mRNA sequence of human OR10H1, or of the variant of human OR10H1, and detecting the binding between said nucleic acid and said mRNA of human OR10H1, or of the variant of human OR10H1.

17. An in vitro method for identifying a compound suitable for the treatment of a disease **characterized by** expression of human OR10H1, or a variant thereof, wherein the variant of human OR10H1 is a protein comprising an amino acid sequence having at least 95% sequence identity to the sequence of SEQ ID NO: 25, the method comprising the steps of
(a) Providing a first cell expressing a protein or mRNA of said human OR10H1, or of the variant of human OR10H1, wherein said first cell is a tumour cell or a cell derived from a tumour, and
(b) Providing a candidate compound, and
(c) Optionally, providing a second cell which is a cytotoxic immune cell, for example a cytotoxic T-lymphocyte (CTL), capable of immunologically recognizing said first cell, and
(d) Bringing into contact the first cell and the candidate compound, and optionally the second cell, and
(e) Determining subsequent to step (d), either or both of:
(i) expression, function and/or stability of said protein or mRNA of said human OR10H1, or of the variant of human OR10H1, in said first cell, wherein a reduced expression, function and/or stability of said protein or mRNA of said human OR10H1, or of the variant of human OR10H1, in said first cell contacted with the candidate compound compared to said first cell not contacted with said candidate compound indicates that the candidate compound is a compound suitable for the treatment of a disease **characterized by** expression of said protein or mRNA of said human OR10H1, or of the variant of human OR10H1; and/or
(ii) cytotoxicity of said second cell against said first cell, wherein an enhanced cytotoxicity of said second cell against said first cell contacted with the candidate compound compared to the cytotoxicity of said second cell against said first cell not contacted with the candidate compound indicates that the candidate compound is a compound suitable for the treatment of a disease **characterized by** expression of said human OR10H1, or the variant of human OR10H1.

18. The compound for use according to any of claims 1 to 8, the isolated antigen binding construct according to any of claims 9 to 11, the pharmaceutical composition according to claim 12, the pharmaceutical composition for use according to claim 13, the in vitro method according to any of claims 14 to 17, wherein said human OR10H1 is a protein comprising an amino acid sequence according to SEQ ID NO: 25, and the variant of human OR10H1 is a protein comprising an amino acid sequence having at least 98% sequence identity to the sequence of SEQ ID NO: 25.

19. The compound for use according to any of claims 1 to 8, or 18, the isolated antigen binding construct according to any of claims 9 to 11 or 18, the pharmaceutical composition according to claim 12 or 18, the pharmaceutical composition for use according to claim 13 or 18, the in vitro method according to any of claims 14 to 18, wherein the antigen binding construct is an antibody, antibody-like molecule, or an antigen binding fragment thereof, that specifically binds said human OR10H1, or the variant of human OR10H1.

20. The compound for use, the isolated antigen binding construct, the pharmaceutical composition, the pharmaceutical composition for use, or the in vitro method according to claim 19, wherein the antigen binding construct is a monoclonal antibody.

21. The compound for use, the isolated antigen binding construct, the pharmaceutical composition, the pharmaceutical composition for use, or the in vitro method according to claim 19 or 20, wherein the antigen binding construct is an IgG type antibody.

22. The compound for use, the isolated antigen binding construct, the pharmaceutical composition, the pharmaceutical composition for use, or the in vitro method according to claim 19, wherein the antibody or antibody-like molecule is selected from the list consisting of: intrabodies, chimeric antibodies, fully human antibodies, humanized antibodies, antibody fragments and heteroconjugate antibodies; preferably is selected from the list consisting of: single-chain antibody, chimeric antibody, bifunctional antibody, CDR-grafted antibody, CAR and humanized antibody.

## Patentansprüche

1. Eine Verbindung zur Verwendung bei der Behandlung einer Erkrankung eines Subjekts, wobei die Verbindung ein Modulator der Expression, Funktion und / oder Stabilität von humanem Olfactory Receptor, Family 10, Subfamily H, Member 1 (OR10H1) ist, oder von einer Variante von humanem OR10H1 ist;
wobei die Variante von humanem OR10H1 ein Protein ist, umfassend eine Aminosäuresequenz mit mindestens 95% Sequenzidentität zu der Sequenz von SEQ ID NR: 25; und
wobei die Erkrankung ein humanes OR10H1-positiver Krebs oder ein Krebs ist, der für die Variante von humanem OR10H1 positiv ist; und
wobei die Verbindung ist: (i) eine kleine interferierende RNA (siRNA), die direkt an mRNA des humanen OR10H1 oder der Variante des humanen OR10H1 bindet und so die Expression des menschlichen OR10H1 oder der Variante des menschlichen OR10H1 hemmt; oder (ii) ein Antigen-Bindungskonstrukt, das spezifisch das humane OR10H1 oder die Variante von humanem OR10H1 bindet und das die Resistenz einer Tumor- oder Krebszelle, die durch die Expression von humanem OR10H1 oder der Variante von humanem OR10H1 gekennzeichnet ist, gegen zytotoxische T-Lymphozyten (CTL)-Antworten verringert.

2. Die Verbindung zur Verwendung nach Anspruch 1, wobei die Variante von humanem OR10H1 ausgewählt ist aus der Gruppe bestehend aus einem Ortholog von humanem OR10H1 und einem funktionellen Fragment eines humanen OR10H1-Proteins, wobei das funktionelle Fragment eine zellbasierte Immunantwort auf eine Krebszelle hemmt, die ein solches funktionelles Fragment exprimiert.

3. Die Verbindung zur Verwendung nach Anspruch 1 oder 2, wobei der humanes OR10H1-positive Krebs oder der für die Variante von humanem OR10H1 positive Krebs durch eine pathologische Immunantwort und / oder Expression des humanen OR10H1 oder der Variante von humanem OR10H1 gekennzeichnet ist.

4. Die Verbindung zur Verwendung nach einem der Ansprüche 1 bis 3, wobei der humanes OR10H1-positive Krebs oder der Krebs, der für die Variante von humanem OR10H1 positiv ist, ausgewählt ist aus Melanom, Pankreaskrebs oder Darmkrebs.

5. Die Verbindung zur Verwendung nach einem der Ansprüche 1 bis 4, wobei die Verbindung eine Immunantwort verstärkt, vorzugsweise eine zellvermittelte Immunantwort in dem Subjekt, wie eine T-Zell-vermittelte Immunantwort in dem Subjekt.

6. Die Verbindung zur Verwendung nach einem der Ansprüche 1 bis 5, wobei die Tumor- oder Krebszelle durch eine nachweisbare Zelloberflächenexpression von humanem OR10H1 oder der Variante von humanem OR10H1 gekennzeichnet ist.

7. Die Verbindung zur Verwendung nach einem der Ansprüche 1 bis 6, wobei die Tumor- oder Krebszelle durch eine nachweisbare Zelloberflächenexpression von humanem OR10H1 oder der Variante von humanem OR10H1 gekennzeichnet ist, bevor die Zelle mit der Verbindung in Kontakt gebracht wird.

8. Die Verbindung zur Verwendung nach einem der Ansprüche 1 bis 7, wobei die Tumor- oder Krebszelle durch eine abweichende Expression von humanem OR10H1 oder der Variante von humanem OR10H1 auf einer solchen Zelle im Vergleich zu der in einem gesunden Subjekt oder einer normalen Zelle, gekennzeichnet ist.

9. Ein isoliertes Antigen-Bindungskonstrukt, das ein Antikörper ist, der aus dem Hybridom Di-8A11-H12-E6 (DSMZ-Hinterlegungsnummer: DSM ACC3310, hinterlegt am 26. Oktober 2016) erhältlich ist, oder ein Antigen-Bindungsfragment, das aus einem solchen Antikörper erhältlich ist; wobei das Antigen-Bindungsfragment spezifisch an humanes OR10H1 oder an eine Variante von humanem OR10H1 bindet, wobei die Variante von humanem OR10H1 ein Protein ist, das eine Aminosäuresequenz mit mindestens 95% Sequenzidentität zu der Sequenz von SEQ ID NR: 25 umfasst.

10. Ein isoliertes Antigen-Bindungskonstrukt, das spezifisch an humanes OR10H1 oder an eine Variante von humanem OR10H1 bindet, wobei die Variante von humanem OR10H1 ein Protein ist, das eine Aminosäuresequenz mit mindestens 95% Sequenzidentität zu der Sequenz von SEQ ID NR: 25 umfasst; und
wobei das Antigen-Bindungskonstrukt ein Antikörper oder ein Antigen-Bindungsfragment davon ist, zusammengesetzt aus mindestens einer Sequenz der schweren Kette eines Antikörpers, gepaart mit mindestens einer Sequenz der leichten Kette eines Antikörpers, wobei das Antigen-Bindungskonstrukt ausgewählt ist aus (I), (II) oder (III):
(I) Ein Antikörper oder ein Antigen-bindendes Fragment davon: wobei (Ia) die Sequenz der schweren Kette des Antikörpers CDR1- bis CDR₃-Sequenzen mit den Aminosäuresequenzen von SEQ ID NR: 1 bis 3 umfasst und wobei (Ib) die Sequenz der leichten Kette des Antikörpers CDR1 bis CDR₃-Sequenzen mit den Aminosäuresequenzen der SEQ ID NR: 5 bis 7 umfasst, jeweils einer der CDR unabhängig voneinander gegebenenfalls mit einer Aminosäuresubstitution, Insertion oder Deletion im Vergleich zu diesen Sequenzen;
(II) Ein Antikörper oder ein Antigen-bindendes Fragment davon: wobei (IIa) die Sequenz der schweren Kette des Antikörpers CDR1- bis CDR₃-Sequenzen mit den Aminosäuresequenzen von SEQ ID NR: 9 bis 11 umfasst und wobei (IIb) die Sequenz der leichten Kette des Antikörpers CDR1 bis CDR₃-Sequenzen mit den Aminosäuresequenzen der SEQ ID NR: 13 bis 15 umfasst, jeweils einer der CDR unabhängig voneinander gegebenenfalls mit einer Aminosäuresubstitution, Insertion oder Deletion im Vergleich zu diesen Sequenzen;
(III) Ein Antikörper oder ein Antigen-bindendes Fragment davon: wobei (IIIa) die Sequenz der schweren Kette des Antikörpers CDR1- bis CDR₃-Sequenzen mit den Aminosäuresequenzen von SEQ ID NR: 17 bis 19 umfasst und wobei (IIIb) die Sequenz der leichten Kette des Antikörpers CDR1 bis CDR₃-Sequenzen mit den Aminosäuresequenzen der SEQ ID NR: 21 bis 23 umfasst, jeweils einer der CDR unabhängig voneinander gegebenenfalls mit einer Aminosäuresubstitution, Insertion oder Deletion im Vergleich zu diesen Sequenzen.

11. Das isoliertes Antigen-Bindungskonstrukt nach Anspruch 10:
(I) wobei die Sequenz der schweren Kette des Antikörpers oder Antigen-bindendes Fragment davon von (Ia) gemäß Anspruch 10 eine Sequenz der variablen Region mit der Aminosäuresequenz von SEQ ID NR: 4 umfasst und wobei die Sequenz der leichten Kette des Antikörpers oder Antigen-bindendes Fragment davon von (Ib) gemäß Anspruch 10 eine Sequenz einer variablen Region mit der Aminosäuresequenz von SEQ ID NR: 8 umfasst, jeweils eine Sequenz einer variablen Region unabhängig voneinander nicht mehr als zehn, neun, acht, sieben, sechs, fünf, vier, drei, zwei oder eins, vorzugsweise nicht mehr als drei, Aminosäuresubstitutionen, Insertionen oder Deletionen im Vergleich zu diesen Sequenzen aufweist; oder
(II) wobei die Sequenz der schweren Kette des Antikörpers oder Antigen-bindendes Fragment davon von (IIa) gemäß Anspruch 10 eine Sequenz der variablen Region mit der Aminosäuresequenz von SEQ ID NR: 12 umfasst und wobei die Sequenz der leichten Kette des Antikörpers oder Antigen-bindendes Fragment davon von (IIb) gemäß Anspruch 10 eine Sequenz einer variablen Region mit der Aminosäuresequenz von SEQ ID NR: 16 umfasst, jeweils eine Sequenz einer variablen Region unabhängig voneinander nicht mehr als zehn, neun, acht, sieben, sechs, fünf, vier, drei, zwei oder eins, vorzugsweise nicht mehr als drei, Aminosäuresubstitutionen, Insertionen oder Deletionen im Vergleich zu diesen Sequenzen aufweist; oder
(III) wobei die Sequenz der schweren Kette des Antikörpers oder Antigen-bindendes Fragment davon von (IIIa) gemäß Anspruch 10 eine Sequenz der variablen Region mit der Aminosäuresequenz von SEQ ID NR: 20 umfasst und wobei die Sequenz der leichten Kette des Antikörpers oder Antigen-bindendes Fragment davon von (IIIb) gemäß Anspruch 10 eine Sequenz einer variablen Region mit der Aminosäuresequenz von SEQ ID NR: 24 umfasst, jeweils eine Sequenz einer variablen Region unabhängig voneinander nicht mehr als zehn, neun, acht, sieben, sechs, fünf, vier, drei, zwei oder eins, vorzugsweise nicht mehr als drei, Aminosäuresubstitutionen, Insertionen oder Deletionen im Vergleich zu diesen Sequenzen aufweist.

12. Ein pharmazeutische Zusammensetzung, umfassend das isolierte Antigen-Bindungskonstrukt nach einem der Ansprüche 9 bis 11 oder eine Nukleinsäure oder Nukleinsäuren, die für das Antigen-Bindungskonstrukt kodieren; und einen pharmazeutisch verträglichen Träger, Stabilisator und / oder Hilfsstoff.

13. Eine pharmazeutische Zusammensetzung zur Verwendung bei der Behandlung einer Erkrankung eines Subjekts, wobei die Erkrankung ein für humanes OR10H1-positiver Krebs oder ein für die Variante von humanem OR10H1 positiver Krebs ist, umfassend die in einem der Ansprüche 1 bis 8 genannte Verbindung, das isolierte Antigen-Bindungskonstrukt nach einem der Ansprüche 9 bis 11 oder eine Nukleinsäure oder Nukleinsäuren, die für das Antigen-Bindungskonstrukt kodieren; und
einen pharmazeutisch verträglichen Träger, Stabilisator und / oder Hilfsstoff.

14. Ein in-vitro Verfahren zum Diagnostizieren eines Resistenzphänotyps einer Krebserkrankung gegen eine Immunantwort wie eine zellvermittelte Immunantwort bei einem humanen Subjekt, wobei das Verfahren die folgenden Schritte umfasst:
(a) Bereitstellen einer Probe von Tumor- oder Krebszellen des Subjekts,
(b) Bestimmen der Anwesenheit oder Abwesenheit von Protein oder mRNA von humanem OR10H1 oder einer Variante von humanem OR10H1 in den Tumor- oder Krebszellen, wobei die Variante von humanem OR10H1 ein Protein ist, das eine Aminosäuresequenz mit mindestens 95% Sequenzidentität zu der Sequenz von SEQ ID NR: 25 umfasst, und
(c) Diagnostizieren eines Resistenzphänotyps der Krebserkrankung gegen eine Immunantwort in dem Subjekt, wenn Protein oder mRNA des humanen OR10H1 oder der Variante des humanen OR10H1 in den Tumor- oder Krebszellen vorhanden ist.

15. Das Verfahren nach Anspruch 14, wobei die Anwesenheit einer Menge oder eines Levels des Proteins oder der mRNA von humanem OR10H1 oder der Variante von humanem OR10H1 in Schritt (b) als eine gemessene Menge bestimmt wird.

16. Das in-vitro Verfahren nach Anspruch 14 oder 15, wobei Schritt (b) das Inkontaktbringen der Probe mit Mitteln zum Nachweis von Protein oder mRNA des humanen OR10H1 oder der Variante des humanen OR10H1 umfasst, wobei die Mittel vorzugsweise ausgewählt sind aus: (i) einem Antigen-Bindungskonstrukt, das spezifisch an das Protein von humanem OR10H1 oder der Variante von humanem OR10H1 bindet und das die Bindung zwischen dem Antigen-Bindungskonstrukt und dem Protein von humanem OR10H1 oder der Variante von humanem OR10H1 nachweist; und / oder (ii) eine Nukleinsäure, die an die mRNA-Sequenz von humanem OR10H1 oder der Variante von humanem OR10H1 bindet und die Bindung zwischen der Nukleinsäure und der mRNA von humanem OR10H1 oder der Variante von humanem OR10H1 nachweist.

17. Ein in-vitro-Verfahren zur Identifizierung einer Verbindung, die zur Behandlung einer Erkrankung geeignet ist, die durch die Expression von humanem OR10H1 oder einer Variante davon gekennzeichnet ist, wobei die Variante von humanem OR10H1 ein Protein ist, das eine Aminosäuresequenz mit mindestens 95% Sequenzidentität gemäß der Sequenz von SEQ ID NR: 25 umfasst, wobei das Verfahren die folgenden Schritte umfasst:
(a) Bereitstellen einer ersten Zelle, die ein Protein oder eine mRNA des humanen OR10H1 oder der Variante des humanen OR10H1 exprimiert, wobei die erste Zelle eine Tumorzelle oder eine von einem Tumor abgeleitete Zelle ist, und
(b) Bereitstellen einer Kandidatenverbindung und
(c) gegebenenfalls Bereitstellen einer zweiten Zelle, die eine cytotoxische Immunzelle ist, beispielsweise ein cytotoxischer T-Lymphozyt (CTL), der in der Lage ist, die erste Zelle immunologisch zu erkennen, und
(d) Inkontaktbringen der ersten Zelle und der Kandidatenverbindung und gegebenenfalls der zweiten Zelle und
(e) Bestimmen nachfolgend Schritt (d) von einem oder beidem von:
(i) Expression, Funktion und / oder Stabilität des Proteins oder der mRNA des humanen OR10H1 oder der Variante des humanen OR10H1 in der ersten Zelle, wobei eine reduzierte Expression, Funktion und / oder Stabilität des Proteins oder der mRNA des humanen OR10H1 oder der Variante von humanem OR10H1 in der ersten Zelle, die mit der Kandidatenverbindung in Kontakt gebracht wurde, im Vergleich zu der ersten Zelle, die nicht mit der Kandidatenverbindung in Kontakt gebracht wurde, anzeigt, dass die Kandidatenverbindung eine Verbindung ist, die zur Behandlung einer Erkrankung geeignet ist, die durch die Expression von genanntem Protein oder mRNA des humanen OR10H1 oder der Variante des humanen OR10H1 gekennzeichnet ist; und / oder
(ii) Zytotoxizität der zweiten Zelle gegen die erste Zelle, wobei eine erhöhte Zytotoxizität der zweiten Zelle gegen die erste Zelle, die mit der Kandidatenverbindung in Kontakt gebracht wurde, im Vergleich zur Zytotoxizität der zweiten Zelle gegen die erste Zelle, die nicht mit der Kandidatenverbindung in Kontakt gebracht wurde, anzeigt, dass die Kandidatenverbindung eine Verbindung ist, die zur Behandlung einer Erkrankung geeignet ist, die durch Expression des humanen OR10H1 oder der Variante des humanen OR10H1 gekennzeichnet ist.

18. Die Verbindung zur Verwendung nach einem der Ansprüche 1 bis 8, das isolierte Antigen-Bindungskonstrukt nach einem der Ansprüche 9 bis 11, die pharmazeutische Zusammensetzung nach Anspruch 12, die pharmazeutische Zusammensetzung zur Verwendung nach Anspruch 13, das in vitro Verfahren nach einem der Ansprüche 14 bis 17, wobei das humane OR10H1 ein Protein ist, das eine Aminosäuresequenz gemäß SEQ ID NR: 25 umfasst, und wobei die Variante des humanen OR10H1 ein Protein ist, das eine Aminosäuresequenz mit mindestens 98% Sequenzidentität zu der Sequenz von SEQ ID NR: 25 umfasst.

19. Die Verbindung zur Verwendung nach einem der Ansprüche 1 bis 8 oder 18, das isoliertes Antigen-Bindungskonstrukt nach einem der Ansprüche 9 bis 11 oder 18, die pharmazeutische Zusammensetzung nach Anspruch 12 oder 18, die pharmazeutische Zusammensetzung zur Verwendung nach Anspruch 13 oder 18, das in-vitro-Verfahren nach einem der Ansprüche 14 bis 18, wobei das Antigen-Bindungskonstrukt ein Antikörper, ein Antikörper-ähnliches Molekül oder ein Antigen-Bindungsfragment davon ist, das spezifisch das humane OR10H1 oder die Variante von humanem OR10H1 bindet.

20. Die Verbindung zur Verwendung, das isolierte Antigen-Bindungskonstrukt, die pharmazeutische Zusammensetzung, die pharmazeutische Zusammensetzung zur Verwendung oder das in-vitro-Verfahren, nach Anspruch 19, wobei das Antigen-Bindungskonstrukt ein monoklonaler Antikörper ist.

21. Die Verbindung zur Verwendung, das isolierte Antigen-Bindungskonstrukt, die pharmazeutische Zusammensetzung, die pharmazeutische Zusammensetzung zur Verwendung oder das in-vitro-Verfahren, nach Anspruch 19 oder 20, wobei das Antigen-Bindungskonstrukt ein Antikörper vom IgG-Typ ist.

22. Die Verbindung zur Verwendung, das isolierte Antigen-Bindungskonstrukt, die pharmazeutische Zusammensetzung, die pharmazeutische Zusammensetzung zur Verwendung oder das in-vitro-Verfahren, nach Anspruch 19, wobei der Antikörper oder das Antikörper-ähnliche Molekül ausgewählt ist aus der Liste bestehend aus: Intrabodies chimäre Antikörper, vollständig humane Antikörper, humanisierte Antikörper, Antikörperfragmente und heterokonjugierte Antikörper; bevorzugt ist ausgewählt aus der Liste bestehend aus: einkettigem Antikörper, chimärem Antikörper, bifunktionalem Antikörper, CDR-gegrafteten Antikörper, CAR und humanisiertem Antikörper.

## Revendications

1. Composé destiné à être utilisé dans le traitement d'une maladie chez un sujet, le composé étant un modulateur de l'expression, de la fonction et/ou de la stabilité du récepteur olfactif humain, famille 10, sous-famille H, membre 1 (OR10H1), ou d'une variante de l'OR10H1 humain ;
dans lequel le variant de I'OR10H1 humain est une protéine comprenant une séquence d'acides aminés présentant au moins 95 % d'identité de séquence par rapport à la séquence de SEQ ID N° : 25 ; et
dans lequel la maladie est un cancer positif de I'OR10H1 humain ou un cancer positif du variant de I'OR10H1 humain ; et
dans lequel le composé est : (i) un petit ARN interférant (ARNsi) qui se lie directement à l'ARNm dudit OR10H1 humain ou du variant de l'OR10H1 humain en inhibant ainsi l'expression dudit OR10H1 humain ou du variant de I'OR10H1 humain ; ou (ii) une construction de liaison à un antigène qui se lie spécifiquement audit OR10H1 humain ou au variant de I'OR10H1 humain et qui réduit la résistance d'une cellule tumorale ou cancéreuse, **caractérisée par** l'expression de l'OR10H1 humain, ou du variant de l'OR10H1 humain, aux réponses des lymphocytes T cytotoxiques (CTL).

2. Composé destiné à être utilisé selon la revendication 1, dans lequel le variant de l'OR10H1 humain est choisi dans le groupe constitué par un orthologue de l'OR10H1 humain et un fragment fonctionnel d'une protéine OR10H1 humaine, ledit fragment fonctionnel inhibant une réponse immunitaire à base de cellules vis-à-vis d'une cellule cancéreuse qui exprime un tel fragment fonctionnel.

3. Composé destiné à être utilisé selon la revendication 1 ou 2, dans lequel le cancer positif de l'OR10H1 humain ou le cancer positif du variant de l'OR10H1 humain est **caractérisé par** une réponse immunitaire pathologique et/ou par l'expression dudit OR10H1 humain ou du variant de l'OR10H1 humain.

4. Composé destiné à être utilisé selon l'une quelconque des revendications 1 à 3, dans lequel le cancer positif de l'OR10H1 humain ou le cancer positif du variant de l'OR10H1 humain est choisi parmi le mélanome, le cancer du pancréas ou le cancer colorectal.

5. Composé destiné à être utilisé selon l'une quelconque des revendications 1 à 4, dans lequel le composé améliore une réponse immunitaire, de préférence une réponse immunitaire à médiation cellulaire chez le sujet, telle qu'une réponse immunitaire à médiation par des cellules T chez le sujet.

6. Composé destiné à être utilisé selon l'une quelconque des revendications 1 à 5, dans lequel la tumeur ou la cellule cancéreuse est **caractérisée par** une expression détectable à la surface des cellules de l'OR10H1 humain ou du variant de l'OR10H1 humain.

7. Composé destiné à être utilisé selon l'une quelconque des revendications 1 à 6, dans lequel la tumeur ou la cellule cancéreuse est **caractérisée par** une expression détectable à la surface des cellules de l'OR10H1 humain ou du variant de l'OR10H1 humain, avant la mise en contact de la cellule avec le composé.

8. Composé destiné à être utilisé selon l'une quelconque des revendications 1 à 7, dans lequel la tumeur ou la cellule cancéreuse est **caractérisée par** l'expression aberrante de l'OR10H1 humain ou du variant de l'OR10H1 humain sur cette cellule par rapport à celle d'un sujet en bonne santé ou à une cellule normale.

9. Construction de liaison à un antigène isolé qui est un anticorps pouvant être obtenu à partir de l'hybridome Di-8A11-H12-E6 (DSMZ numéro de dépôt : DSM ACC3310, déposé le 26 octobre 2016), ou un fragment de liaison d'antigène obtenu à partir d'un tel anticorps ; dans laquelle ledit fragment de liaison à l'antigène se lie spécifiquement à l'OR10H1 humain ou à un variant de l'OR10H1 humain, le variant de l'OR10H1 humain étant une protéine comprenant une séquence d'acides aminés présentant au moins 95 % d'identité de séquence par rapport à la séquence de SEQ ID N° : 25.

10. Construction de liaison à un antigène isolé qui se lie spécifiquement à l'OR10H1 humain ou à un variant de l'OR10H1 humain, dans laquelle le variant de l'OR10H1 humain est une protéine comprenant une séquence d'acides aminés présentant au moins 95 % d'identité de séquence par rapport à la séquence de SEQ ID N° : 25 ; et
la construction de liaison à l'antigène étant un anticorps ou l'un de ses fragments de liaison à un antigène, composé d'au moins une séquence de chaîne lourde d'anticorps, appariée avec au moins une séquence de chaîne légère d'anticorps, la construction de liaison à l'antigène étant choisie parmi les éléments (I), (II) ou (III) suivants :
(I) un anticorps ou un fragment de liaison à l'antigène de celui-ci : (la) ladite séquence de chaîne lourde d'anticorps comprenant les séquences CDR1 à CDR3 comportant les séquences d'acides aminés de SEQ ID N° : 1 à 3, et (Ib) ladite séquence de chaîne légère d'anticorps comprenant des séquences CDR1 à CDR3 comportant les séquences d'acides aminés de SEQ ID NO : 5 à 7, dans chaque cas d'une CDR indépendamment éventuellement avec une substitution, une insertion ou une délétion d'acides aminés par rapport à ces séquences ;
(II) un anticorps, ou un fragment de liaison à l'antigène de celui-ci, (la) ladite séquence de chaîne lourde d'anticorps comprenant les séquences CDR1 à CDR3 comportant les séquences d'acides aminés de SEQ ID N° : 9 à 11, et (IIb) ladite séquence de chaîne légère d'anticorps comprenant des séquences CDR1 à CDR3 comportant les séquences d'acides aminés de SEQ ID NO : 13 à 15, dans chaque cas d'une CDR indépendamment éventuellement avec une substitution, une insertion ou une délétion d'acides aminés par rapport à ces séquences ;
(III) un anticorps, ou un fragment de liaison à l'antigène de celui-ci, (IIIa) ladite séquence de chaîne lourde d'anticorps comprenant les séquences CDR1 à CDR3 comportant les séquences d'acides aminés de SEQ ID N° : 17 à 19, et (IIIb) ladite séquence de chaîne légère d'anticorps comprenant des séquences CDR1 à CDR3 comportant les séquences d'acides aminés de SEQ ID NO : 21 à 23, dans chaque cas d'une CDR indépendamment éventuellement avec une substitution, une insertion ou une délétion d'acides aminés par rapport à ces séquences.

11. Construction de liaison à un antigène isolé selon la revendication 10 :
(I) dans laquelle ladite séquence de chaîne lourde d'anticorps, ou un fragment de liaison à l'antigène de celui-ci, de (la) selon la revendication 10 comprend une région variable comportant la séquence d'acides aminés de SEQ ID N° : 4, et dans laquelle ladite séquence de chaîne légère d'anticorps ou l'un de ses fragments de liaison à l'antigène de (Ib) selon la revendication 10 comprend une séquence de région variable comportant la séquence d'acides aminés de SEQ ID N° : 8, dans chaque cas d'une séquence de région variable indépendamment avec pas plus de dix, neuf, huit, sept, six, cinq, quatre, trois, deux ou un, de préférence pas plus de trois substitutions, insertions ou délétions d'acides aminés par rapport à ces séquences ; ou
(II) dans laquelle ladite séquence de chaîne lourde d'anticorps, ou un fragment de liaison à l'antigène de celui-ci, de (la) selon la revendication 10 comprend une région variable comportant la séquence d'acides aminés de SEQ ID N° : 12, et dans laquelle ladite séquence de chaîne légère d'anticorps ou l'un de ses fragments de liaison à l'antigène de (IIb) selon la revendication 10 comprend une séquence de région variable comportant la séquence d'acides aminés de SEQ ID N° : 16, dans chaque cas d'une séquence de région variable indépendamment avec pas plus de dix, neuf, huit, sept, six, cinq, quatre, trois, deux ou un, de préférence pas plus de trois substitutions, insertions ou délétions d'acides aminés par rapport à ces séquences ; ou
(III) dans laquelle ladite séquence de chaîne lourde d'anticorps, ou un fragment de liaison à l'antigène de celui-ci, de (IIIa) selon la revendication 10 comprend une région variable comportant la séquence d'acides aminés de SEQ ID N° : 20, et dans laquelle ladite séquence de chaîne légère d'anticorps ou l'un de ses fragments de liaison à l'antigène de (IIIb) selon la revendication 10 comprend une séquence de région variable comportant la séquence d'acides aminés de SEQ ID N° : 24, dans chaque cas d'une séquence de région variable indépendamment avec pas plus de dix, neuf, huit, sept, six, cinq, quatre, trois, deux ou un, de préférence pas plus de trois substitutions, insertions ou délétions d'acides aminés par rapport à ces séquences.

12. Composition pharmaceutique comprenant la construction de liaison à un antigène isolé selon l'une quelconque des revendications 9 à 11, ou acide nucléique ou acides nucléiques codant ladite construction de liaison à l'antigène ; et vecteur, stabilisant et/ou excipient pharmaceutiquement acceptable.

13. Composition pharmaceutique destinée à être utilisée dans le traitement d'une maladie d'un sujet, la maladie étant un cancer humain positif de l'OR10H1 ou un cancer positif du variant de l'OR10H1 humain, comprenant
le composé selon l'une quelconque des revendications 1 à 8, la construction de liaison à l'antigène isolé selon l'une quelconque des revendications 9 à 11 ou un acide nucléique ou des acides nucléiques codant ladite construction de liaison à l'antigène ; et
un vecteur, un stabilisant et/ou un excipient pharmaceutiquement acceptable.

14. Procédé in vitro de diagnostic d'un phénotype de résistance d'une maladie cancéreuse contre une réponse immunitaire, telle qu'une réponse immunitaire à médiation cellulaire chez un sujet humain, le procédé comprenant les étapes
(a) d'utilisation d'un échantillon de cellules tumorales ou cancéreuses du sujet,
(b) de détermination de la présence ou de l'absence de la protéine ou de l'ARNm de l'OR10H1 humain, ou d'un variant de l'OR10H1 humain dans les cellules tumorales ou cancéreuses, le variant de l'OR10H1 humain étant une protéine comprenant une séquence d'acides aminés présentant au moins 95 % d'identité de séquence par rapport à la séquence de SEQ ID N° : 25, et
(c)de diagnostic d'un phénotype de résistance de la maladie cancéreuse contre une réponse immunitaire chez le sujet lorsque la protéine ou l'ARNm dudit OR10H1 humain ou du variant de l'OR10H1 humain se trouve dans les cellules tumorales ou cancéreuses.

15. Procédé selon la revendication 14, dans lequel la présence d'une quantité ou d'un niveau de ladite protéine ou dudit ARNm de l'OR10H1 humain ou du variant de l'OR10H1 humain est déterminée à l'étape (b) comme quantité mesurée.

16. Procédé in vitro selon la revendication 14 ou 15, dans lequel l'étape (b) implique la mise en contact de l'échantillon avec un moyen de détection de la protéine ou de l'ARNm dudit OR10H1 humain ou du variant de l'OR10H1 humain, ledit moyen est de préférence choisi parmi : (i) une construction de liaison à un antigène se liant spécifiquement à ladite protéine de l'OR10H1 humain ou du variant de l'OR10H1 humain et la détection de la liaison entre ladite construction de liaison à l'antigène et ladite protéine de l'OR10H1 humain ou du variant de l'OR10H1 humain ; et/ou (ii) un acide nucléique se liant à ladite séquence d'ARNm de l'OR10H1 humain ou du variant de l'OR10H1 humain et la détection de la liaison entre ledit acide nucléique et ledit ARNm de l'OR10H1 humain ou du variant de l'OR10H1 humain.

17. Procédé in vitro d'identification d'un composé approprié pour le traitement d'une maladie **caractérisée par** l'expression de l'OR10H1 humain ou d'un variant de celui-ci, dans lequel le variant de l'OR10H1 humain est une protéine comprenant une séquence d'acides aminés présentant au moins 95 % d'identité de séquence par rapport à la séquence de SEQ ID N° : 25, le procédé comprenant les étapes
(a) d'utilisation d'une première cellule exprimant une protéine ou un ARNm dudit OR10H1 humain ou du variant de l'OR10H1 humain, dans lequel ladite première cellule est une cellule tumorale ou une cellule dérivée d'une tumeur, et
(b) d'utilisation d'un composé candidat, et
(c) éventuellement, d'utilisation d'une seconde cellule qui est une cellule immunitaire cytotoxique, par exemple un lymphocyte T cytotoxique (CTL), capable de reconnaître immunologiquement ladite première cellule, et
(d) de mise en contact de la première cellule et du composé candidat, et éventuellement de la seconde cellule, et
(e) de détermination ultérieure à l'étape (d) :
(i) de l'expression, de la fonction et/ou de la stabilité de ladite protéine ou de l'ARNm dudit OR10H1 humain, ou du variant de l'OR10H1 humain, dans ladite première cellule, une expression, une fonction et/ou une stabilité réduite de ladite protéine ou de l'ARNm dudit OR10H1 humain ou du variant de l'OR10H1 humain dans ladite première cellule mise en contact avec le composé candidat par rapport à ladite première cellule non mise en contact avec ledit composé candidat indiquant que le composé candidat est un composé approprié pour le traitement d'une maladie **caractérisée par** l'expression de ladite protéine ou dudit ARNm dudit OR10H1 humain ou du variant de l'OR10H1 humain ; et/ou
(ii) de la cytotoxicité de ladite seconde cellule contre ladite première cellule, une cytotoxicité accrue de ladite seconde cellule contre ladite première cellule mise en contact avec le composé candidat par rapport à la cytotoxicité de ladite seconde cellule contre ladite première cellule non mise en contact avec le composé candidat indiquant que le composé candidat est un composé approprié pour le traitement d'une maladie **caractérisée par** l'expression dudit OR10H1 humain ou du variant de l'OR10H1 humain.

18. Composé destiné à être utilisé selon l'une quelconque des revendications 1 à 8, construction de liaison à un antigène isolé selon l'une quelconque des revendications 9 à 11, composition pharmaceutique selon la revendication 12, composition pharmaceutique destinée à être utilisée selon la revendication 13, procédé in vitro selon l'une quelconque des revendications 14 à 17, dans lesquels ledit OR10H1 humain est une protéine comprenant une séquence d'acides aminés selon SEQ ID N° : 25, et le variant de l'OR10H1 humain est une protéine comprenant une séquence d'acides aminés présentant au moins 98 % d'identité de séquence par rapport à la séquence de SEQ ID N° : 25.

19. Composé destiné à être utilisé selon l'une quelconque des revendications 1 à 8 ou 18, construction de liaison à un antigène isolé selon l'une quelconque des revendications 9 à 11 ou 18 composition pharmaceutique selon la revendication 12 ou 18, composition pharmaceutique destinée à être utilisée selon la revendication 13 ou 18, procédé in vitro selon l'une quelconque des revendications 14 à 18, dans lesquels la construction de liaison à l'antigène est un anticorps, une molécule de type anticorps ou l'un de ses fragments de liaison à l'antigène, qui se lie spécifiquement audit OR10H1 humain ou au variant de l'OR10H1 humain.

20. Composé destiné à être utilisé, construction de liaison à un antigène isolé, composition pharmaceutique, composition pharmaceutique destinée à être utilisée ou procédé in vitro selon la revendication 19, dans lesquels la construction de liaison à l'antigène est un anticorps monoclonal.

21. Composé destiné à être utilisé, construction de liaison à un antigène isolé, composition pharmaceutique, composition pharmaceutique destinée à être utilisée ou procédé in vitro selon la revendication 19 ou 20, dans lesquels la construction de liaison à l'antigène est un anticorps de type IgG.

22. Composé destiné à être utilisé, construction de liaison à un antigène isolé, composition pharmaceutique, composition pharmaceutique destinée à être utilisée ou procédé in vitro selon la revendication 19, dans lesquels l'anticorps ou la molécule de type anticorps est choisi dans la liste constituée par : des anticorps, des anticorps chimériques, des anticorps entièrement humains, des anticorps humanisés, des fragments d'anticorps et des anticorps hétéroconjugués ; de préférence choisi dans la liste constituée par : un anticorps à chaîne unique, un anticorps chimérique, un anticorps bifonctionnel, un anticorps greffé CDR, un CAR et un anticorps humanisé.


### SEQUENCE LISTING

<110> Deutsches Krebsforschungszentrum Stiftung des oeffentlichen Rechts
<120> IMMUNE MODULATORS FOR REDUCING IMMUNE-RESISTANCE IN MELANOMA AND OTHER PROLIFERATIVE DISEASES
<130> D31508EP
<160> 34
<170> PatentIn version 3.5
<210> 1
   <211> 7
   <212> PRT
   <213> Rattus norvegicus
<400> 1
   Phe Thr Phe Asp Asn Ala Trp
   1               5
<210> 2
   <211> 10
   <212> PRT
   <213> Rattus norvegicus
<400> 2
   Ile Lys Ala Lys Ser Asn Asn Tyr Ala Thr
   1               5                   10
<210> 3
   <211> 6
   <212> PRT
   <213> Rattus norvegicus
<400> 3
   Thr Arg Leu Tyr Tyr Asp
   1               5
<210> 4
   <211> 115
   <212> PRT
   <213> Rattus norvegicus
<400> 4
   Glu Val Gln Leu Val Glu Thr Gly Gly Asn Leu Val Gln Pro Gly Lys
   1               5                   10                  15
   Ser Leu Lys Leu Thr Cys Ala Thr Ser Gly Phe Thr Phe Asp Asn Ala
               20                  25                  30
   Trp Met His Trp Val Arg Gln Ser Pro Glu Lys Gln Leu Glu Trp Val
           35                  40                  45
   Ala Gln Ile Lys Ala Lys Ser Asn Asn Tyr Ala Thr Tyr Tyr Ala Glu
       50                  55                  60
   Ser Val Lys Gly Arg Phe Thr Ile Ser Arg Asp Asp Ser Lys Ser Ser
   65                  70                  75                  80
   Val Tyr Leu Gln Met Asn Arg Leu Lys Glu Glu Asp Thr Ala Ile Tyr
                   85                  90                  95
   Tyr Cys Thr Arg Leu Tyr Tyr Asp Trp Gly Gln Gly Val Met Val Thr
               100                 105                 110
   Val Ser Ser
           115
<210> 5
   <211> 6
   <212> PRT
   <213> Rattus norvegicus
<400> 5
   Gln Asp Ile Gly Asn Tyr
   1               5
<210> 6
   <211> 3
   <212> PRT
   <213> Rattus norvegicus
<400> 6
   Arg Ala Thr
   1
<210> 7
   <211> 8
   <212> PRT
   <213> Rattus norvegicus
<400> 7
   Leu Gln His Lys Gln Tyr Pro Leu
   1 5
<210> 8
   <211> 108
   <212> PRT
   <213> Rattus norvegicus
<400> 8
   Asp Ile Gln Met Thr Gln Ser Pro Ser Ser Ile Ser Val Ser Leu Gly
   1 5 10 15
   Asp Arg Phe Thr Phe Thr Cys Arg Ala Ser Gln Asp Ile Gly Asn Tyr
   20 25 30
   Leu Ser Trp Phe Gln Gln Lys Pro Glu Lys Ser Pro Lys Leu Met Ile
   35 40 45
   Tyr Arg Ala Thr Asn Leu Glu Asp Gly Val Pro Ser Arg Phe Ser Gly
   50 55 60
   Ser Arg Ser Gly Ser Asp Tyr Ser Leu Thr Ile Asn Ser Leu Glu Ser
   65 70 75 80
   Glu Asp Thr Gly Ile Tyr Phe Cys Leu Gln His Lys Gln Tyr Pro Leu
   85 90 95
   Thr Phe Gly Ser Gly Thr Lys Leu Glu Ile Lys Arg
   100 105
<210> 9
   <211> 7
   <212> PRT
   <213> Rattus norvegicus
<400> 9
   Phe Thr Phe Ser Asn Tyr Asp
   1 5
<210> 10
   <211> 10
   <212> PRT
   <213> Rattus norvegicus
<400> 10
   Ile Ser His Ser Gly Asp Ser Thr Tyr Phe
   1 5 10
<210> 11
   <211> 6
   <212> PRT
   <213> Rattus norvegicus
<400> 11
   His Gly Val Tyr Thr Asn
   1 5
<210> 12
   <211> 122
   <212> PRT
   <213> Rattus norvegicus
<400> 12
   Glu Val Gln Leu Gln Glu Ser Gly Gly Gly Leu Ile Gln Pro Gly Arg
   1 5 10 15
   Ser Leu Lys Leu Ser Cys Ala Ala Ser Gly Phe Thr Phe Ser Asn Tyr
   20 25 30
   Asp Met Ala Trp Val Arg Gln Ala Pro Thr Lys Gly Leu Glu Trp Val
   35 40 45
   Ala Ser Ile Ser His Ser Gly Asp Ser Thr Tyr Phe Arg Ala Ser Val
   50 55 60
   Lys Gly Arg Phe Thr Val Ser Arg Asp Asn Ala Lys Ser Ser Leu Tyr
   65 70 75 80
   Leu Gln Met Asp Ser Leu Arg Ser Glu Asp Thr Ala Thr Tyr Tyr Cys
   85 90 95
   Ala Arg His Gly Val Tyr Thr Asn Tyr Gly Ile Trp Phe Ala Tyr Trp
   100 105 110
   Gly Gln Gly Thr Leu Val Thr Val Ser Ser
   115 120
<210> 13
   <211> 6
   <212> PRT
   <213> Rattus norvegicus
<400> 13
   Gln Asp Ile Gly Asn Tyr
   1 5
<210> 14
   <211> 3
   <212> PRT
   <213> Rattus norvegicus
<400> 14
   Arg Ala Thr
   1
<210> 15
   <211> 8
   <212> PRT
   <213> Rattus norvegicus
<400> 15
   Leu Gln His Lys Gln Tyr Pro Leu
   1               5
<210> 16
   <211> 108
   <212> PRT
   <213> Rattus norvegicus
<400> 16
   Asp Ile Gln Met Thr Gln Ser Pro Ser Ser Ile Ser Val Ser Leu Gly
   1               5                   10                  15
   Asp Arg Phe Thr Phe Thr Cys Arg Ala Ser Gln Asp Ile Gly Asn Tyr
               20                  25                  30
   Leu Ser Trp Phe Gln Gln Lys Pro Glu Lys Ser Pro Lys Leu Met Ile
           35                  40                  45
   Tyr Arg Ala Thr Asn Leu Glu Asp Gly Val Pro Ser Arg Phe Ser Gly
       50                  55                  60
   Ser Arg Ser Gly Ser Asp Tyr Ser Leu Thr Ile Asn Ser Leu Glu Ser
   65                  70                  75                  80
   Glu Asp Thr Gly Ile Tyr Phe Cys Leu Gln His Lys Gln Tyr Pro Leu
                   85                  90                  95
   Thr Phe Gly Ser Gly Thr Lys Leu Glu Ile Lys Arg
               100                 105
<210> 17
   <211> 7
   <212> PRT
   <213> Rattus norvegicus
<400> 17
   Phe Thr Phe Ser Ser Ala Trp
   1 5
<210> 18
   <211> 10
   <212> PRT
   <213> Rattus norvegicus
<400> 18
   Ile Lys Gly Lys Ser Asn Asn Tyr Ala Thr
   1 5 10
<210> 19
   <211> 8
   <212> PRT
   <213> Rattus norvegicus
<400> 19
   Thr Trp Phe Gly Pro Met Asp Ala
   1 5
<210> 20
   <211> 117
   <212> PRT
   <213> Rattus norvegicus
<400> 20
   Glu Val Gln Leu Gln Glu Ser Gly Gly Arg Leu Val Gln Pro Gly Lys
   1               5                   10                  15
   Ser Leu Lys Leu Thr Cys Ala Ala Ser Gly Phe Thr Phe Ser Ser Ala
               20                  25                  30
   Trp Ile His Trp Val Arg Gln Ser Pro Glu Lys Gln Leu Glu Trp Val
           35                  40                  45
   Ala Gln Ile Lys Gly Lys Ser Asn Asn Tyr Ala Thr Tyr Tyr Ala Glu
       50                  55                  60
   Ser Val Lys Gly Arg Phe Thr Ile Ser Arg Asp Asp Ser Lys Ser Ser
   65                  70                  75                  80
   Val Tyr Leu Gln Met Asn Ser Leu Lys Glu Glu Asp Thr Ala Ile Tyr
                   85                  90                  95
   Tyr Cys Thr Trp Phe Gly Pro Met Asp Ala Trp Gly Gln Gly Ala Ser
               100                 105                 110
   Val Thr Val Ser Ser
           115
<210> 21
   <211> 11
   <212> PRT
   <213> Rattus norvegicus
<400> 21
   Lys Ser Leu Leu His Asn Asn Gly Lys Thr Phe
   1               5                   10
<210> 22
   <211> 3
   <212> PRT
   <213> Rattus norvegicus
<400> 22
   Trp Met Ser
   1
<210> 23
   <211> 8
   <212> PRT
   <213> Rattus norvegicus
<400> 23
   Gln Gln Phe Leu Glu Tyr Pro Leu
   1 5
<210> 24
   <211> 113
   <212> PRT
   <213> Rattus norvegicus
<400> 24
   Asp Ile Val Met Thr Gln Gly Ala Leu Pro Asn Pro Val Pro Ser Gly
   1 5 10 15
   Glu Ser Ala Ser Ile Thr Cys Gln Ser Ser Lys Ser Leu Leu His Asn
   20 25 30
   Asn Gly Lys Thr Phe Leu Asn Trp Tyr Leu Gln Arg Pro Gly Gln Ser
   35 40 45
   Pro Gln Leu Leu Ile Tyr Trp Met Ser Thr Arg Ala Ser Gly Val Ser
   50 55 60
   Asp Arg Phe Ser Gly Ser Gly Ser Gly Ala Asp Phe Thr Leu Lys Ile
   65 70 75 80
   Ser Ser Val Glu Ala Glu Asp Val Gly Val Tyr Tyr Cys Gln Gln Phe
   85 90 95
   Leu Glu Tyr Pro Leu Thr Phe Gly Ser Gly Thr Lys Leu Glu Ile Lys
   100 105 110
   Arg
<210> 25
   <211> 318
   <212> PRT
   <213> Homo sapiens
<400> 25
   Met Gln Arg Ala Asn His Ser Thr Val Thr Gln Phe Ile Leu Val Gly
   1               5                   10                  15
   Phe Ser Val Phe Pro His Leu Gln Leu Met Leu Phe Leu Leu Phe Leu
               20                  25                  30
   Leu Met Tyr Leu Phe Thr Leu Leu Gly Asn Leu Leu Ile Met Ala Thr
           35                  40                  45
   Val Trp Ser Glu Arg Ser Leu His Thr Pro Met Tyr Leu Phe Leu Cys
       50                  55                  60
   Ala Leu Ser Val Ser Glu Ile Leu Tyr Thr Val Ala Ile Ile Pro Arg
   65                  70                  75                  80
   Met Leu Ala Asp Leu Leu Ser Thr Gln Arg Ser Ile Ala Phe Leu Ala
                   85                  90                  95
   Cys Ala Ser Gln Met Phe Phe Ser Phe Ser Phe Gly Phe Thr His Ser
               100                 105                 110
   Phe Leu Leu Thr Val Met Gly Tyr Asp Arg Tyr Val Ala Ile Cys His
           115                 120                 125
   Pro Leu Arg Tyr Asn Val Leu Met Ser Pro Arg Gly Cys Ala Cys Leu
       130                 135                 140
   Val Gly Cys Ser Trp Ala Gly Gly Leu Val Met Gly Met Val Val Thr
   145                 150                 155                 160
   Ser Ala Ile Phe His Leu Ala Phe Cys Gly His Lys Glu Ile His His
                   165                 170                 175
   Phe Ala Cys His Val Pro Pro Leu Leu Lys Leu Ala Cys Gly Asp Asp
               180                 185                 190
   Val Leu Val Val Ala Lys Gly Val Gly Leu Val Cys Ile Thr Ala Leu
           195                 200                 205
   Leu Gly Cys Phe Leu Leu Ile Leu Leu Ser Tyr Ala Phe Ile Val Ala
       210                 215                 220
   Ala Ile Leu Lys Ile Pro Ser Ala Glu Gly Arg Asn Lys Ala Phe Ser
   225                 230                 235                 240
   Thr Cys Ala Ser His Leu Thr Val Val Val Val His Tyr Gly Phe Ala
   245 250 255
   Ser Val Ile Tyr Leu Lys Pro Lys Ser Pro Gln Ser Leu Glu Gly Asp
   260 265 270
   Thr Leu Met Gly Ile Thr Tyr Thr Val Leu Thr Pro Phe Leu Ser Pro
   275 280 285
   Ile Ile Phe Ser Leu Arg Asn Lys Glu Leu Lys Val Ala Met Lys Lys
   290 295 300
   Thr Phe Phe Ser Lys Leu Tyr Pro Glu Lys Asn Val Met Met
   305 310 315
<210> 26
   <211> 19
   <212> RNA
   <213> Artificial
<220>
   <223> siRNA
<400> 26
   ggagacaccu ugaugggca 19
<210> 27
   <211> 19
   <212> RNA
   <213> Artificial
<220>
   <223> siRNA
<400> 27
   aguaaacucu acccagaaa 19
<210> 28
   <211> 19
   <212> RNA
   <213> Artificial
<220>
   <223> siRNA
<400> 28
   gcagagagcc aaucacucc 19
<210> 29
   <211> 19
   <212> RNA
   <213> Artificial
<220>
   <223> siRNA
<400> 29
   ggucgugcac uauggcuuu 19
<210> 30
   <211> 21
   <212> DNA
   <213> Artificial
<220>
   <223> shRNA
<400> 30
   gttcctgctg atgtacctgt t 21
<210> 31
   <211> 21
   <212> DNA
   <213> Artificial
<220>
   <223> shRNA
<400> 31
   tgcgctacaa cgtgctcatg a 21
<210> 32
   <211> 21
   <212> DNA
   <213> Artificial
<220>
   <223> shRNA
<400> 32
   tggctttgcc tccgtcattt a 21
<210> 33
   <211> 21
   <212> DNA
   <213> Artificial
<220>
   <223> shRNA
<400> 33
   tctgctgaag gtcggaacaa g 21
<210> 34
   <211> 21
   <212> DNA
   <213> Artificial
<220>
   <223> shRNA
<400> 34
   acacaaggag atccaccatt t 21